# EUROPEAN PATENT APPLICATION

(11) **EP 1 739 171 A1**
(43) Date of publication of application: **03.01.2007**
(21) Application number: 05719773.3
(22) Date of filing: 02.03.2005
(51) Int. Cl.: C12N 15/09

(54) **NOVEL GENE PARTICIPATING IN EPIDERMAL DIFFERENTIATION AND USE THEREOF**

(30) Priority: 02.03.2004 JP 2004057559
(71) Applicant: Eisai R&D Management Co., Ltd., Tokyo 112-8088 (JP)
(72) Inventor: MATSUI, Takeshi, c/o KAN Research Institute, Inc., Shimogyo-ku, Kyoto-shi Kyoto 6008815 (JP); KISUMI, Fumie, c/o KAN Research Institute, Inc., Shimogyo-ku, Kyoto-shi Kyoto 6008815 (JP); KINOSHITA, Yoko, c/o KAN Research Institute, Inc., Shimogyoku, Kyoto-shi Kyoto 6008815 (JP)
(74) Representative: Vossius & Partner
(86) International application number: PCT/JP2005/003458
(87) International publication number: WO 2005/083075

(57) **Abstract**

A high-throughput *in situ* hybridization system was applied to the mouse foot pad epidermis to identify novel genes involved in epidermal differentiation. As a result, epidermal expression patterns were detected for about 100 unique mRNAs. Of these, two novel secreted proteins, dermokine-α and -β, which are expressed in the suprabasal layer of the differentiated epithelia, were successfully identified. Further, the gene encoding the above proteins was found to constitute a novel gene complex (SSC).

SEQUENCE LISTING

<110>  EISAI CO.,LTD.

<120>  Novel gene participating in epidermal differentiation and use thereof

<130>  E1-A0208P

<150>  2004-03-02
<151>  JP 2004-057559

<160>  53

<170>  PatentIn version 3.1

<210>  1
<211>  661
<212>  DNA
<213>  Mus musculus

<220>
<221>  CDS
<222>  (23)..(337)
<223>

<400>  1
ctgactgtac gagagcacaa cc atg aaa cca gtc acg gcc tct gct ctg ctg      52
                         Met Lys Pro Val Thr Ala Ser Ala Leu Leu
                         1               5                   10

ctt atc ctg ctg ggt gtg gcc tgg cgt gga gac agc cac agc tgg ggt      100
Leu Ile Leu Leu Gly Val Ala Trp Arg Gly Asp Ser His Ser Trp Gly
                15                  20                  25

tca gat ctg tca tct ctg cag aag agg gca ggt gga gct gac cag ttt      148
Ser Asp Leu Ser Ser Leu Gln Lys Arg Ala Gly Gly Ala Asp Gln Phe
            30                  35                  40

tct aag cct gaa gca aga caa gat ctt tca gct gac tca tcc aag aac     196
Ser Lys Pro Glu Ala Arg Gln Asp Leu Ser Ala Asp Ser Ser Lys Asn
        45                  50                  55

tac tac aat aac cag cag gtg aat cct act tac aac tgg caa tac tat     244
Tyr Tyr Asn Asn Gln Gln Val Asn Pro Thr Tyr Asn Trp Gln Tyr Tyr
    60                  65                  70

acc aag acc act gcc aag gcg gga gtc aca cct tca tct tcc tcg gct     292
Thr Lys Thr Thr Ala Lys Ala Gly Val Thr Pro Ser Ser Ser Ser Ala
75                  80                  85                  90

tcc cgg gca caa cct ggc ctg ctg aag tgg ctg aag ttt tgg tag         337
Ser Arg Ala Gln Pro Gly Leu Leu Lys Trp Leu Lys Phe Trp
                95                  100

aacattcctt ctagtcactg cggactcctc acgaatgcac acaggtcttc agggagtttg    397

actgtcctta cccagagtcc tctctgatgc agctgaccta cctgggcatg acaagcctgt    457

catctcgcct ggggacctgg tttatctgtc ctcattctcc ccattcgatt gtggtgtctt    517

ggcgactaat cagtttcatt gtataaccag ccagatcttc acctcttctt ccgtacgtga    577

ccgcaagtcc ctggaacgag gcatctggag cttcctactc tccagtttct ctgtggaaat    637

aaaacatgac tctttgtttc cctg                                           661

<210>  2
<211>  104
<212>  PRT
<213>  Mus musculus

<400>  2
Met Lys Pro Val Thr Ala Ser Ala Leu Leu Leu Ile Leu Leu Gly Val

1               5                   10                  15

Ala Trp Arg Gly Asp Ser His Ser Trp Gly Ser Asp Leu Ser Ser Leu
            20                  25                  30

Gln Lys Arg Ala Gly Gly Ala Asp Gln Phe Ser Lys Pro Glu Ala Arg
        35                  40                  45

Gln Asp Leu Ser Ala Asp Ser Ser Lys Asn Tyr Tyr Asn Asn Gln Gln
    50                  55                  60

Val Asn Pro Thr Tyr Asn Trp Gln Tyr Tyr Thr Lys Thr Thr Ala Lys
65                  70                  75                  80

Ala Gly Val Thr Pro Ser Ser Ser Ser Ala Ser Arg Ala Gln Pro Gly
                85                  90                  95

Leu Leu Lys Trp Leu Lys Phe Trp
            100

<210>  3
<211>  2043
<212>  DNA
<213>  Mus musculus

<220>
<221>  CDS
<222>  (170)..(1723)
<223>

<400>  3
gctggaaagc agggaagtct gggaacagag agagaaggct gtgggtcctg gggaaggaga      60

ataaggaagc aaggaaggaa aggaaaaggt ctcagcccag aggaagaaag aagggaacaa     120

atagactggg ctgcagacat cctcagagga gagagggagc tgggcagag atg aag cta     178

Met Lys Leu
1

cag ggc tct ctg gcc tgc ctc ctg ctg gcc cta tgt ctg ggt ggt ggg 226
Gln Gly Ser Leu Ala Cys Leu Leu Leu Ala Leu Cys Leu Gly Gly Gly
    5                   10                  15

gca gct aac ccg ctg cac agt gga ggg gag ggc aca ggg gca agt gct 274
Ala Ala Asn Pro Leu His Ser Gly Gly Glu Gly Thr Gly Ala Ser Ala
20                  25                  30                  35

gcc cat gga gca gga gat gcc att agc cat gga att gga gag gct gtg 322
Ala His Gly Ala Gly Asp Ala Ile Ser His Gly Ile Gly Glu Ala Val
                40                  45                  50

ggc caa ggg gct aaa gaa gca gcc agc tct gga atc cag aat gcc cta 370
Gly Gln Gly Ala Lys Glu Ala Ala Ser Ser Gly Ile Gln Asn Ala Leu
            55                  60                  65

ggc cag ggg cac gga gag gaa ggt ggc tcc aca ttg atg ggg agc aga 418
Gly Gln Gly His Gly Glu Glu Gly Gly Ser Thr Leu Met Gly Ser Arg
        70                  75                  80

ggc gat gtt ttt gag cac cgg ctt ggg gaa gca gca aga tct ctg ggg 466
Gly Asp Val Phe Glu His Arg Leu Gly Glu Ala Ala Arg Ser Leu Gly
    85                  90                  95

aac gct ggg aat gag att ggc aga cag gct gag gat atc att cgc caa 514
Asn Ala Gly Asn Glu Ile Gly Arg Gln Ala Glu Asp Ile Ile Arg Gln
100                 105                 110                 115

ggg gta gat gct gtc cac aac gct ggg tcc tgg ggg aca tct gga ggt 562
Gly Val Asp Ala Val His Asn Ala Gly Ser Trp Gly Thr Ser Gly Gly
                120                 125                 130

cat ggc gca tat ggc tct caa ggt ggt gct gga gtc cag ggc aat cct 610
His Gly Ala Tyr Gly Ser Gln Gly Gly Ala Gly Val Gln Gly Asn Pro

            135                 140                 145

ggt cct caa ggg aca ccc tgg gcc tca gga ggc aac tat ggg act aac 658
Gly Pro Gln Gly Thr Pro Trp Ala Ser Gly Gly Asn Tyr Gly Thr Asn
        150                 155                 160

tct ctg ggt ggc tct gtg ggt cag ggt ggc aat ggc gga cca ctc aac 706
Ser Leu Gly Gly Ser Val Gly Gln Gly Gly Asn Gly Gly Pro Leu Asn
    165                 170                 175

tat gaa acc aat gcc cag gga gct gtg gct cag cct ggc tac ggg aca 754
Tyr Glu Thr Asn Ala Gln Gly Ala Val Ala Gln Pro Gly Tyr Gly Thr
180                 185                 190                 195

gtg aga ggc aac aac cag aac tca ggg tgt acc aac ccc cca cct tct 802
Val Arg Gly Asn Asn Gln Asn Ser Gly Cys Thr Asn Pro Pro Pro Ser
                200                 205                 210

ggc tcc cat gaa agc ttc agt aac tct ggg gga agc agc aat gat ggc 850
Gly Ser His Glu Ser Phe Ser Asn Ser Gly Gly Ser Ser Asn Asp Gly
            215                 220                 225

agt cgt ggt agc caa ggc agt cat ggc agt aat ggt cag ggc agc agc 898
Ser Arg Gly Ser Gln Gly Ser His Gly Ser Asn Gly Gln Gly Ser Ser
        230                 235                 240

ggt aga ggc ggt ggc caa ggc aac agc gac aac aat ggc agc agt agc 946
Gly Arg Gly Gly Gly Gln Gly Asn Ser Asp Asn Asn Gly Ser Ser Ser
    245                 250                 255

agt agc agc ggc agc aac agt ggc aac agc aac agt ggc aac agc ggc 994
Ser Ser Ser Gly Ser Asn Ser Gly Asn Ser Asn Ser Gly Asn Ser Gly
260                 265                 270                 275

aac agc aac agt ggc aac agc ggc aac agc ggt tct ggg tcc cgg gac 1042
Asn Ser Asn Ser Gly Asn Ser Gly Asn Ser Gly Ser Gly Ser Arg Asp
                280                 285                 290

ata gaa aca tct aat ttt gat gaa ggc tat tcg gtc tcc agg gga acc 1090
Ile Glu Thr Ser Asn Phe Asp Glu Gly Tyr Ser Val Ser Arg Gly Thr
            295                 300                 305

ggc agc agg ggt gga agt ggt gga agt ggt gga agt ggt gga agt ggt 1138
Gly Ser Arg Gly Gly Ser Gly Gly Ser Gly Gly Ser Gly Gly Ser Gly
        310                 315                 320

gga agt ggt gga agt ggt ggt gga aac aaa ccc gag tgt aac aac cca 1186
Gly Ser Gly Gly Ser Gly Gly Gly Asn Lys Pro Glu Cys Asn Asn Pro
    325                 330                 335

ggg aat gat gtg cgc atg gcc gga gga tct ggg agt cag ggg cat ggg 1234
Gly Asn Asp Val Arg Met Ala Gly Gly Ser Gly Ser Gln Gly His Gly
340                 345                 350                 355

tcc aat ggt ggc aat ata caa aaa gaa gct gtc aat gga ctc aac act 1282
Ser Asn Gly Gly Asn Ile Gln Lys Glu Ala Val Asn Gly Leu Asn Thr
                360                 365                 370

atg aac tcg gat gca tct acc ttg ccc ttc aac att gac aat ttc tgg 1330
Met Asn Ser Asp Ala Ser Thr Leu Pro Phe Asn Ile Asp Asn Phe Trp
            375                 380                 385

gag aat ctt aag tcc aag acg cgc ttc att aac tgg gat gcc ata aac 1378
Glu Asn Leu Lys Ser Lys Thr Arg Phe Ile Asn Trp Asp Ala Ile Asn
        390                 395                 400

aag ggt cat gcc cca tct ccc agc act cgg gct tta ctc tac ttc cgc 1426
Lys Gly His Ala Pro Ser Pro Ser Thr Arg Ala Leu Leu Tyr Phe Arg
    405                 410                 415

aaa ctg tgg gag aat ttc aaa cgc agc act cct ttc ttc aac tgg aaa 1474
Lys Leu Trp Glu Asn Phe Lys Arg Ser Thr Pro Phe Phe Asn Trp Lys
420                 425                 430                 435

cag att gag ggt tca gat ctg tca tct ctg cag aag agg gca ggt gga     1522
Gln Ile Glu Gly Ser Asp Leu Ser Ser Leu Gln Lys Arg Ala Gly Gly
                440                 445                 450

gct gac cag ttt tct aag cct gaa gca aga caa gat ctt tca gct gac     1570
Ala Asp Gln Phe Ser Lys Pro Glu Ala Arg Gln Asp Leu Ser Ala Asp
            455                 460                 465

tca tcc aag aac tac tac aat aac cag cag gtg aat cct act tac aac     1618
Ser Ser Lys Asn Tyr Tyr Asn Asn Gln Gln Val Asn Pro Thr Tyr Asn
        470                 475                 480

tgg caa tac tat acc aag acc act gcc aag gcg gga gtc aca cct tca     1666
Trp Gln Tyr Tyr Thr Lys Thr Thr Ala Lys Ala Gly Val Thr Pro Ser
    485                 490                 495

tct tcc tcg gct tcc cgg gca caa cct ggc ctg ctg aag tgg ctg aag     1714
Ser Ser Ser Ala Ser Arg Ala Gln Pro Gly Leu Leu Lys Trp Leu Lys
500                 505                 510                 515

ttt tgg tag aacattcctt ctagtcactg cggactcctc acgaatgcac             1763
Phe Trp

acaggtcttc agggagtttg actgtcctta cccagagtcc tctctgatgc agctgaccta   1823

cctgggcatg acaagcctgt catctcgcct ggggacctgg tttatctgtc ctcattctcc   1883

ccattcgatt gtggtgtctt ggcgactaat cagtttcatt gtataaccag ccagatcttc   1943

acctcttctt ccgtacgtga ccgcaagtcc ctggaacgag gcatctggag cttcctactc   2003

tccagtttct ctgtggaaat aaaacatgac tctttgtttc                         2043

<210>  4
<211>  517

<212>  PRT
<213>  Mus musculus

<400>  4
Met Lys Leu Gln Gly Ser Leu Ala Cys Leu Leu Leu Ala Leu Cys Leu
1               5                   10                  15

Gly Gly Gly Ala Ala Asn Pro Leu His Ser Gly Gly Glu Gly Thr Gly
            20                  25                  30

Ala Ser Ala Ala His Gly Ala Gly Asp Ala Ile Ser His Gly Ile Gly
        35                  40                  45

Glu Ala Val Gly Gln Gly Ala Lys Glu Ala Ala Ser Ser Gly Ile Gln
    50                  55                  60

Asn Ala Leu Gly Gln Gly His Gly Glu Glu Gly Gly Ser Thr Leu Met
65                  70                  75                  80

Gly Ser Arg Gly Asp Val Phe Glu His Arg Leu Gly Glu Ala Ala Arg
                85                  90                  95

Ser Leu Gly Asn Ala Gly Asn Glu Ile Gly Arg Gln Ala Glu Asp Ile
            100                 105                 110

Ile Arg Gln Gly Val Asp Ala Val His Asn Ala Gly Ser Trp Gly Thr
        115                 120                 125

Ser Gly Gly His Gly Ala Tyr Gly Ser Gln Gly Gly Ala Gly Val Gln
    130                 135                 140

Gly Asn Pro Gly Pro Gln Gly Thr Pro Trp Ala Ser Gly Gly Asn Tyr
145                 150                 155                 160

Gly Thr Asn Ser Leu Gly Gly Ser Val Gly Gln Gly Gly Asn Gly Gly
                165                 170                 175

Pro Leu Asn Tyr Glu Thr Asn Ala Gln Gly Ala Val Ala Gln Pro Gly
            180                 185                 190

Tyr Gly Thr Val Arg Gly Asn Asn Gln Asn Ser Gly Cys Thr Asn Pro
        195                 200                 205

Pro Pro Ser Gly Ser His Glu Ser Phe Ser Asn Ser Gly Gly Ser Ser
    210                 215                 220

Asn Asp Gly Ser Arg Gly Ser Gln Gly Ser His Gly Ser Asn Gly Gln
225                 230                 235                 240

Gly Ser Ser Gly Arg Gly Gly Gly Gln Gly Asn Ser Asp Asn Asn Gly
                245                 250                 255

Ser Ser Ser Ser Ser Ser Gly Ser Asn Ser Gly Asn Ser Asn Ser Gly
            260                 265                 270

Asn Ser Gly Asn Ser Asn Ser Gly Asn Ser Gly Asn Ser Gly Ser Gly
        275                 280                 285

Ser Arg Asp Ile Glu Thr Ser Asn Phe Asp Glu Gly Tyr Ser Val Ser
    290                 295                 300

Arg Gly Thr Gly Ser Arg Gly Gly Ser Gly Gly Ser Gly Gly Ser Gly
305                 310                 315                 320

Gly Ser Gly Gly Ser Gly Gly Ser Gly Gly Gly Asn Lys Pro Glu Cys
                325                 330                 335

Asn Asn Pro Gly Asn Asp Val Arg Met Ala Gly Gly Ser Gly Ser Gln
            340                 345                 350

Gly His Gly Ser Asn Gly Gly Asn Ile Gln Lys Glu Ala Val Asn Gly
        355                 360                 365

Leu Asn Thr Met Asn Ser Asp Ala Ser Thr Leu Pro Phe Asn Ile Asp

370                     375                     380

Asn Phe Trp Glu Asn Leu Lys Ser Lys Thr Arg Phe Ile Asn Trp Asp
385                     390                     395                     400

Ala Ile Asn Lys Gly His Ala Pro Ser Pro Ser Thr Arg Ala Leu Leu
                    405                     410                     415

Tyr Phe Arg Lys Leu Trp Glu Asn Phe Lys Arg Ser Thr Pro Phe Phe
                420                     425                     430

Asn Trp Lys Gln Ile Glu Gly Ser Asp Leu Ser Ser Leu Gln Lys Arg
            435                     440                     445

Ala Gly Gly Ala Asp Gln Phe Ser Lys Pro Glu Ala Arg Gln Asp Leu
    450                     455                     460

Ser Ala Asp Ser Ser Lys Asn Tyr Tyr Asn Asn Gln Gln Val Asn Pro
465                     470                     475                     480

Thr Tyr Asn Trp Gln Tyr Tyr Thr Lys Thr Thr Ala Lys Ala Gly Val
                    485                     490                     495

Thr Pro Ser Ser Ser Ser Ala Ser Arg Ala Gln Pro Gly Leu Leu Lys
                500                     505                     510

Trp Leu Lys Phe Trp
            515

<210>  5
<211>  610
<212>  DNA
<213>  Homo sapiens

<220>
<221>  CDS

<222>  (16)..(282)
<223>

<400>  5
   gacaagcaca tgaac atg aag ccg gcc act gcc tct gct ctg ctc ctg ctc      51
                    Met Lys Pro Ala Thr Ala Ser Ala Leu Leu Leu Leu
                    1               5                   10
   ctg ctg ggc ctg gcc tgg acc cag ggg agc cac ggc tgg ggt gcg gac      99
   Leu Leu Gly Leu Ala Trp Thr Gln Gly Ser His Gly Trp Gly Ala Asp
           15                  20                  25
   gcg tca tca ctg cag aaa cgt gca ggc aga gcc gat cag aac tac aat      147
   Ala Ser Ser Leu Gln Lys Arg Ala Gly Arg Ala Asp Gln Asn Tyr Asn
       30                  35                  40
   tac aac cag cat gcg tat ccc act gcc tat ggt ggg aag tac tca gtc      195
   Tyr Asn Gln His Ala Tyr Pro Thr Ala Tyr Gly Gly Lys Tyr Ser Val
   45                  50                  55                  60
   aag acc cct gca aag ggg gga gtc tca cct tct tcc tcg gct tcc cgg      243
   Lys Thr Pro Ala Lys Gly Gly Val Ser Pro Ser Ser Ser Ala Ser Arg
                   65                  70                  75
   gtg caa cct ggc ctg ctg cag tgg gtg aag ttt tgg tag gcaatttctt      292
   Val Gln Pro Gly Leu Leu Gln Trp Val Lys Phe Trp
               80                  85
   gcaaccacca ccgaggcccc gaaaagcact ggtcgtcagg gagctcctcc ccttggcccc      352
   cagcctgtgc cagccctggc ccggctgcca cacctctgtt tcctaggctg gggacccagc      412
   ttgtctctcc ttgtttcttc ccactgcact gtggtgcttc agtggccacc agcctcgtca      472
   catacaccag catctttctg tacctcctcc ctttggtgac ctgaagtcac tgtgacagtt      532
   ctccaggaag gaggagcttc ctacttttga gtttctctgt ggaaataaaa catgaatctt      592
   gtttccctaa aaaaaaaa                                                    610

<210>  6
<211>  88
<212>  PRT
<213>  Homo sapiens

<400>  6
Met Lys Pro Ala Thr Ala Ser Ala Leu Leu Leu Leu Leu Leu Gly Leu
1               5                   10                  15

Ala Trp Thr Gln Gly Ser His Gly Trp Gly Ala Asp Ala Ser Ser Leu
            20                  25                  30

Gln Lys Arg Ala Gly Arg Ala Asp Gln Asn Tyr Asn Tyr Asn Gln His
        35                  40                  45

Ala Tyr Pro Thr Ala Tyr Gly Gly Lys Tyr Ser Val Lys Thr Pro Ala
    50                  55                  60

Lys Gly Gly Val Ser Pro Ser Ser Ser Ala Ser Arg Val Gln Pro Gly
65                  70                  75                  80

Leu Leu Gln Trp Val Lys Phe Trp
                85

<210>  7
<211>  1982
<212>  DNA
<213>  Homo sapiens

<220>
<221>  CDS
<222>  (178)..(1608)

<223>

<400> 7
tctgagaagc ccaggcagtt gaggacagga gagagaaggc tgcagaccca gagggaggga 60

ggacagggag tcggaaggag gaggacagag gagggcacag agacgcagag caagggcggc 120

aaggaggaga ccctggtggg aggaagacac tctggagaga gagggggctg ggcagag 177

atg aag ttc cag ggg ccc ctg gcc tgc ctc ctg ctg gcc ctc tgc ctg 225
Met Lys Phe Gln Gly Pro Leu Ala Cys Leu Leu Leu Ala Leu Cys Leu
1               5                   10                  15

ggc agt ggg gag gct ggc ccc ctg cag agc gga gag gaa agc act ggg 273
Gly Ser Gly Glu Ala Gly Pro Leu Gln Ser Gly Glu Glu Ser Thr Gly
            20                  25                  30

aca aat att ggg gag gcc ctt gga cat ggc ctg gga gac gcc ctg agc 321
Thr Asn Ile Gly Glu Ala Leu Gly His Gly Leu Gly Asp Ala Leu Ser
        35                  40                  45

gaa ggg gtg gga aag gcc att ggc aaa gag gcc gga ggg gca gct ggc 369
Glu Gly Val Gly Lys Ala Ile Gly Lys Glu Ala Gly Gly Ala Ala Gly
    50                  55                  60

tct aaa gtc agt gag gcc ctt ggc caa ggg acc aga gaa gca gtt ggc 417
Ser Lys Val Ser Glu Ala Leu Gly Gln Gly Thr Arg Glu Ala Val Gly
65                  70                  75                  80

act gga gtc agg cag gtt cca ggc ttt ggc gca gca gat gct ttg ggc 465
Thr Gly Val Arg Gln Val Pro Gly Phe Gly Ala Ala Asp Ala Leu Gly
                85                  90                  95

aac agg gtc ggg gaa gca gcc cat gct ctg gga aac act ggg cac gag 513
Asn Arg Val Gly Glu Ala Ala His Ala Leu Gly Asn Thr Gly His Glu
            100                 105                 110

att ggc aga cag gca gaa gat gtc att cga cac gga gca gat gct gtc 561
Ile Gly Arg Gln Ala Glu Asp Val Ile Arg His Gly Ala Asp Ala Val
        115                 120                 125

cgc ggc tcc tgg cag ggg gtg cct ggc cac aat ggt gct tgg gaa act 609
Arg Gly Ser Trp Gln Gly Val Pro Gly His Asn Gly Ala Trp Glu Thr
    130                 135                 140

tct gga ggc cat ggc atc ttt ggc tct caa ggt ggc ctt gga ggc cag 657
Ser Gly Gly His Gly Ile Phe Gly Ser Gln Gly Gly Leu Gly Gly Gln
145                 150                 155                 160

ggc cag ggc aat cct gga ggt ctg ggg act ccg tgg gtc cac gga tac 705
Gly Gln Gly Asn Pro Gly Gly Leu Gly Thr Pro Trp Val His Gly Tyr
                165                 170                 175

ccc gga aac tca gca ggc agc ttt gga atg aat cct cag gga gct ccc 753
Pro Gly Asn Ser Ala Gly Ser Phe Gly Met Asn Pro Gln Gly Ala Pro
            180                 185                 190

tgg ggt caa gga ggc aat gga ggg cca cca aac ttt ggg acc aac act 801
Trp Gly Gln Gly Gly Asn Gly Gly Pro Pro Asn Phe Gly Thr Asn Thr
        195                 200                 205

cag gga gct gtg gcc cag cct ggc tat ggt tca gtg aga gcc agc aac 849
Gln Gly Ala Val Ala Gln Pro Gly Tyr Gly Ser Val Arg Ala Ser Asn
    210                 215                 220

cag aat gaa ggg tgc acg aat ccc cca cca tct ggc tca ggt gga ggc 897
Gln Asn Glu Gly Cys Thr Asn Pro Pro Pro Ser Gly Ser Gly Gly Gly
225                 230                 235                 240

tcc agc aac tct ggg gga ggc agc ggc tca cag tcg ggc agc agt ggc 945
Ser Ser Asn Ser Gly Gly Gly Ser Gly Ser Gln Ser Gly Ser Ser Gly
                245                 250                 255

agt ggc agc aat ggt gac aac aac aat ggc agc agc agt ggt ggc agc 993

Ser Gly Ser Asn Gly Asp Asn Asn Asn Gly Ser Ser Ser Gly Gly Ser
            260                 265                 270

agc agt ggc agc agc agt ggc ggc agc agt ggc ggc agc agt ggt ggc 1041
Ser Ser Gly Ser Ser Ser Gly Gly Ser Ser Gly Gly Ser Ser Gly Gly
        275                 280                 285

agc agt ggc aac agt ggt ggc agc aga ggt gac agc ggc agt gag tcc 1089
Ser Ser Gly Asn Ser Gly Gly Ser Arg Gly Asp Ser Gly Ser Glu Ser
    290                 295                 300

tcc tgg gga tcc agc acc ggc tcc tcc tcc ggc aac cac ggt ggg agc 1137
Ser Trp Gly Ser Ser Thr Gly Ser Ser Ser Gly Asn His Gly Gly Ser
305                 310                 315                 320

ggc gga gga aat gga cat aaa ccc ggg tgt gaa aag cca ggg aat gaa 1185
Gly Gly Gly Asn Gly His Lys Pro Gly Cys Glu Lys Pro Gly Asn Glu
                325                 330                 335

gcc cgc ggg agc ggg gaa tct ggg att cag aac tct gag acg tct cct 1233
Ala Arg Gly Ser Gly Glu Ser Gly Ile Gln Asn Ser Glu Thr Ser Pro
            340                 345                 350

ggg atg ttt aac ttt gac act ttc tgg aag aat ttt aaa tcc aag ctg 1281
Gly Met Phe Asn Phe Asp Thr Phe Trp Lys Asn Phe Lys Ser Lys Leu
        355                 360                 365

ggt ttc atc aac tgg gat gcc ata aac aag aac cag gtc ccg ccc ccc 1329
Gly Phe Ile Asn Trp Asp Ala Ile Asn Lys Asn Gln Val Pro Pro Pro
    370                 375                 380

agc acc cga gcc ctc ctc tac ttc agc cga ctc tgg gag gat ttc aaa 1377
Ser Thr Arg Ala Leu Leu Tyr Phe Ser Arg Leu Trp Glu Asp Phe Lys
385                 390                 395                 400

cag aac act cct ttc ctc aac tgg aaa gca att att gag ggt gcg gac 1425
Gln Asn Thr Pro Phe Leu Asn Trp Lys Ala Ile Ile Glu Gly Ala Asp

                405                 410                 415

gcg tca tca ctg cag aaa cgt gca ggc aga gcc gat cag aac tac aat 1473
Ala Ser Ser Leu Gln Lys Arg Ala Gly Arg Ala Asp Gln Asn Tyr Asn
            420                 425                 430

tac aac cag cat gcg tat ccc act gcc tat ggt ggg aag tac tca gtc 1521
Tyr Asn Gln His Ala Tyr Pro Thr Ala Tyr Gly Gly Lys Tyr Ser Val
        435                 440                 445

aag acc cct gca aag ggg gga gtc tca cct tct tcc tcg gct tcc cgg 1569
Lys Thr Pro Ala Lys Gly Gly Val Ser Pro Ser Ser Ser Ala Ser Arg
    450                 455                 460

gtg caa cct ggc ctg ctg cag tgg gtg aag ttt tgg tag gcaatttctt 1618
Val Gln Pro Gly Leu Leu Gln Trp Val Lys Phe Trp
465                 470                 475

gcaaccacca ccgaggcccc gaaaagcact ggtcgtcagg gagctcctcc ccttggcccc 1678

cagcctgtgc cagccctggc ccggctgcca cacctctgtt tcctaggctg gggacccagc 1738

ttgtctctcc ttgtttcttc ccactgcact gtggtgcttc agtggccacc agcctcgtca 1798

catacaccag catctttctg tacctcctcc ctttggtgac ctgaagtcac tgtgacagtt 1858

ctccaggaag gaggagcttc ctacttttga gtttctctgt ggaaataaaa catgaatctt 1918

gtttccctaa aaaaaaaaaa aaaaaaaaaa aaaaaaaaaa aaaaaaaaaa aaaaaaaaaa 1978

aaaa 1982

<210> 8
<211> 476
<212> PRT
<213> Homo sapiens

<400> 8
Met Lys Phe Gln Gly Pro Leu Ala Cys Leu Leu Leu Ala Leu Cys Leu
1               5                   10                  15

Gly Ser Gly Glu Ala Gly Pro Leu Gln Ser Gly Glu Glu Ser Thr Gly
            20                  25                  30

Thr Asn Ile Gly Glu Ala Leu Gly His Gly Leu Gly Asp Ala Leu Ser
        35                  40                  45

Glu Gly Val Gly Lys Ala Ile Gly Lys Glu Ala Gly Gly Ala Ala Gly
    50                  55                  60

Ser Lys Val Ser Glu Ala Leu Gly Gln Gly Thr Arg Glu Ala Val Gly
65                  70                  75                  80

Thr Gly Val Arg Gln Val Pro Gly Phe Gly Ala Ala Asp Ala Leu Gly
                85                  90                  95

Asn Arg Val Gly Glu Ala Ala His Ala Leu Gly Asn Thr Gly His Glu
            100                 105                 110

Ile Gly Arg Gln Ala Glu Asp Val Ile Arg His Gly Ala Asp Ala Val
        115                 120                 125

Arg Gly Ser Trp Gln Gly Val Pro Gly His Asn Gly Ala Trp Glu Thr
    130                 135                 140

Ser Gly Gly His Gly Ile Phe Gly Ser Gln Gly Gly Leu Gly Gly Gln
145                 150                 155                 160

Gly Gln Gly Asn Pro Gly Gly Leu Gly Thr Pro Trp Val His Gly Tyr
                165                 170                 175

Pro Gly Asn Ser Ala Gly Ser Phe Gly Met Asn Pro Gln Gly Ala Pro
            180                 185                 190

Trp Gly Gln Gly Gly Asn Gly Gly Pro Pro Asn Phe Gly Thr Asn Thr
        195                 200                 205

Gln Gly Ala Val Ala Gln Pro Gly Tyr Gly Ser Val Arg Ala Ser Asn
    210                 215                 220

Gln Asn Glu Gly Cys Thr Asn Pro Pro Pro Ser Gly Ser Gly Gly Gly
225                 230                 235                 240

Ser Ser Asn Ser Gly Gly Gly Ser Gly Ser Gln Ser Gly Ser Ser Gly
                245                 250                 255

Ser Gly Ser Asn Gly Asp Asn Asn Asn Gly Ser Ser Ser Gly Gly Ser
            260                 265                 270

Ser Ser Gly Ser Ser Ser Gly Gly Ser Ser Gly Gly Ser Ser Gly Gly
        275                 280                 285

Ser Ser Gly Asn Ser Gly Gly Ser Arg Gly Asp Ser Gly Ser Glu Ser
    290                 295                 300

Ser Trp Gly Ser Ser Thr Gly Ser Ser Ser Gly Asn His Gly Gly Ser
305                 310                 315                 320

Gly Gly Gly Asn Gly His Lys Pro Gly Cys Glu Lys Pro Gly Asn Glu
                325                 330                 335

Ala Arg Gly Ser Gly Glu Ser Gly Ile Gln Asn Ser Glu Thr Ser Pro
            340                 345                 350

Gly Met Phe Asn Phe Asp Thr Phe Trp Lys Asn Phe Lys Ser Lys Leu
        355                 360                 365

Gly Phe Ile Asn Trp Asp Ala Ile Asn Lys Asn Gln Val Pro Pro Pro
    370                 375                 380

Ser Thr Arg Ala Leu Leu Tyr Phe Ser Arg Leu Trp Glu Asp Phe Lys
385                 390                 395                 400

Gln Asn Thr Pro Phe Leu Asn Trp Lys Ala Ile Ile Glu Gly Ala Asp
                405                 410                 415

Ala Ser Ser Leu Gln Lys Arg Ala Gly Arg Ala Asp Gln Asn Tyr Asn
            420                 425                 430

Tyr Asn Gln His Ala Tyr Pro Thr Ala Tyr Gly Gly Lys Tyr Ser Val
        435                 440                 445

Lys Thr Pro Ala Lys Gly Gly Val Ser Pro Ser Ser Ser Ala Ser Arg
    450                 455                 460

Val Gln Pro Gly Leu Leu Gln Trp Val Lys Phe Trp
465                 470                 475

<210>  9
<211>  21
<212>  DNA
<213>  Artificial

<220>
<223>  an artificially synthesized primer sequence

<400>  9
ggacgcccac ctttcatctt c                                                21

<210>  10
<211>  19
<212>  DNA
<213>  Artificial

<220>

<223>  an artificially synthesized primer sequence

<400>  10
atctggcggt tggtggagg                                                    19

<210>  11
<211>  25
<212>  DNA
<213>  Artificial

<220>
<223>  an artificially synthesized primer sequence

<400>  11
cagttctaca tttgtgttgc acgtc                                             25

<210>  12
<211>  25
<212>  DNA
<213>  Artificial

<220>
<223>  an artificially synthesized primer sequence

<400>  12
ttggacagac tctggaggaa gtcag                                             25

<210>  13
<211>  26
<212>  DNA
<213>  Artificial

<220>
<223>  an artificially synthesized primer sequence

<400>  13
ttgaaaggac cccagtgctg aactgc                                          26

<210>  14
<211>  28
<212>  DNA
<213>  Artificial

<220>
<223>  an artificially synthesized primer sequence

<400>  14
atggagctgc ccacaatgag tggtacag                                        28

<210>  15
<211>  18
<212>  DNA
<213>  Artificial

<220>
<223>  an artificially synthesized primer sequence

<400>  15
cctacctggc cgtgcaag                                                   18

<210>  16
<211>  22
<212>  DNA
<213>  Artificial

<220>
<223>  an artificially synthesized primer sequence

<400> 16
catgagaaag ttaagcccat cg 22

<210> 17
<211> 35
<212> DNA
<213> Artificial

<220>
<223> an artificially synthesized primer sequence

<400> 17
gtcgacgcca ccatgaacat gaagccggcc actgc 35

<210> 18
<211> 33
<212> DNA
<213> Artificial

<220>
<223> an artificially synthesized primer sequence

<400> 18
gcggccgccc aaaacttcac ccactgcagc agg 33

<210> 19
<211> 34
<212> DNA
<213> Artificial

<220>
<223> an artificially synthesized primer sequence

<400> 19

gtcgacgcca ccatgaagtt ccaggggccc ctgg 34

<210> 20
<211> 33
<212> DNA
<213> Artificial

<220>
<223> an artificially synthesized primer sequence

<400> 20
gcggccgccc aaaacttcac ccactgcagc agg 33

<210> 21
<211> 36
<212> DNA
<213> Artificial

<220>
<223> an artificially synthesized primer sequence

<400> 21
gtcgacgcca ccatgaagat cccggtcctt cctgcc 36

<210> 22
<211> 30
<212> DNA
<213> Artificial

<220>
<223> an artificially synthesized primer sequence

<400> 22
gcggccgcct gggcatcagg agttgcgctc 30

<210> 23
<211> 39
<212> DNA
<213> Artificial

<220>
<223> an artificially synthesized primer sequence

<400> 23
aattgtcgac gccaccatgc atcttgcacg tctggtcgg 39

<210> 24
<211> 36
<212> DNA
<213> Artificial

<220>
<223> an artificially synthesized primer sequence

<400> 24
atatgcggcc gcgcagctgg ttggcctcct tgctgg 36

<210> 25
<211> 23
<212> DNA
<213> Artificial

<220>
<223> an artificially synthesized primer sequence

<400> 25
gactgtacga gagcacaacc atg 23

<210> 26
<211> 18
<212> DNA
<213> Artificial

<220>
<223> an artificially synthesized primer sequence

<400> 26
ctgaacccca gctgtggc 18

<210> 27
<211> 18
<212> DNA
<213> Artificial

<220>
<223> an artificially synthesized primer sequence

<400> 27
catgccccat ctcccagc 18

<210> 28
<211> 24
<212> DNA
<213> Artificial

<220>
<223> an artificially synthesized primer sequence

<400> 28
ccctcaatct gtttccagtt gaag 24

<210> 29
<211> 26
<212> DNA
<213> Artificial

<220>
<223> an artificially synthesized primer sequence

<400> 29
actggcacgt catcactgat atgttc 26

<210> 30
<211> 20
<212> DNA
<213> Artificial

<220>
<223> an artificially synthesized primer sequence

<400> 30
ggaatcagga gcggcacttc 20

<210> 31
<211> 25
<212> DNA
<213> Artificial

<220>
<223> an artificially synthesized primer sequence

<400> 31
gtcaacaagc catttatcaa cttcc 25

<210> 32

<211>  20
<212>  DNA
<213>  Artificial

<220>
<223>  an artificially synthesized primer sequence

<400>  32
gtgtgacaac cggagcattc                                                    20


<210>  33
<211>  24
<212>  DNA
<213>  Artificial

<220>
<223>  an artificially synthesized primer sequence

<400>  33
aaggtggtga agcaggcatc tgag                                               24


<210>  34
<211>  27
<212>  DNA
<213>  Artificial

<220>
<223>  an artificially synthesized primer sequence

<400>  34
ggaagagtgg gagttgctgt tgaagtc                                            27


<210>  35
<211>  20

<212>  DNA
<213>  Artificial

<220>
<223>  an artificially synthesized primer sequence

<400>  35
atgaacatga agccggccac                                                    20

<210>  36
<211>  21
<212>  DNA
<213>  Artificial

<220>
<223>  an artificially synthesized primer sequence

<400>  36
cgtttctgca gtgatgacgc g                                                  21

<210>  37
<211>  22
<212>  DNA
<213>  Artificial

<220>
<223>  an artificially synthesized primer sequence

<400>  37
aaaggccatt ggcaaagagg cc                                                 22

<210>  38
<211>  22
<212>  DNA

<213>  Artificial

<220>
<223>  an artificially synthesized primer sequence

<400>  38
accctgttgc ccaaagcatc tg                                                22

<210>  39
<211>  27
<212>  DNA
<213>  Artificial

<220>
<223>  an artificially synthesized primer sequence

<400>  39
aactggcacg ccctctttga gtctatc                                           27

<210>  40
<211>  24
<212>  DNA
<213>  Artificial

<220>
<223>  an artificially synthesized primer sequence

<400>  40
atggtcactg ggcatcagga gttg                                              24

<210>  41
<211>  23
<212>  DNA
<213>  Artificial

<220>
<223>  an artificially synthesized primer sequence

<400>  41
aaccagctgc tgaatggcaa cca                                              23

<210>  42
<211>  22
<212>  DNA
<213>  Artificial

<220>
<223>  an artificially synthesized primer sequence

<400>  42
atgaaaggcg tgttgaccga gg                                               22

<210>  43
<211>  25
<212>  DNA
<213>  Artificial

<220>
<223>  an artificially synthesized primer sequence

<400>  43
cttggcagaa acagaaggtc gctac                                            25

<210>  44
<211>  23
<212>  DNA
<213>  Artificial

<220>
<223> an artificially synthesized primer sequence

<400> 44
cggtttcagc tcgaatctct tgc 23

<210> 45
<211> 25
<212> DNA
<213> Artificial

<220>
<223> an artificially synthesized primer sequence

<400> 45
ccacccaaac ataaataacc acccg 25

<210> 46
<211> 24
<212> DNA
<213> Artificial

<220>
<223> an artificially synthesized primer sequence

<400> 46
tagcggaccc gaaataagtg gagc 24

<210> 47
<211> 20
<212> DNA
<213> Artificial

<220>

<223>  an artificially synthesized primer sequence

<400>  47
atgtctcagg ccacgtcaag                                                    20

<210>  48
<211>  20
<212>  DNA
<213>  Artificial

<220>
<223>  an artificially synthesized primer sequence

<400>  48
ctgctcccag taacgtgagg                                                    20

<210>  49
<211>  24
<212>  DNA
<213>  Artificial

<220>
<223>  an artificially synthesized primer sequence

<400>  49
atcaagatca ttgctcctcc tgag                                               24

<210>  50
<211>  21
<212>  DNA
<213>  Artificial

<220>
<223>  an artificially synthesized primer sequence

<400> 50
tgcttgctga tccacatctg c 21

<210> 51
<211> 22
<212> DNA
<213> Artificial

<220>
<223> an artificially synthesized primer sequence

<400> 51
acttcaacag cgacacccac tc 22

<210> 52
<211> 22
<212> DNA
<213> Artificial

<220>
<223> an artificially synthesized primer sequence

<400> 52
cctgttgctg tagccaaatt cg 22

<210> 53
<211> 104
<212> PRT
<213> Mus musculus

<400> 53
Met Lys Pro Val Thr Ala Ser Ala Leu Leu Leu Ile Leu Leu Gly Val
1               5                   10                  15

Ala Trp Arg Gly Asp Ser His Ser Trp Gly Ser Asp Leu Ser Ser Leu
            20                  25                  30
Gln Lys Arg Ala Gly Gly Ala Asp Gln Phe Ser Lys Pro Glu Ala Arg
        35                  40                  45
Gln Asp Leu Ser Ala Asp Ser Ser Lys Asn Tyr Tyr Asn Asn Gln Gln
    50                  55                  60
Val Asn Pro Thr Tyr Asn Trp Gln Tyr Tyr Thr Lys Thr Thr Ala Lys
65                  70                  75                  80
Ala Gly Val Thr Pro Ser Ser Ser Ser Ala Ser Arg Ala Gln Pro Gly
                85                  90                  95
Leu Leu Lys Trp Leu Lys Phe Trp
            100

## Description

### Technical Field

The present invention relates to novel secreted proteins expressed in the stratified epithelium.

### Background Art

The epidermis is a typical highly keratinized, stratified squamous epithelium composed of basal, spinous, granular, and cornified cell layers. Epidermal cells (keratinocytes) migrate upward from the basal to the cornified cell layer. This migration is accompanied by a characteristic differentiation of keratinocytes with concomitant and characteristic morphological changes. In the final stages of differentiation, keratinocytes lose their intracellular organellae and flatten to constitute the cornified layer. Thus, the spatial and temporal change patterns in gene expression during keratinocyte differentiation have drawn interest from not only dermatologists but also cell biologists (Non-Patent Documents 1 to 3). For example, keratinocytes in the basal cell layer are highly proliferative and express the basal-type keratin pair (K5/K14). As they move away from the basal membrane, keratinocytes undergo differentiation, characterized by changes in keratin expression from K5/K14 to the suprabasal-type pair K1/K10 (Non-Patent Documents 4 to 7). Further, in the upper spinous and granular cell layers, keratinocytes begin to synthesize the cornified envelope (CE) and precursor proteins of a crosslinking enzyme, transglutaminase (TGase) 1, and so on. The CE is a highly insoluble structure on the cytoplasmic side of plasma membranes of keratinocytes in the cornified layer and is composed of various distinct types of proteins, which are cross-linked by TGase 1 (Non-Patent Documents 8 to 11).

In terms of epidermal barrier function, the components of the CE are well identified, and in addition to cystatin, desmoplakin, envoplakin, elafin, filaggrin, and other types of keratin, they comprise involucrin, small proline-rich protein family members (SPRRPs) and loricrin, for example (Non-Patent Document 11). Furthermore, repetin, hornein, and periphilin have been identified as putative CE precursor proteins (Non-Patent Documents 12 to 14). Interestingly, many of these CE-related proteins are encoded by genes clustered as the so-called "epidermal differentiation complex (EDC)" on human chromosome 1q21 (Non-Patent Documents 15 to 17) and mouse chromosome 3 (Non-Patent Documents 18 and 19). However, a few CE protein genes, as those encoding periplakin, sciellin, envoplakin, hornein, and periphilin, are not located in the EDC (Non-Patent Documents 13, 14, and 20 to 22). The EDC comprises at least 32 genes that cause the keratinization process, and these genes are classified into three groups based on their structures (Non-Patent Document 23). Involucrin, loricrin, and SPRRPs of the first group comprise a short tandemly-repeated sequence in their central region (Non-Patent Document 24). The second group of profilaggrin, trichohyalin, and repetin is called the fused gene subgroup: proteins comprise an EF-hand domain in their N-terminal region followed by multiple tandemly-repeated sequences. S 100 family members, which are the third group, comprise an EF-hand motif.

All of the genes in the human genome were recently identified, as is the case for the mouse genome, and it is considered that the spatial and temporal changes in gene expression patterns during keratinocyte differentiation will be analyzed more systematically, using genome-wide methods.

Recent advances in the techniques of molecular biology and biochemistry have made it possible to study the expression of numerous genes and proteins in human keratinocytes both in *vivo and in vitro.* Celis *et al.* clarified the expression of numerous proteins in the epidermis and in cultured keratinocytes using two-dimensional polyacrylamide electrophoresis (Non-Patent Document 25). Katz *et al.* reported a partial catalogue of secreted proteins in keratinocyte culture supernatants (Non-Patent Document 26). The expression profiles of numerous keratinocyte genes and proteins have been provided by keratinocyte cDNA libraries produced by random sequencing (Non-Patent Documents 27 and 28), differential display PCR (Non-Patent Documents 29 to 34), cDNA microarrays (Non-Patent Documents 35 to 37), serial analysis of gene expression (Non-Patent Documents 38 and 39), and signal sequence trapping (Non-Patent Document 40).

However, when the entire skin is used to prepare cDNA libraries or cell lysates, heterogenous structures such as keratinocytes, melanocytes, fibroblasts, sebocytes, endothelial cells, neuronal cells, or muscle cells are comprised, which makes it difficult to analyze the gene expression profile of keratinocytes alone. In some reports, researchers used primary cultured keratinocytes, which, after selection, were formed into a stratified epithelium by raft culture to obtain information on just the keratinocytes (Non-Patent Documents 41 to 45). However, even in this system, it is difficult to obtain information on gene expression for the individual layers of the epidermis.
[Non-Patent Document 1] Watt FM, "Terminal differentiation of epidermal keratinocytes", Curr. Opin. Cell Biol., 1989, Vol.1, p.1107-1115.
[Non-Patent Document 2] Eckert RL, "Structure, function and differentiation of the keratinocyte", Physiol. Rev., 1989, Vol.69, p.1316-1346.
[Non-Patent Document 3] Eckert RL, Crish JF, Robinson NA, "The epidermal keratinocyte as a model for the study of gene regulation and cell differentiation", Physiol. Rev., 1997, Vol.77, p.397-424.
[Non-Patent Document 4] Fuchs E, Green H, "Changes in keratin gene expression during terminal differentiation of the keratinocyte", Cell, 1980, Vol. 19, p.1033-1042.
[Non-Patent Document 5] Moll R, Franke WW, Volc-Platzer B, Krepler R, "Different keratin polypeptides in epidermis and other epithelia of human skin: a specific cytokeratin of molecular weight 46,000 in epithelia of the pilosebaceous tract and basal cell epitheliomas", J Cell Biol, 1982, Vol.95, p.285-295.
[Non-Patent Document 6] Fuchs E and Byrne C, "The epidermis rising to the surface", Curr. Opin. Genetics Dev., 1994, Vol.4, p.725-736.
[Non-Patent Document 7] Fuchs E, "Keratins and the skin", Annu. Rev. Cell Dev. Biol., 1995, Vol.11, p.123-153.
[Non-Patent Document 8] Roop, D., "Defects in the barrier", Science, 1995, Vol.267, p.474-475.
[Non-Patent Document 9] Ishida-Yamamoto A, Iizuka H, "Structural organization of cornified cell envelopes and alterations in inherited skin disorders", Exp. Dermatol., 1998, Vol. 7, p.1-10.
[Non-Patent Document 10] Kalinin A, Marekov LN, Steinert PM, "Assembly of epidermal cornified cell envelope", J. Cell Sci., 2001, Vol. 114, p.3069-3070.
[Non-Patent Document 11 ] Kalinin AE, Kaj ava AV, Steinert PM, "Epithelial barrier function: assembly and structural features of the cornified cell envelope", BioEssays, 2002, Vol.24, p.789-800.
[Non-Patent Document 12] Krieg P, Schuppler M, Koesters R, Mincheva A, Lichter P, and Marks F, "Repetin (Rptn), a new member of the "fused gene" subgroup within the S 100 gene family encoding a murine epidermal differentiation protein", Genomics, 1997, Vol.43, p.339-348.
[Non-Patent Document 13] Makino T, Takaishi M, Morohashi M, Huh N, "Hornein, a novel profilaggrin-like protein and differentiation-specific marker isolated from mouse skin", J Biol. Chem., 2001, Vol .276, p.47445-47452.
[Non-Patent Document 14] Kazerounian S, Aho S, "Characterization of periphilin, a widespread, highly insoluble nuclear protein and potential constituent of the keratinocyte cornified envelope", J. Biol. Chem., 2003, Vol.278, p.36707-36717.
[Non-Patent Document 15] Volz A, Korge BP, Compton JG, Ziegler A, Steinert PM, Mischke D, "Physical mapping of a functional cluster of epidermal differentiation genes on chromosome 1q21", Genomics, 1993, Vol.18, p.92-99. [Non-Patent Document 16] Mischke D, Korge BP, Marenholz I, Volz A, Ziegler A, "Genes encoding structural proteins of epidermal cornification and S100 calcium-binding proteins form a gene complex ("epidermal differentiation complex") on human chromosome 1q21", J. Invest. Dermatol., 1996, Vol. 106, p.989-992.
[Non-Patent Document 17] Marenholz I, Volz A, Ziegler A, Davies A, Ragoussis I, Korge BP, Mischke D, "Genetic analysis of the epidermal differentiation complex (EDC) on human chromosome 1q21: chromosomal orientation, new markers, and a 6-Mb YAC contig", Genomics, 1996, Vol.37, p.295-302.
[Non-Patent Document 18] Rothnagel JA, Longley MA, Bundman DS, Naylor SL, Lalley PA, Jenkins NA, Gilbert DJ, Copeland NG, Roop DR, "Characterization of the mouse loricrin gene: linkage with profillagerin and the flaky tail and soft coat mutant loci on chromosome 3", Genomics, 1994, Vol.23, p.450-456.
[Non-Patent Document 19] Song HJ, Poy G, Darwiche N, Lichti U, Kuroki T, Steinert PM, Kartasova T, "Mouse Sprr2 genes: a clustered family of genes showing differential expression in epithelial tissues", Genomics, 1999, Vol.55, p.28-42.
[Non-Patent Document 20] Aho S, McLean WHI, Li K, Uitto J, "cDNA cloning, mRNA expression, and chromosomal mapping of human and mouse periplakin genes", Genomics, 1998, Vol.48, p.242-247.
[Non-Patent Document 21] Champliaud M, Burgeson RE, Jin W, Baden HP, Olson PF, "cDNA cloning and characterization of sciellin, a LIM domain protein of the keratinocyte cornified envelope", J. Biol. Chem., 1998, Vol.273, p.31547-31554.
[Non-Patent Document 22] Maata A, Ruhrberg C, Watt F, "Structure and regulation of the envoplakin gene", J. Biol. Chem., 2000, Vol.275, p.19857-19865 (the "a"'s in "Maata" have an umlaut).
[Non-Patent Document 23] Marenholtz I, Zirra M, Fischer DF, Backendorf C, Ziegler A, Mischke D, "Identification of human epidermal differentiation complex (EDC)-encoded genes by subtractive hybridization of entire YACs to a gridded keratinocyte cDNA library", Genome Research, 2001, Vol.11, p.341-355.
[Non-Patent Document 24] Backendorf C and Hohl D, "A common origin for cornified envelope proteins?", Nat. Genet., 1992, Vol.2, p.91.
[Non-Patent Document 25] Celis JE, Rasmussen HH, Gromov P, et al. "The human keratinocyte two-dimentional gel protein database (update 1995).mapping components of signal transduction pathways", Electrophoresis, 1995, Vol. 16, p.2177-2240.
[Non-Patent Document 26] Katz AB, and Taichman LB, "A partial catalogue of proteins secreted by epidermal keratinocytes in culture", J. Invest. Dermatol., 1999, Vol. 112, p.818-821.
[Non-Patent Document 27] Konishi K, Morishima Y, Ueda E, Kibe Y, Nonomura K, Yamanishi K, Yasuno H, "Cataloging of the genes expressed in human keratinocytes: analysis of 607 randomly isolated cDNA sequences", Biochem. Biophys. Res. Commun., 1994, Vol.202, p.976-983.
[Non-Patent Document 28] Kita H, Okubo K, Matsubara K, "an expression profile of active genes in cultured human keratinocytes", DNA Res., 1996, Vol.3, p.1-7.
[Non-Patent Document 29] Frank S, Werner S, "The human homologue of the yeast CHL1 gene is a novel keratinocyte growth factor-regulated gene", J. Biol. Chem., 1996, Vol.271, p.24337-24340.
[Non-Patent Document 30] Frank S, Munz B, Werner S, "The human homologue of a bovine nonselenium glutathione peroxidase is a novel keratinocyte growth factor-regulated gene", Oncogene, 1997, Vol.14, p.915-921.
[Non-Patent Document 31] Munz B, Gerke V, Gillitzer R, Werner S, "Differential expression of the calpain I subunits annexin II and p11 in cultured keratinocytes and during wound repair", J. Invest. Dermatol., 1997, Vol.108, p.307-312.
[Non-Patent Document 32] Rivas MV, Jarvis ED, Morisaki S, Carbonaro H, Gottlieb AB, Krueger JG, "Identification of aberrantly regulated genes in diseased skin using the cDNA differential display technique", J. Invest. Dermatol., 1997, Vol. 108, p.188-194.
[Non-Patent Document 33] Rutberg SE, Lee EJ, Hansen LH, Glick AB, Yuspa SH, "Identification of differentially expressed genes in chemically induced skin tumors", Mol. Carcinogen., 1997, Vol.20, p.80-98.
[Non-Patent Document 34] DiSepio D, Ghosn C, Eckert RL, Deucher A, Robinson N, Duvic M, Chandraratna RAS, Nagpal S, "Identification and characterization of a retinoid-induced class II tumor suppressor/growth regulatory gene", Proc. Natl. Acad. Sci. USA., 1998, Vol.95, p.14811-14815.
[Non-Patent Document 35] Trenkle T, Welsh J, Jung B, Mathieu-Daude F, McClelland M, "Nonstoichiometric reduced complexity probes for cDNA arrays", Nucleic Acids Res., 1998, Vol.26, p.3883-3891.
[Non-Patent Document 36] Cole J, Tsou R, Wallance K, Gibran N, Isik F, "Early gene expression profile of human skin to injury using high-density cDNA microarrays", Wound Repair and Degeneration, 2001, Vol.9, p.360-370.
[Non-Patent Document 37] Bernard FX, Pedretti N, Rosdy M, Deguercy A, "Comparison of gene expression profiles in human keratinocyte mono-layer cultures, reconstituted epidermis and normal human skin; transcriptional effects of retinoid treatments in reconstituted human epidermis", Exp. Dermatol., 2002, Vol.11, p.59-74.
[Non-Patent Document 38] Jansen BJH, Van Ruissen F, De Jongh G, Zeeuwen PLJM, Schalkwijk J, "Serial analysis of gene expression indifferentiated cultures of human epidermal keratinocytes", J. Invest. Dermatol., 2001, Vol. 116, p.12-22.
[Non-Patent Document 39] Ruissen FV, Jansen BJH, deJongh GJ, Zeeuwen PLJM, Schalkwijk J, "A partial transcriptome of human epidermis", Genomics, 2002, Vol.79, p.671-678.
[Non-Patent Document 40] Bonkobara M, Das A, Takao J, Cruzjr PD, Ariizumi K, "Identification of novel genes for secreted and membrane-anchored proteins in human keratinocytes", Brit. J. Dermatology, 2003, Vol. 148, p.654-664.
[Non-Patent Document 41] Bell E, Ehrlich HP, Buttle DJ, Nakatsuji T, "Living tissue formed in vivo and accepted as skin-equivalent tissue of full thickness", Science, 1981, Vol.211, p.1052-1054.
[Non-Patent Document 42] Mackenzie IC, Fusenig NE, "Regeneration of organized epithelial structure", J. Invest. Dermatol., 1983, Vol.81, p.189s-194s.
[Non-Patent Document 43] Asselineau D, Prunieras M, "Reconstruction of simplified skin: control of fabrication", Br.J. Dermatol., 1984, Vol. 111, p.219-222.
[Non-Patent Document 44] Parenteau NL, Nolte CM, Bilbo P, Rosenberg M, Wilkins LM, Johnson EW, Watson S, Mason VS, Bell E, "Epidermis generated in vitro: practical considerations and applications", J Cell Biochem., 1991, Vol.45, p.245-251.
[Non-Patent Document 45] Stark HJ, Baur M, Breitkreutz D, Mirancea M, Fusenig NE, "Organotypic keratinocyte cocultures in defined medium with regular epidermal morphogenesis and differentiation", J. Invest. Dermatol., 1999, Vol. 112, p.681-691.

### Disclosure of the Invention

### Problems to be Solved by the Invention

An objective of the present invention is to provide novel genes involved in epidermal differentiation.

### Means to Solve the Problems

The present inventors conducted dedicated research to solve the above-mentioned problems. To identify novel genes involved in epidermal differentiation, the present inventors applied a recently established method for screening genes, called "high-throughput *in situ* hybridization (HT-ISH) system" (Komiya T., Tanigawa Y., and Hirohashi S., Anal. Biochem., 254: 23-30, 1997), to the mouse foot pad epidermis. This method enables the analysis of gene expression patterns of keratinocytes in each layer of an 'intact' epidermis, without having to separate each cell layer. By applying this method to mouse plantar epidermal sections, the present inventors succeeded in identifying about 100 genes showing layer-specific expression in the intact epidermis. From these genes, the present inventors identified and characterized dermokine-α and -β, which are two splicing variants of a novel gene for secreted peptides expressed specifically in the spinous layer of the epidermis. Searches for these sequences in genomic databases revealed that the gene was located at a genomic locus encoding two other keratinocyte proteins: suprabasin (Park GT., Lim S.E., Jang S., and Morasso M.I., J. Biol. Chem., 277: 45195-45202, 2002) and keratinocyte differentiation-associated protein (Kdap) (Oomizu S., Sahuc F., Asahina K., Inamatsu M., Matsuzaki T., Sasaki M., Obara M., and Yoshizato K., Gene, 256: 19-27, 2000). The present inventors showed that suprabasin and Kdap were also secreted peptides, and that dermokine-α/-β, suprabasin, and Kdap were primarily expressed in the stratified epithelia. These findings revealed the existence of a novel stratified epithelium-related gene cluster, tentatively named SSC (stratified epithelium secreted peptide complex).

Further, the present inventors found that when expressed in keratinocytes, dermokine-α had the activity of differentiating keratinocytes into stratified epithelial cells.

The above results indicate that the four secreted proteins, dermokine-α and -β, Kdap, and suprabasin, form a novel gene complex which was named SSC; that they are expressed at the onset of stratification; and that they are involved in the induction of keratinocyte differentiation.

Although GenBank AY444557 and AY444558 disclose sequences similar to the dermokine-α/-β gene nucleotide sequences, the nucleotide sequences differ from the dermokine-α/-β genes described in the present specification, and their activities have not been clarified either.

Thus, the present invention relates to novel genes involved in epidermal differentiation, and gene complexes comprising the genes. More specifically, the invention provides the following:
(1) a polynucleotide of any one of (a) to (d):
   (a) a polynucleotide encoding a polypeptide comprising an amino acid sequence of any one of SEQ ID NO: 2, 4, 6, or 8;
   (b) a polynucleotide comprising the coding region of a nucleotide sequence of any one of SEQ ID NO: 1, 3, 5, or 7;
   (c) a polynucleotide encoding a polypeptide having an activity of causing a keratinocyte to differentiate into a stratified epithelial cell when expressed in the keratinocyte, wherein the polypeptide comprises an amino acid sequence with a substitution, deletion, insertion, and/or addition of one or more amino acids in the amino acid sequence of any one of SEQ ID NO: 2, 4, 6, or 8; and
   (d) a polynucleotide encoding a polypeptide having an activity of causing a keratinocyte to differentiate into a stratified epithelial cell when expressed in the keratinocyte, wherein the polynucleotide hybridizes under stringent conditions with a DNA comprising a nucleotide sequence of any one of SEQ ID NO: 1, 3, 5, or 7;
(2) the polynucleotide of (1) that is a gene involved in keratinocyte differentiation or proliferation, wherein the polynucleotide encodes a secreted protein;
(3) a polypeptide encoded by the polynucleotide of (1) or (2);
(4) a vector into which the polynucleotide of (1) or (2) is inserted;
(5) a host cell carrying the polynucleotide of (1) or (2) or the vector of (4);
(6) a method for producing the polypeptide of (3), comprising the steps of culturing the host cell of (5), and recovering a produced polypeptide from the host cell or its culture supernatant;
(7) a gene complex related to keratinocyte differentiation or proliferation, comprising each of (1) a Kdap gene; (2) a dermokine-α gene; (3) a dermokine-β gene; and (4) a suprabasin gene, wherein regulation of gene expression is commonly controlled;
(8) a polynucleotide that hybridizes specifically with the polynucleotide of (1) or (2), wherein the polynucleotide has a chain length of at least 15 nucleotides;
(9) an antisense polynucleotide against the polynucleotide of (1) or (2), or a portion thereof;
(10) an antibody that binds to the polypeptide of (3);
(11) an agent for inducing keratinocyte differentiation, comprising a dermokine-α protein or a dermokine-β protein as an active ingredient;
(12) an inhibitor of keratinocyte differentiation, comprising a compound selected from the group of (a) to (d):
   (a) an antisense nucleic acid against a transcript of a dermokine-α gene or a dermokine-β gene;
   (b) a nucleic acid having a ribozyme activity that specifically cleaves a transcript of a dermokine-α gene or a dermokine-β gene;
   (c) a nucleic acid having an effect of inhibiting the expression of a dermokine-α gene or a dermokine-β gene through an RNAi effect; and
   (d) an antibody that binds to a dermokine-α protein or a dermokine-β protein;
(13) a method of screening for an inducer of keratinocyte differentiation, comprising steps (a) to (c):
   (a) contacting a cell that expresses a dermokine-α protein or a dermokine-β protein with a test compound;
   (b) measuring an expression level or an activity of a dermokine-α protein or a dermokine-β protein in the cell; and
   (c) selecting a compound that increases the above expression level or activity, compared to in the absence of contact with the test compound;
(14) a method of screening for an inhibitor of keratinocyte differentiation, comprising steps (a) to (c):
   (a) contacting a cell that expresses a dermokine-α protein or a dermokine-β protein with a test compound;
   (b) measuring an expression level or an activity of a dermokine-α protein or a dermokine-β protein in the cell; and
   (c) selecting a compound that decreases the above expression level or activity, compared to in the absence of contact with the test compound;
(15) a method of screening for an inducer of keratinocyte differentiation, comprising steps (a) to (c):
   (a) coexisting a test compound and a keratinocyte with a dermokine-α protein, a dermokine-β protein, or a cell expressing these proteins;
   (b) measuring the differentiation of a keratinocyte into a stratified epithelial cell; and
   (c) selecting a compound that increases differentiation into a stratified epithelial cell, compared to in the absence of the test compound;
(16) a method of screening for an inhibitor of keratinocyte differentiation, comprising steps (a) to (c):
   (a) coexisting a test compound and a keratinocyte with a dermokine-α protein, a dermokine-β protein, or a cell expressing these proteins;
   (b) measuring the differentiation of a keratinocyte into a stratified epithelial cell; and
   (c) selecting a compound that decreases differentiation into a stratified epithelial cell, compared to in the absence of the test compound;
(17) a method for examining whether or not a subject cell is a cancer cell derived from a stratified epithelium, comprising steps (a) and (b):
   (a) measuring an expression level or an activity of a dermokine-α protein or a dermokine-β protein in a subject cell; and
   (b) determining that the subject cell is a cancer cell derived from a stratified epithelium when the above expression level or activity is different from a control;
(18) a method for diagnosing a squamous epithelial cancer or a basal cell cancer in a subject, comprising steps (a) and (b):
   (a) determining whether or not a cell sample prepared from a subject is a cancer cell derived from a stratified epithelium, using the method of (17); and
   (b) determining that the subject is affected with a squamous epithelial cancer or a basal cell cancer when the cell sample is determined to be a cancer cell derived from a stratified epithelium in the above step;
(19) a method for diagnosing a skin disease in a subject, comprising steps (a) and (b):
   (a) measuring an expression level or activity of a dermokine-α protein or a dermokine-β protein in a test sample prepared from a subject; and
   (b) determining that the subject is affected with a skin disease when the above expression level or activity is different from a control;
(20) the method for diagnosing of (19), wherein the skin disease is a xeroderma, psoriasis, or ichthyosis;
(21) a method for treating a disease caused by reduced induction of keratinocyte differentiation, comprising the step of administering a dermokine-α protein or a dermokine-β protein;
(22) a method for treating a disease caused by reduced induction of keratinocyte differentiation, comprising the step of administering an inducer of keratinocyte differentiation obtained by the screening method of (13) or (15);
(23) a method for treating a disease caused by an increase in keratinocyte differentiation, comprising the step of administering a compound selected from the following group (a) to (d):
   (a) an antisense nucleic acid against a transcript of a dermokine-α gene or a dermokine-β gene;
   (b) a nucleic acid having a ribozyme activity that specifically cleaves the transcript of a dermokine-α gene or a dermokine-β gene;
   (c) a nucleic acid having an effect of inhibiting the expression of a dermokine-α gene or a dermokine-β gene through an RNAi effect; and
   (d) an antibody that binds to a dermokine-α protein or a dermokine-β protein;
(24) a method for treating a disease caused by an increase in keratinocyte differentiation, comprising the step of administering an inhibitor of keratinocyte differentiation obtained by the screening method of (14) or (16);
(25) the treatment method of (21) or (22), wherein the disease caused by reduced induction of keratinocyte differentiation is psoriasis vulgaris or solar keratosis;
(26) the method of (23) or (24), wherein the disease caused by an increase in keratinocyte differentiation is ichthyosis vulgaris accompanied by excessive keratinization, X-linked ichthyosis, common wart, lichen amyloidosis, lichen planus accompanied by hypergranulosis, or bullous typed ichthyosiform erythroderma;
(27) a use of a dermokine-α protein or a dermokine-β protein for producing a drug for treating a disease caused by reduced induction of keratinocyte differentiation;
(28) a use of an inducer of keratinocyte differentiation, obtained by the screening method of (13) or (15), for producing a drug for treating of a disease caused by reduced induction of keratinocyte differentiation;
(29) a use of a compound for producing a drug for treating of a disease caused by an increase in keratinocyte differentiation, wherein the compound is selected from the following group of (a) to (d):
   (a) an antisense nucleic acid for a transcript of a dermokine-α gene or a dermokine-β gene;
   (b) a nucleic acid having a ribozyme activity that specifically cleaves a transcript of a dermokine-α gene or a dermokine-β gene;
   (c) a nucleic acid having an activity of inhibiting the expression of a dermokine-α gene or a dermokine-β gene through an RNAi effect; and
   (d) an antibody that binds to dermokine-α protein or a dermokine-β protein;
(30) a use of an inhibitor of keratinocyte differentiation, obtained by the screening method of (14) or (16), for producing a drug for treating a disease caused by an increase in keratinocyte differentiation;
(31) the use of (27) or (28), wherein the disease caused by reduced induction of keratinocyte differentiation is psoriasis vulgaris or solar keratosis; and
(32) the use of (29) or (30), wherein the disease caused an increase in keratinocyte differentiation is ichthyosis vulgaris accompanied by excessive keratinization, X-linked ichthyosis, common wart, lichen amyloidosis, lichen planus accompanied by hypergranulosis, or bullous typed ichthyosiform erythroderma.

### Brief Description of the Drawings

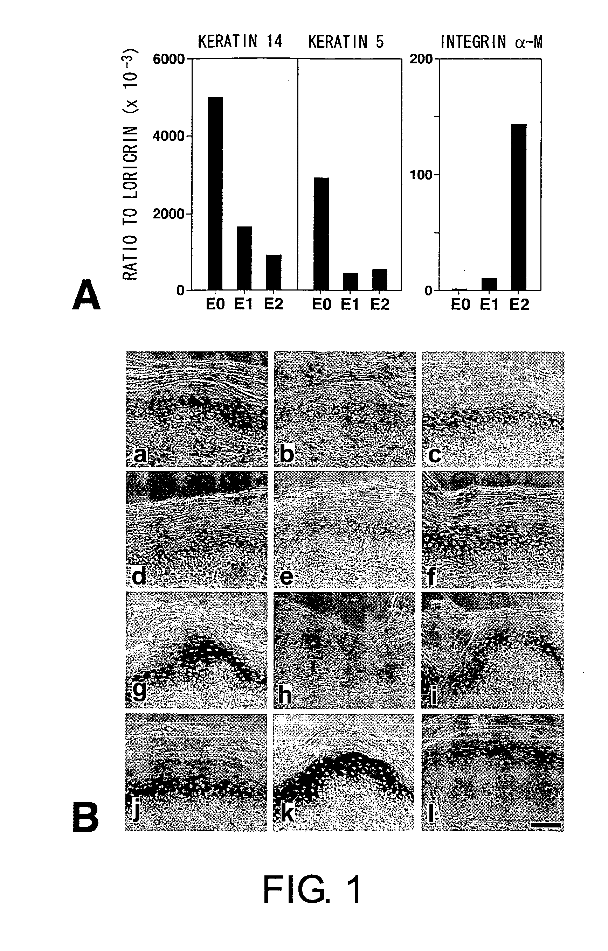

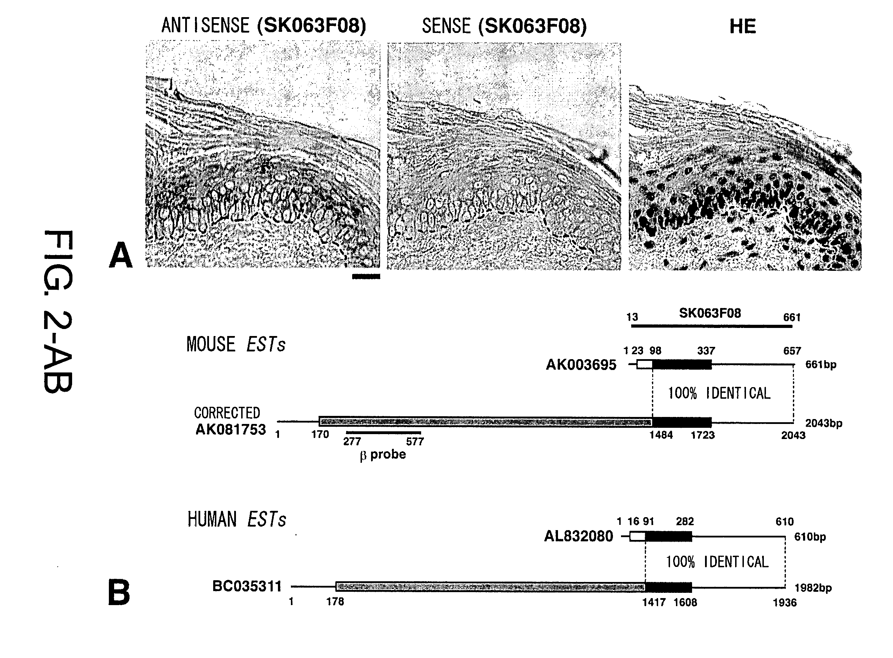

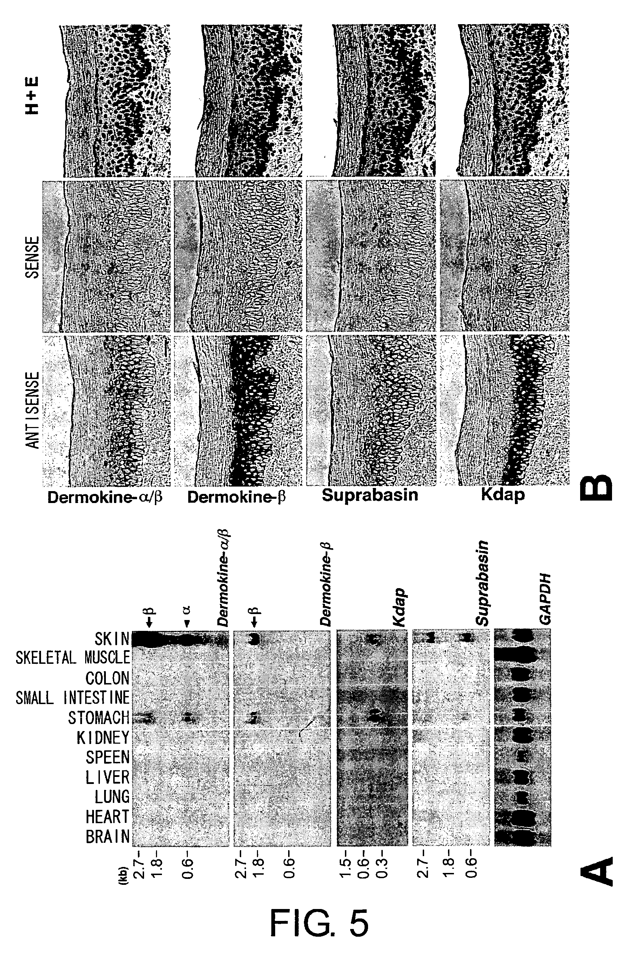

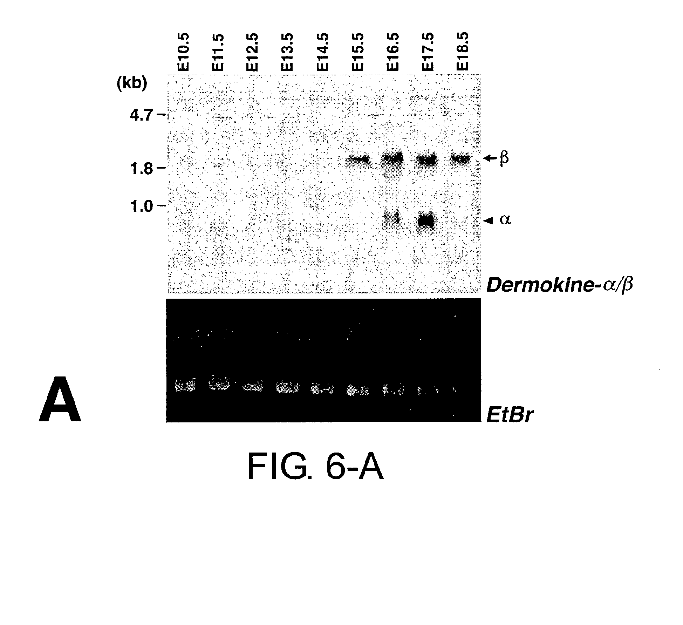

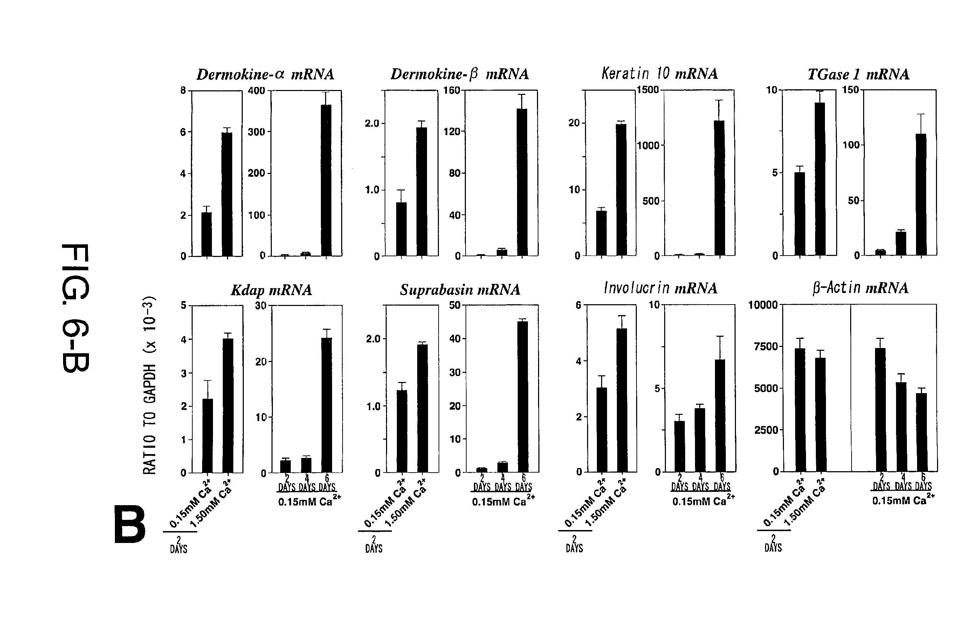

Fig. 1 shows diagrams and photographs indicating the results of *high-throughput in situ* hybridization (HT-ISH).
   A: Results of evaluating an equalized mouse back skin cDNA library. The starting library (E0) and the equalized libraries (E1 and E2) were analyzed in duplicate using quantitative real-time PCR. All data were normalized to an internal standard, loricrin. Compared to E0, in E2 both keratin 14 and keratin 5 cDNAs (abundant cDNAs) were reduced to about one-fifth; however, integrin α-M cDNAs (rare cDNAs) were increased by about 42 times.
   B: Photographs showing examples of HT-ISH signals. cRNA probes prepared from the equalized cDNA library (E2) were hybridized to sections of adult mouse food pad epidermis using a 96-well format. (a) Cyclophilin A (NM008907) was expressed specifically in the basal and suprabasal layers. (b) Kdap was detected in the suprabasal cell layer. (c) Prothymosin α (NM008972), (d) diazepam-binding inhibitor (BC028874), (e) a gene similar to Tubulin β-2 chain (XM130158), (f) lamin A (BC015302), (g) nuclear distribution C homologue (NM010948), (h) desmoplakin (XM138593), (i) keratin-associated protein 16-5 (NM130857), and (j) nucleoside diphosphate kinase C (AF288691) were detected in the spinous and granular cell layers (k). (1) Loricrin (U09189) mRNAs were mostly detected in only the granular layer. Specific signals were not detected for any of the clones when using the sense probe (data not shown). Dashed lines represent the border between the epidermis and the dermis. Scale bar: 50 µm.

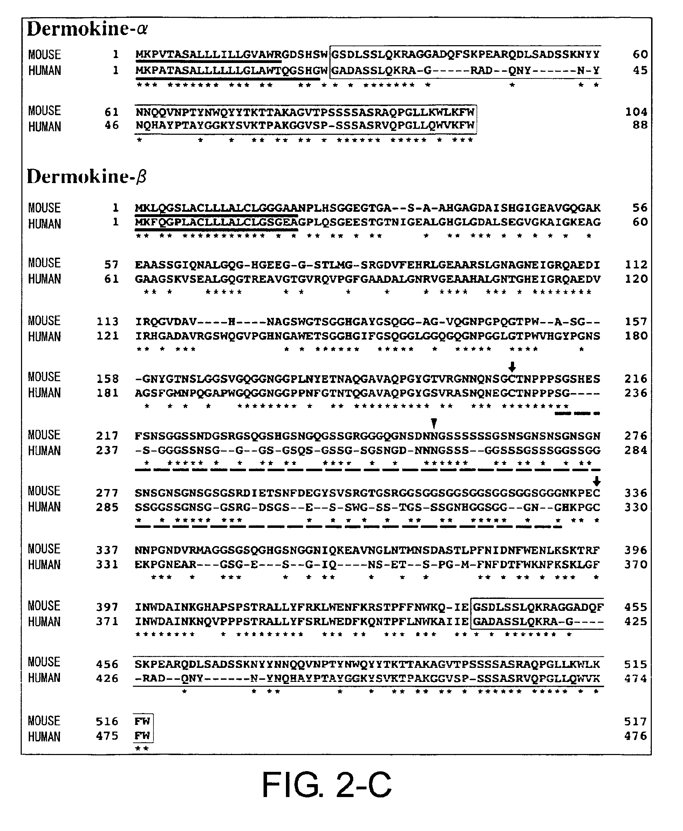

Fig. 2-AB shows a diagram and photographs showing the identity of dermokine-α and -β.
   A: A photograph showing HT-ISH signals obtained with the SK063F08 probe for sections of adult mouse foot pad epidermis. The antisense SK063F08 probe gave intense signals in the spinous layer of epidermis (antisense), whereas no signal was detected with the sense probe (sense). H+E is an image of hematoxylin-eosin staining. Dashed lines represent the border between the epidermis and the dermis. Scale bar: 50 µm.
   B: A diagram showing mouse ESTs comprising the SK063F08 sequence and their human homologue ESTs. A mouse EST comprising the entire SK063F08 sequence, and a mouse EST (AK081753) sharing the same sequence as AK003695 at its 3'-end region (98 to 661 bp in AK003695, and 1505 to 2064 bp in AK081753) suggest that these ESTs are splicing variants. Human homologues of AK003695 and AK081753 were found as two ESTs, AL832080 and BC035311, respectively. The ORFs are shown as boxes. Aβ probe was used for Northern blotting and *in situ* hybridization (see Figs. 5 and 6). A single nucleotide insertion (guanine) was found at position 743 of the AK081753 sequence. Further, a 27 bp insertion between 1132 bp and 1133 bp, and a deletion of 1197-1244 bp were confirmed.
Fig. 2-C shows a diagram indicating the identity of dermokine-α and -β.
   This shows the amino acid sequences of mouse and human dermokine-α and -β, deduced from each of the ESTs of AK003695/AL832080 and AK081753/BC035311. Putative signal sequences predicted by SignalP server are underlined, and regions identical in both dermokine-α and -β are boxed. The glycine- and serine-rich domain in dermokine-β is indicated by a dashed line. The sequence data of dermokine-α and dermokine-β are deposited with GenBank under accession numbers AK003695 and AK081753 for mice, and AL832080 and BC035311 for humans, respectively.

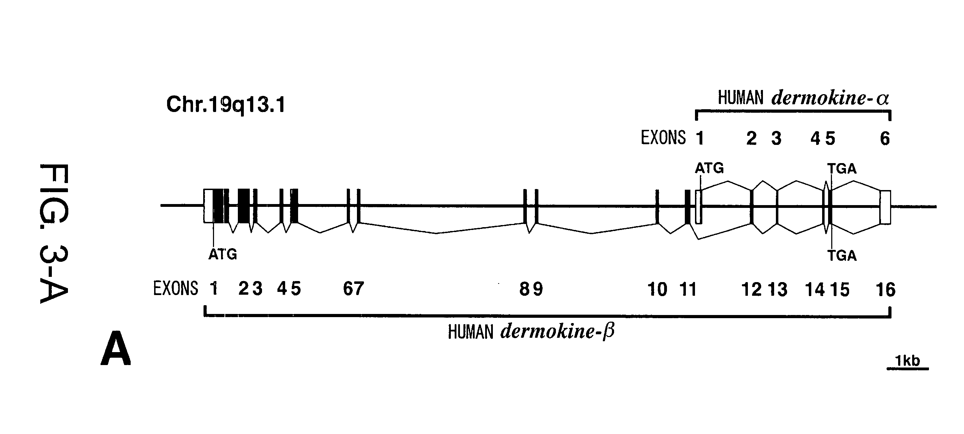

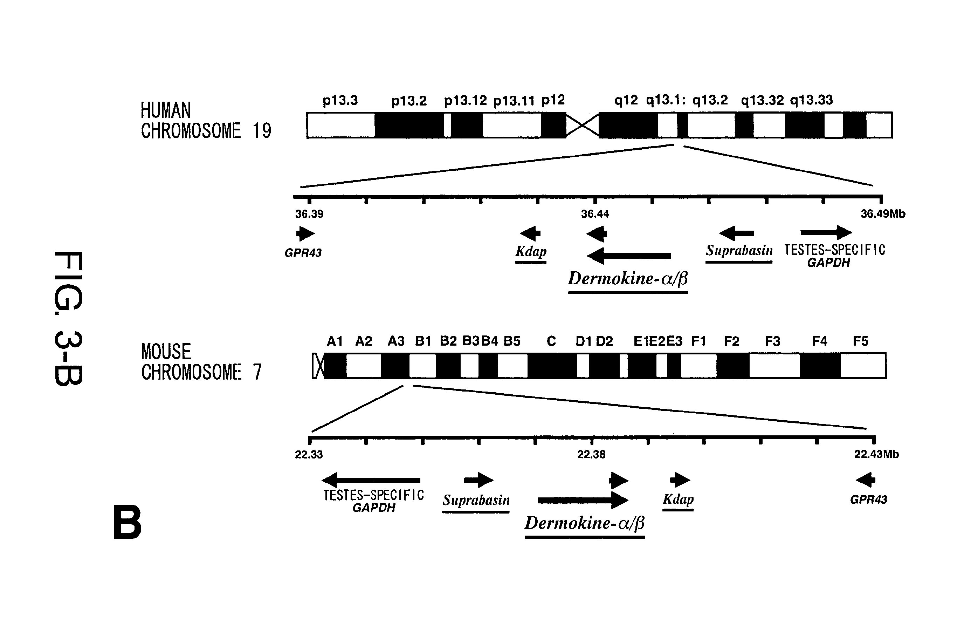

Fig. 3-A shows a diagram indicating the structure of a dermokine-α and -β gene.
   This shows the exons of a human dermokine-α and -β gene on chromosome 19q13.1. Open and closed boxes represent the exons for coding and non-coding regions of the mRNA, respectively. The putative translation start and termination sites are shown as ATG and TGA, respectively. Exons 2 to 6 of dermokine-α are utilized in dermokine-β as exons 12 to 16, and their carboxyl-terminal sequences and 3'-non-coding regions are identical.
Fig. 3-B shows a diagram indicating the structure of the dermokine-α and -β gene.
   This shows mouse and human loci comprising genes for suprabasin, dermokine-α/-β, and keratinocyte differentiation-associated protein (Kdap). Arrows indicate the direction of transcription. Testis-specific GAPDH: testis-specific glyceraldehyde-3-phosphate dehydrogenase gene; GPR43: G-protein-coupled receptor 43 gene.

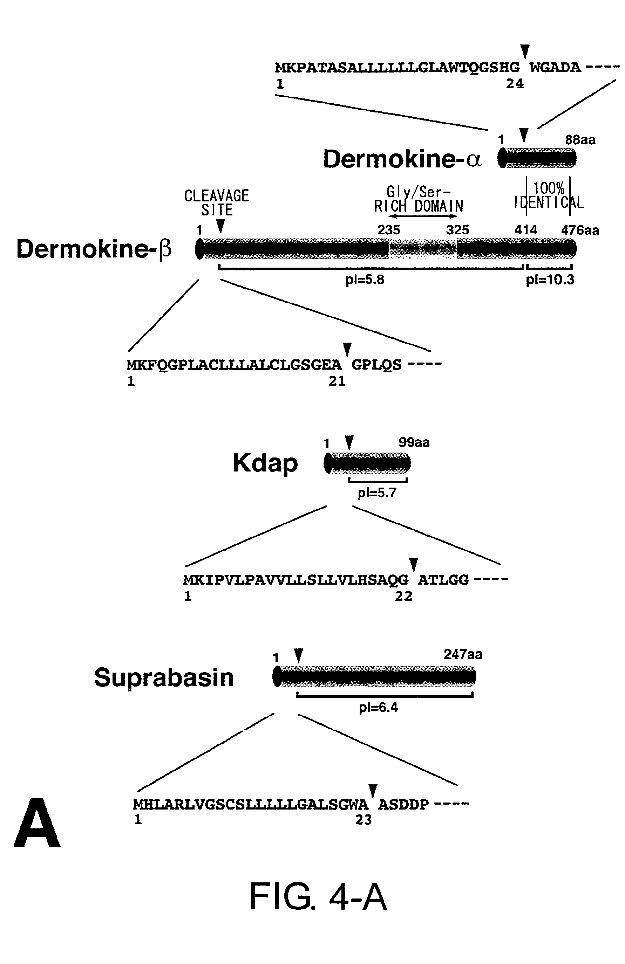

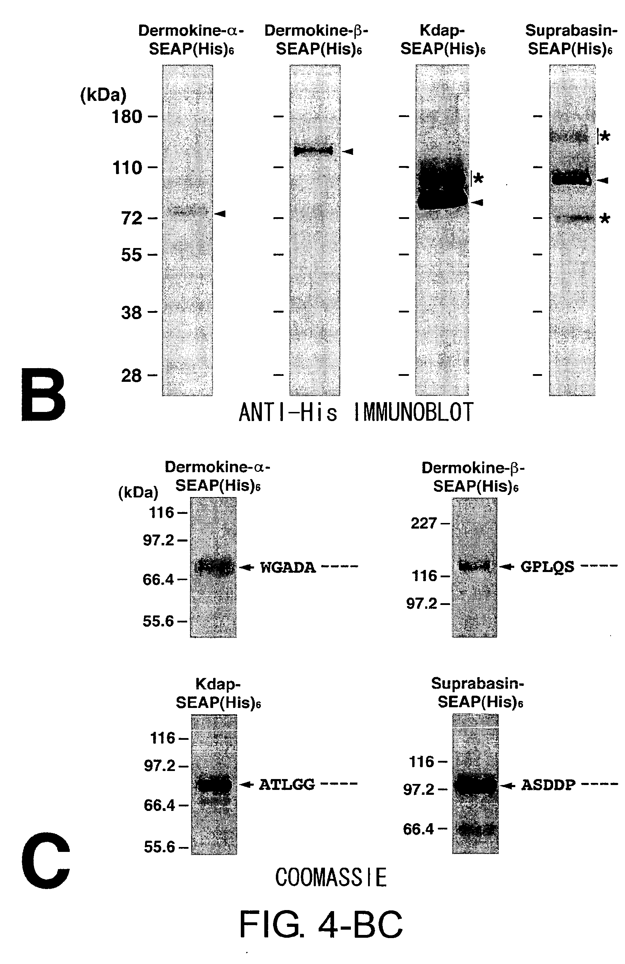

Fig. 4-A shows a schematic representation of putative signal peptides at the N-termini of human dermokine-α/-β, Kdap, and suprabasin (0.7-kb transcript).
   Arrowheads represent the predicted cleavage sites. The p*I* values were calculated from the amino acid residues comprised. Note that the predicted secreted form of dermokine-α and the corresponding C-terminal region of dermokine-β showed a fairly high p*I* value (p*I* 10.3).
Fig. 4-BC shows photographs indicating the results of analysis of the secretion of dermokine-α and -β, Kdap, and suprabasin.
B: The photographs show the four SEAP(His)₆ fusion proteins: dermokine-α-SEAP(His)₆, dermokine-β-SEAP(His)₆, Kdap-SEAP(His)₆, and suprabasin-SEAP(His)₆, in 293/EBNA-1 cells. Dermokine-α, dermokine-β, Kdap, and suprabasin were fused to the N-terminus of a secreted alkaline phosphatase (SEAP) that lacked its own signal peptide, in addition to a C-terminal (His)₆ tag. These fusion proteins carrying the predicted signal sequences of dermokine-α and -β, Kdap, and suprabasin (and also SEAP(His)₆ as a control) were transiently expressed in 293/EBNA-1 cells. The culture medium (medium) was separated by SDS-PAGE (10 µg for each lane) and subjected to immunoblotting with anti-His antibody. Fusion proteins secreted into the medium are marked with arrowheads. All four of fusion proteins were secreted into the medium. The secreted Kdap-SEAP(His)₆ was detected as a high molecular mass band (asterisk).
C: A photograph of dermokine-α-SEAP(His)₆, dermokine-β-SEAP(His)₆, Kdap-SEAP(His)₆, and suprabasin-SEAP(His)₆ purified from the culture medium. 293/EBNA-1 cell culture media comprising secreted fusion proteins were collected, and fusion proteins were purified using TALON metal affinity resin. Purified proteins (arrows) were resolved by SDS-PAGE, followed by Coomassie brilliant blue staining and the N-terminal amino acid sequences were analyzed. As predicted in A, the N-terminal amino acid sequences of secreted dermokine-α-SEAP(His)₆, dermokine-β-SEAP(His)₆, Kdap-SEAP(His)₆, and suprabasin-SEAP(His)₆ were determined to be WGADA, GPLQS, ATLGG, and ASDDP, respectively.
Fig. 5 shows photographs indicating expression patterns of dermokine-α and -β, Kdap, and suprabasin in tissues.
   A: Photographs showing the results of Northern blotting. Nylon membranes onto which total RNAs (20 µg) from mouse tissues have been blotted were probed with DIG-labeled cRNA fragments specific to each gene. The SK063F08 probe, which hybridizes with both dermokine-α (arrowhead) and the corresponding carboxyl-terminal region of dermokine-β (arrow), formed two bands (Dermokine-α/-β), whereas the β probe, which specifically hybridizes to dermokine-β, formed only one band (Dermokine-β) (see Fig. 2B). Membranes were hybridized with a Kdap-specific probe (Kdap), suprabasin-specific probe (Suprabasin), and control GAPDH-specific probe (GAPDH). All the genes were expressed abundantly in the skin and at low levels in the stomach. Kdap was also detected in the lungs.
   B: Photographs showing expression patterns of dermokine-α and -β, Kdap, and suprabasin within the epidermis. Serial sections of mouse foot pad epidermis were hybridized with antisense (antisense) and sense (sense) probes specific to dermokine-β (SK063F08; Dermokine-α/-β), dermokine-β (β probe; Dermokine-β), Kdap (Kdap), and the dermokine-α/C-terminal region of suprabasin. Hematoxylin-eosin staining of sections hybridized with sense probes is shown (H+E). Dermokine-α/-β-, dermokine-β-, and suprabasin-specific hybridization signals were equally detected in the spinous layer of mouse foot pad epidermis. However, Kdap-specific signals were restricted to the suprabasal region of the spinous layer. No specific signals were detected in sections hybridized with the sense probes. Dashed lines represent the border between the epidermis and the dermis. Scale bar: 50 µm.
Fig. 6-A shows photographs of the products of SSC.
   These are photographs showing expression of dermokine-α and -β during mouse embryonic development. A Mouse Embryo Full Stage Blot (Seegene) was probed with DIG-labeled cRNA fragments of SK063F08. Dermokine-β (arrow) began to be expressed at E15.5. Dermokine-α (arrowhead) first appeared at E16, and its expression increased up to E17.5 and had decreased by E18.5. The bottom panel shows an ethidium bromide-stained membrane.
Fig. 6-B shows a graph indicating *in vitro* differentiation of primary cultured human keratinocytes.
   Human primary keratinocytes were cultured under different Ca²⁺ concentrations (0.15 mM and 1.50 mM Ca²⁺) for two days, or at different cell densities (two, four and six days) under a 0.15 mM Ca²⁺ concentration. Differentiation was induced by increasing the Ca²⁺ concentration (from 0.15 mM to 1.50 mM) or by decreasing the cell density (from two to six days) at 0.15 mM Ca²⁺. Total RNAs were prepared from these cells, and SYBR green-based qRT-PCR analysis was performed for dermokine-α and -β, Kdap, suprabasin, and differentiation-specific genes (keratin 10, involucrin, loricrin, and transglutaminase 1), using specific primers and the control β-actin gene. All data were normalized to an internal GAPDH mRNA control. As for differentiation-specific genes, the dermokine-α and -β, Kdap, and suprabasin genes were significantly upregulated upon induction of differentiation. Transcription of the control β-actin gene was not induced. Bars represent the standard deviations of three independent experiments done in duplicate.

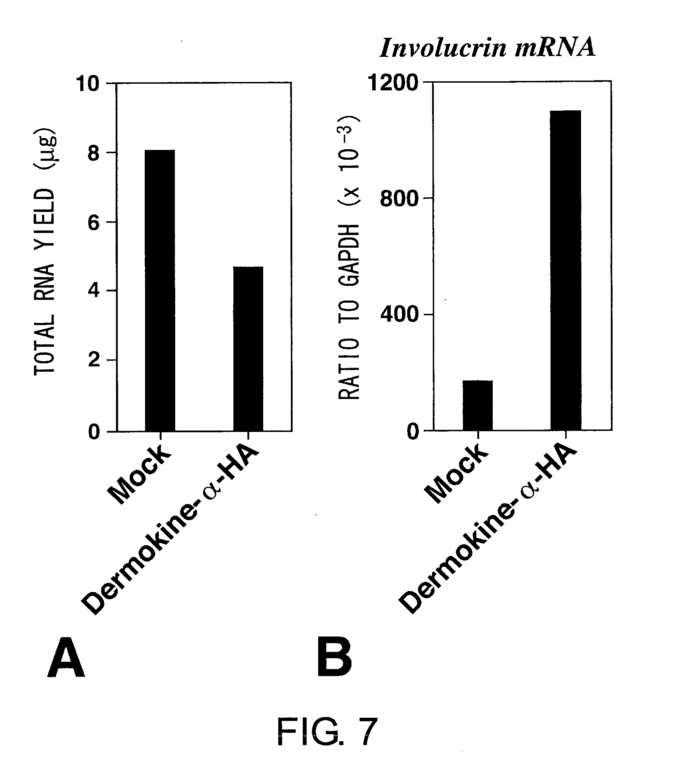

Fig. 7 shows a diagram indicating the effect of dermokine-α on keratinocyte differentiation.
   A: A plot showing the yield of total RNA recovered from a 12-well plate. Keratinocytes were seeded at 2500 cells/cm² in a 12-well plate, and made to express pcDNA-human dermokine-α-HA using TransIT LT1 keratinocyte. pcDNA3.1 only was used for the Mock. On extracting total RNAs, the yield of total RNAs was significantly decreased in keratinocytes which were made to express human dermokine-α-HA.
   B: A plot showing the expression of involucrin. A qRT-PCR for involucrin mRNAs was performed using the total RNAs prepared in A. Expression of dermokine-α significantly increased involucrin mRNAs.

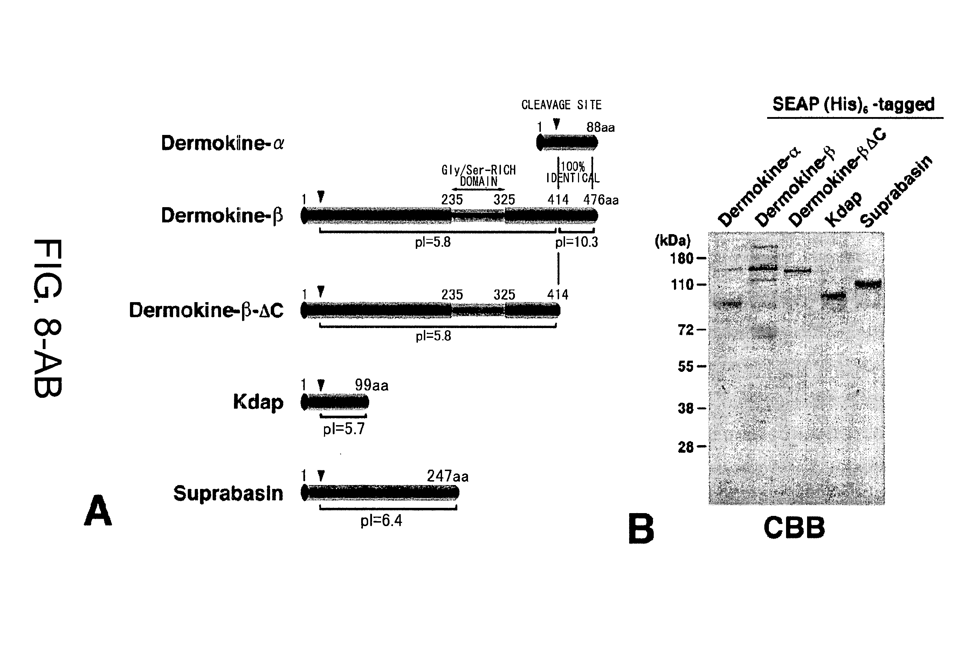

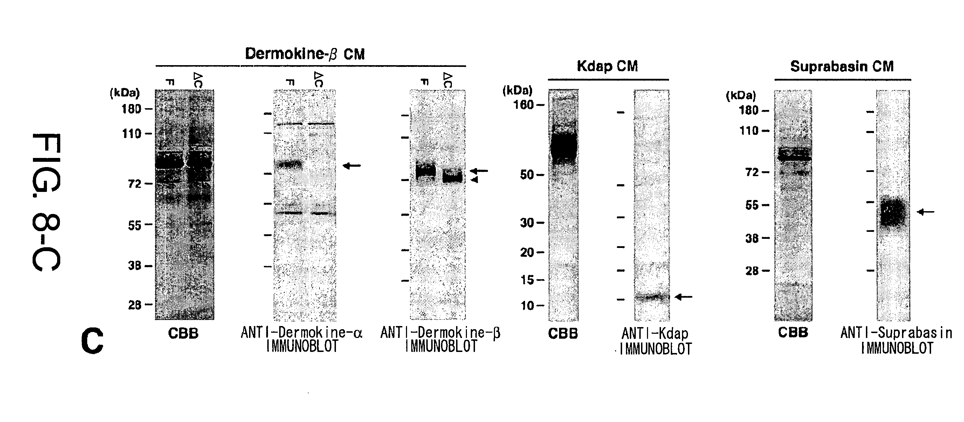

Fig. 8-AB shows a photograph and diagrams indicating the generation of antibodies against the SSC protein group.
   A: Diagram of constructs used for antigen generation. Dermokine-β-ΔC, in which the region shared between dermokine-α and -β has been deleted, was used to generate dermokine-(β-specific antibodies.
   B: A photograph showing the proteins used as antigens. Fusion proteins of dermokine-α, dermokine-β-ΔC, Kdap, and suprabasin with SEAP(His)₆ which were transiently expressed in 293/EBNA-1 cells were used as antigens. The diagram is an image of a CBB staining.
Fig. 8-C shows photographs indicating the generation of antibodies against the SSC protein group.
   These are photographs showing immunoblots using antisera against the SSC protein group. Dermokine-α, dermokine-β, dermokine-β-ΔC, Kdap, and suprabasin were each transiently expressed in 293/EBNA-1 cells, and culture supernatants were prepared. These were subjected to electrophoresis, transferred onto nitrocellulose membranes, and subjected to immunoblotting using antisera against each protein. Images of CBB staining of culture supernatants are also shown. The antibody against dermokine-α did not react with dermokine-β-ΔC.
Fig. 9 shows photographs indicating the results of using CBB (Coomassie Brilliant Blue) and immunoblotting to analyze human and mouse dermokine-β and -β-ΔC expressed in 293 cells. The left and right hand panels show human and mouse results, respectively.
Fig. 10 shows graphs indicating the results of detecting the alkaline phosphatase activity of human and mouse dermokine-β-SEAP(His)₆ or dermokine-β-ΔC-SEAP(His)₆. A and B show the results for mice and humans, respectively.
Fig. 11 shows photographs indicating the results of silver nitrate-staining and the results of immunoblotting using anti-hDK mAb after pcDNA3.1-human dermokine-β and pcDNA3.1-human dermokine-β-ΔC were transfected into 293/EBNA-1 cells and SDS-PAGE was performed.

### Best Mode for Carrying Out the Invention

The present invention relates to novel secreted proteins expressed in the suprabasal layer of differentiated epithelia, and polynucleotides encoding these proteins. The proteins of the present invention are considered to be involved in the differentiation and/or proliferation of keratinocytes.

The present inventors named the two types of novel protein identified in the present invention "dermokine-α" and "dermokine-β". The nucleotide sequence of a mouse dermokine-α gene and the amino acid sequence of a protein encoded by this gene are shown in SEQ ID NOs: 1 and 2, respectively. The nucleotide sequence of a mouse dermokine-β gene and the amino acid sequence of a protein encoded by this gene are shown in SEQ ID NOs: 3 and 4, respectively. Further, the nucleotide sequence of a human dermokine-α gene and the amino acid sequence of a protein encoded by this gene are shown in SEQ ID NOs: 5 and 6, respectively. The nucleotide sequence of a human dermokine-β gene and the amino acid sequence of a protein encoded by this gene are shown in SEQ ID NOs: 7 and 8, respectively.

The present invention first provides any of the following polynucleotides:
(a) polynucleotides encoding polypeptides comprising an amino acid sequence of any one of SEQ ID NO: 2, 4, 6, or 8;
(b) polynucleotides comprising coding regions of a nucleotide sequence of any one of SEQ ID NO: 1, 3, 5, or 7;
(c) polynucleotides encoding polypeptides comprising amino acid sequences with one or more amino acids substitution, deletion, insertion, and/or addition in the amino acid sequence of any one of SEQ ID NO: 2, 4, 6, or 8; and
(d) polynucleotides that hybridize under stringent conditions with DNAs comprising the nucleotide sequence of any one of SEQ ID NO: 1, 3, 5, or 7.

In a preferred embodiment of the present invention, the above (c) or (d) are the following polynucleotides:
(c) polynucleotides encoding polypeptides comprising amino acid sequences with one or more amino acids substitution, deletion, insertion, and/or addition in the amino acid sequence of any one of SEQ ID NO: 2, 4, 6, or 8, wherein the polypeptides have the activity of inducing the differentiation of keratinocytes into stratified epithelial cells when expressed in the keratinocytes; or
(d) polynucleotides that hybridize under stringent conditions with DNAs comprising the nucleotide sequence of any one of SEQ ID NO: 1, 3, 5, or 7, wherein the polynucleotides encode polypeptides having the activity of inducing the differentiation of keratinocytes into stratified epithelial cells when expressed in the keratinocytes.

Those skilled in the art can appropriately determine whether an arbitrary polypeptide has "the activity of inducing the differentiation of keratinocytes into stratified epithelial cells when expressed in the keratinocytes" as in the above (c) or (d). For example, this can be determined by introducing a test polypeptide into keratinocytes, and using the expression of a marker for the differentiation of the stratified epithelium, preferably involucrin, as an index. For example, if the expression of the above-described involucrin increases, the test polypeptide is judged to have the "activity of inducing the differentiation of keratinocytes into stratified epithelial cells when expressed in the keratinocytes".

Furthermore, in a preferred embodiment of the present invention, the polynucleotides are polynucleotides of any one of the above (a) to (d) that encode a secreted protein and are genes that are involved in the differentiation and/or proliferation of keratinocytes.

In a preferred embodiment, the present invention provides the above mouse or human dermokine-α and dermokine-β proteins, and their altered forms (mutants).

More specifically, the present invention provides polypeptides comprising the amino acid sequence of any one of SEQ ID NO: 2, 4, 6, or 8, and polypeptides functionally equivalent to dermokine-α or dermokine-β proteins, in which the polypeptides comprise amino acids with one or more amino acids substitution, deletion, insertion and/or addition in said amino acid sequences.

The present invention relates to the polypeptides identified by the present inventors, polypeptides functionally equivalent to these polypeptides, and polynucleotides encoding these polypeptides. Herein, "functionally equivalent" means that a subject polypeptide has a biological function (activity) that is equivalent to that of a polypeptide identified by the present inventors (for example, a polypeptide comprising the amino acid sequence of any one of SEQ ID NO: 2, 4, 6, or 8).

An example of biological functions (activities) that the polypeptides of the present invention have is expression in the suprabasal layer of the differentiated epithelia, or involvement in the differentiation and/or proliferation of keratinocytes. Another example is the activity of inducing keratinocyte differentiation. Further examples comprise the function for specific expression in the stratified epithelium, and the function of participating in the *in vivo* stratification of the epidermis during early development of mice. Other than these, examples comprise the functions (activities) described in the Examples below for dermokine-α and -β of the present invention.

Therefore, the present invention comprises polynucleotides encoding proteins that comprise an amino acid sequence with a substitution, deletion, insertion, and/or addition of one or more amino acids in the amino acid sequence of any one of SEQ ID NO: 2, 4, 6, or 8, and that are expressed in the suprabasal layer of the differentiated epidermis or involved in the differentiation and/or proliferation of keratinocytes. Further, the present invention also comprises polynucleotides that hybridize under stringent conditions with DNAs comprising the nucleotide sequence of any one of SEQ ID NO: 1, 3, 5, or 7, and which encode proteins that are expressed in the suprabasal layer of the differentiated epidermis or involved in the differentiation and/or proliferation of keratinocytes. These polypeptides of the present invention are preferably secreted proteins.

Whether or not a test polypeptide (protein) is a secreted protein can be evaluated by methods commonly known to those skilled in the art. For example, an expression vector for a fusion protein formed by tagging the test protein with a SEAP (secreted alkaline phosphatase)-(His)₆ tag may be constructed, and then transfected into cells. Whether or not a test protein is secreted to the culture medium of these cells can be examined by immunoblotting using an anti-His-antibody. More specifically, this can be carried out according to methods described in the Examples below.

Whether or not a test polypeptide (test gene) is expressed in the suprabasal layer of the differentiated epidermis can be appropriately determined by methods commonly known to those skilled in the art, for example, by Northern blotting, Western blotting, *in situ* hybridization, etc. In addition, whether a test polypeptide (test gene) is involved in the differentiation and/or proliferation of keratinocytes may be examined, for example, by analyzing expression of the test polypeptide (gene) under conditions of high Ca²⁺ concentration or high cell density. More specifically, verification is possible by examining the expression level by qRT-PCR using a differentiation marker for the stratified epithelium. More specifically, it can be appropriately carried out according to methods described in the Examples below.

The aforementioned polynucleotides of the present invention can be isolated by methods well known to those skilled in the art. Examples of such methods comprise hybridization technique (E.M. Southern, J. Mol. Biol. 1975, 98: 503-517) and polymerase chain reaction (PCR) technique (R.K. Saiki et al., Science 1985, 230: 1350-1354; R.K. Saiki et al., Science 1988,239: 487-491). More specifically, those skilled in the art can generally isolate polynucleotides highly homologous to the polynucleotides comprising the nucleotide sequence of any one of SEQ ID NO: 1, 3, 5, or 7 from desired animals, using the polynucleotide of any one of SEQ ID NO: 1, 3, 5, or 7 or parts thereof as probes or using the oligonucleotide which specifically hybridizes with the polynucleotide comprising the nucleotide sequence of any one of SEQ ID NO: 1, 3, 5, or 7 as primers. Furthermore, polynucleotides that can be isolated by hybridization techniques or PCR techniques and that hybridize with polynucleotides comprising the nucleotide sequence of any one of SEQ ID NO: 1, 3, 5, or 7 are also comprised in the polynucleotides of the present invention. Examples of such polynucleotides comprise homologs in mice and organisms other than humans of the gene comprising the nucleotide sequence shown in SEQ ID NO: 1, 3, 5, or 7.

Hybridization reactions to isolate polynucleotides as described above are preferably conducted under stringent conditions. Examples of stringent hybridization conditions comprise conditions comprising 6 M urea, 0.4% SDS and 0.5x SSC, and those having a stringency equivalent to these conditions. Polynucleotides with higher homology are expected to be isolated when hybridization is performed under conditions with higher stringency, for example, 6 M urea, 0.4% SDS and 0.1x SSC. The polynucleotides thus isolated are expected to have a high homology at the amino acid level to the amino acid sequence of SEQ ID NO: 2, 4, 6, or 8. Herein, "high homology" means a sequence identity of at least 70% or more, preferably 80% or more, more preferably 90% or more, and most preferably 95% or more, in the whole amino acid sequence.

The degree of identity at the amino acid sequence level or nucleotide sequence level can be determined using the BLAST algorithm by Karlin and Altschul (S. Karlin and S.F. Altschul, Proc. Natl. Acad. Sci. USA. 1990, 87: 2264-2268; S. Karlin and S.F. Altschul, Proc. Natl. Acad. Sci. USA. 1993, 90: 5873-5877). The BLAST algorithm-based programs, called BLASTN and BLASTX, have been developed (S.F. Altschul et al., J. Mol. Biol. 1990, 215: 403). When a nucleotide sequence is analyzed according to BLASTN, parameters are set, for example, at score= 100 and word length= 12. On the other hand, parameters used for the analysis of amino acid sequences by BLASTX are set, for example, at score= 50 and word length= 3. Default parameters of each program are used when BLAST and Gapped BLAST programs are used. Specific procedures for such analysis are known (http://www.ncbi.nlm.nih.gov).

Polynucleotides of the present invention comprise genomic DNAs, cDNAs, and chemically synthesized DNAs. A genomic DNA or cDNA can be prepared according to conventional methods known to those skilled in the art. For example, genomic DNA can be prepared as follows: (i) extracting genomic DNA from organisms comprising the polynucleotide of the present invention (for example the nucleotide sequence of SEQ ID NO: 1, 3, 5, or 7); (ii) constructing a genomic library (using, for example, a plasmid, phage, cosmid, BAC, or PAC, as a vector); (iii) developing the library; and then (iv) conducting colony hybridization or plaque hybridization using probes prepared based on a polynucleotide of the present invention. Alternatively, the genomic DNA can be prepared by PCR, using primers specific to a polynucleotide of the present invention (e.g., SEQ ID NO: 1, 3, 5, or 7). On the other hand, the cDNA can be prepared, for example, as follows: (i) synthesizing cDNAs based on mRNA extracted from organisms comprising the polynucleotide of the present invention; (ii) constructing a cDNA library by inserting the synthesized cDNA into vectors, such as λZAP; (iii) developing the cDNA library; and (iv) conducting colony hybridization or plaque hybridization as described above. Alternatively, the cDNA can also be prepared by PCR.

The present invention also provides polynucleotides encoding proteins structurally similar to the protein comprising the amino acid sequence of any one of SEQ ID NO: 2, 4, 6, or 8. Examples of such polynucleotides comprise polynucleotides comprising nucleotide sequences encoding proteins comprising an amino acid sequence with a substitution, deletion, insertion, and/or addition of one or more amino acids in the amino acid sequence of this protein. There is no limitation on the number and sites of the amino acid mutation in above-described proteins so long as the functions are maintained. The number of mutations may be typically 10% or less, preferably 5% or less, and more preferably 1% or less of the total amino acid residues.

More specifically, the number of amino acid mutation is normally 50 amino acids or less, preferably 30 amino acids or less, more preferably ten amino acids or less, further more preferably five amino acids or less, and most preferably three amino acids or less.

The present invention provides also polypeptides with the aforementioned functions (activities), and comprising amino acid sequences showing homology to the amino acid sequence of any one of SEQ ID NO: 2, 4, 6, or 8. The level of "homology" is not limited as long as the polypeptide has an aforementioned function (activity); however, it is normally 60% or more, preferably 70% or more, more preferably 80% or more, more preferably 85% or more, further more preferably 90% or more, and most preferably 95% or more.

Exemplary methods well known to those skilled in the art for preparing a polynucleotide as described above comprise site-directed mutagenesis (W. Kramer and H-J. Fritz, Methods Enzymol. 1987, 154: 350) for introducing mutations in the polynucleotide, in addition to the hybridization and PCR techniques described above. The amino acid sequence of a protein may also be mutated in nature due to a mutation of the nucleotide sequence encoding the protein. Additionally, degeneracy mutant polynucleotides, in which nucleotide sequence mutations do not give rise to any amino acid sequence mutations in the protein (degeneracy mutants), are also comprised in the present invention.

The present invention also provides fragments of the polypeptides of the present invention. Such fragments are polypeptides comprising amino acid sequences that are identical to a portion, but not to the entirety of, an amino acid sequence of an aforementioned polypeptide of the present invention. The polypeptide fragments of the present invention normally comprise a sequence of eight amino acid residues or more, and preferably twelve amino acid residues or more (for example, 15 amino acid residues or more). Preferable examples of such fragments are truncation polypeptides comprising an amino acid sequence in which (i) a series of residues comprising the amino-terminal or a series of residues comprising the carboxyl-terminal has been deleted, or (ii) both the series of residues comprising the amino-terminal and the series of residues comprising the carboxyl-terminal have been deleted. Also preferable are fragments comprising a structural or functional characteristic, such as an α-helix and α-helix-forming region, a β-sheet and β-sheet-forming region, a turn and turn-forming region, a coil and coil-forming region, a hydrophilic region, a hydrophobic region, an α-amphipathic region, a β-amphipathic region, a variable region, a surface-forming region, a substrate-binding region, and a region with a high antigenicity index. Mutated forms of identified sequences and fragments also constitute a part of the present invention. Preferable mutations are those that involve substitution of amino acids of a same type. That is, a mutation in which a residue is substituted by another residue having similar properties. Such substitutions typically occur, for example, between Ala, Val, Leu, and Ile; between Ser and Thr; between the acidic residues Asp and Glu; between Asn and Gln; between the basic residues Lys and Arg; or between the aromatic residues Phe and Tyr.

In terms of maintaining protein function, substituted amino acids in the present invention are preferably those having properties similar to the amino acids prior to substitution. For example, Ala, Val, Leu, Ile, Pro, Met, Phe, and Trp are all categorized as non-polar amino acids, and are thus expected to have similar properties. Examples of uncharged amino acids comprise Gly, Ser, Thr, Cys, Tyr, Asn, and Gln. Moreover, examples of acidic amino acids comprise Asp and Glu; and examples of basic amino acids comprise Lys, Arg, and His. Thus, the amino acids to be modified in the present invention are not limited to any particular type; however, it is preferable that the amino acids before and after modification are amino acids categorized as having similar properties, as described above.

Furthermore, the present invention comprises polypeptides which are encoded by the polynucleotides of the present invention as described above.

The present invention provides vectors into which the polynucleotides of the present invention have been inserted, host cells retaining the polynucleotides or the vector of the present invention, and methods for producing polypeptides of the present invention utilizing the host cells.

The vectors of the present invention are not limited so long as the DNA inserted in the vector is stably retained. For example, pBluescript vector (Stratagene) is preferable as a cloning vector when using *E. coli* as the host. When using a vector for producing a polypeptide of the present invention, an expression vector is particularly useful. The expression vector is not specifically limited, so long as it expresses polypeptides *in vitro,* in *E. coli,* in cultured cells and *in vivo.* Preferable examples of the expression vectors comprise the pBEST vector (Promega) *for in vitro* expression, the pET vector (Invitrogen) for the expression in *E. coli,* the pME18S-FL3 vector (GenBank Accession No. AB009864) for the expression in cultured cells and the pME18S vector (Mol. Cell Biol. 1988, 8: 466-472) *for in vitro* expression. The insertion of a DNA of the present invention into a vector can be carried out by conventional methods, for example, by ligase reaction using restriction enzyme sites (Current Protocols in Molecular Biology, edit. Ausubel et al., Publish. John Wiley & Sons, 1987, Section 11.4-11.11).

The host cells into which the vectors of the present invention are introduced are not specifically limited, and various host cells can be used according to the objects of the present invention. Examples cells for expressing the polypeptides comprise bacterial cells *(e.g., Streptococcus, Staphylococcus, E. coli, Streptomyces, Bacillus subtilis),* fungal cells (e.g., yeast, *Aspergillus),* insect cells (e.g., Drosophila S2, Spodoptera SF9), animal cells *(e.g.,* CHO, COS, HeLa, C127, 3T3, BHK, HEK293, Bowes melanoma cell) and plant cells. The introduction of a vector to a host cell can be carried out by conventional methods, such as the calcium phosphate precipitation method, the electroporation method (Current protocols in Molecular Biology, edit. Ausubel et al., Publish. John Wiley & Sons, 1987, Section 9.1-9.9), the Lipofectamine method (GIBCO-BRL), the microinjection method, and such.

Appropriate secretion signals can be incorporated into the polypeptides of interest in order to secrete the polypeptides expressed in host cells into the lumen of endoplasmic reticulum, into cavity around the cell, or into the extracellular environment. These signals may be endogenous signals or heterologous signals to the polypeptides of interest.

When the polypeptides of the present invention are secreted, the culture medium is collected to collect the polypeptides of the present invention. When the polypeptides of the present invention are produced intracellularly, the cells are first lysed, and then, the polypeptides are collected.

In order to collect and purify the polypeptides of the present invention from a recombinant cell culture, methods known in the art, comprising ammonium sulfate or ethanol precipitation, acid extraction, anionic or cationic exchange chromatography, phosphocellulose chromatography, hydrophobic interaction chromatography, affinity chromatography, hydroxylapatite chromatography and lectin chromatography, can be used.

Further, the present inventors discovered that the dermokine-α and -β genes of the present invention were located between the genes encoding keratinocyte differentiation-associated protein (Kdap) and suprabasin. Furthermore, it was found for the first time that these four types of genes encode secreted proteins and constitute a gene complex (gene cluster) expressed at the onset of stratification. In other words, the above four types of genes are under a same gene expression regulation and constitute a gene complex. The present inventors named the gene complex SSC.

The present invention provides a gene complex in which gene expression is commonly regulated, wherein the gene complex is involved in the differentiation and/or proliferation of keratinocytes and comprises (1) the Kdap gene, (2) the dermokine-α gene, (3) the dermokine-β gene, and (4) the suprabasin gene.

The above gene complex of the present invention is located on chromosome 7 in mice and chromosome 19 in humans, as shown in Fig. 3. Based on the information shown in Fig. 3, those skilled in the art can obtain information on the precise location and sequences of the gene complex of the present invention, by appropriately using public databases, commercial analysis tools, and such.

The above gene complexes of the present invention are preferably genomic DNA fragments comprising the above genes (1) to (4), and preferably comprise a common expression regulatory region of these genes. The genomic DNA fragments are preferably isolated and purified DNA fragments. In addition, the DNA fragments are preferably from mice or humans.

Furthermore, in a preferred embodiment, the above genomic DNA fragments of the present invention are DNA fragments of 40 kbp (approximately) in length, although they are not limited thereto.

The present invention provides nucleotides having a chain length of at least 15 nucleotides, which are complementary to polynucleotides of the present invention (for example, polynucleotides, or complementary strands thereof, comprising the nucleotide sequence of any one of SEQ ID NO: 1, 3, 5, or 7). Herein, the phrase "complementary strand" is defined as one strand of a double strand nucleic acid composed of A:T (A:U in case of RNA) and G:C base pairs to the other strand. Furthermore, the term "complementary" is not only used for nucleotides that completely match within a continuous region of at least 15 sequential nucleotides, but also those having a homology of at least 70%, preferably at least 80%, more preferably 90% and most preferably 95% or higher within that region. The homology may be determined using an algorithm described herein. Such nucleotides may be used as probe and primers for detection, isolation or amplification of the polynucleotides of the present invention. Typical polynucleotides used as primers generally have a chain length of 15 to 100 nucleotides and preferably 15 to 35 nucleotides. Alternatively, polynucleotides used as probes may have a chain length of at least 15 nucleotides, preferably at least 30 nucleotides, comprising at least a portion or the whole sequence of a DNA of the present invention. Such polynucleotides preferably hybridize specifically to a polynucleotide encoding a polypeptide of the present invention. The phrase "hybridize specifically" means that a it hybridizes under a normal hybridization condition, preferably a stringent condition with a polynucleotide (for example, SEQ ID NO: 1, 3, 5, or 7) of the present invention, but not with DNAs encoding other polypeptides.

Furthermore, the above-described polynucleotides comprise those that suppress the expression of genes encoding the polypeptides of the present invention. Such polynucleotides comprise antisense polynucleotides (antisense DNA/RNA; antisense RNAs, which are complementary to transcriptional products of the genes encoding the polypeptides of the present invention, and DNAs encoding the RNAs), ribozymes (DNAs encoding RNAs having ribozyme activities to specifically cleave transcriptional products of the genes encoding the polypeptides of the present invention), and nucleic acids having an RNAi effect (siRNAs).

A plurality of factors, such as those described below, arise as a result of actions suppressing the expression of a target gene by an antisense polynucleotide: inhibition of transcription initiation by the formation of a triple strand; suppression of transcription through hybridization with a local open loop conformation site formed by an RNA polymerase; inhibition of transcription by hybridization with RNA, which is in course of synthesis; suppression of splicing through hybridization at a junction of intron and exon; suppression of splicing through hybridization with a spliceosome forming site; suppression of transfer from the nuclei to cytoplasm through hybridization with mRNAs; suppression of splicing through hybridization with capping sites or poly(A) addition sites; suppression of translation initiation through hybridization with a translation initiation factor binding site; suppression of translation through hybridization with the ribosome binding site near the initiation codon; inhibition of elongation of peptide chain through hybridization with the translation regions and polysome binding sites of mRNAs; and suppression of expression of genes by hybridization with the interaction sites between nucleic acids and proteins. These actions inhibit the processes of transcription, splicing or translation and suppress the expression of a target gene (Hirajima and Inoue, "New Biochemistry Experimental Course No. 2, Nucleic Acid IV, Duplication and Expression of Genes", Japan Biochemical Society ed., Tokyo Kagaku Doujin, 1993, 319-347).

The antisense nucleic acids (polynucleotides) of the present invention may suppress the expression of the target gene through any of the above-mentioned actions. According to one embodiment, an antisense sequence designed to be complementary to a non-translated region near the 5' terminus of mRNA of a gene may effectively inhibit the translation of the gene. Additionally, sequences which are complementary to the coding region or the 3' non-translated region can be also used. As described above, polynucleotides comprising antisense sequences not only to the translation region of a gene, but also those to sequences of non-translated regions are comprised in the antisense polynucleotides of the present invention. The antisense polynucleotides to be used in the present invention are preferably linked downstream of an appropriate promoter and a sequence comprising a transcriptional termination signal is preferably linked to the 3'-side thereof. The sequence of the antisense polynucleotide is preferably complementary to the target gene or a part thereof; however, so long as the expression of the gene can be effectively inhibited, it does not have to be a DNA completely complementary to the target sequence. The transcribed RNA (antisense polynucleotide) is preferably 90% or more, more preferably 95% or more, complementary to the transcribed product of the target gene. In order to effectively inhibit the expression of the target gene using an antisense sequence, the antisense polynucleotide has at least a chain length of 15 nucleotides or more, preferably 100 nucleotides, more preferably 500 nucleotides or more, and usually has a chain length of 3000 nucleotides or less, preferably 2000 or less nucleotides to cause an antisense effect.

The antisense polynucleotide can be prepared, for example, by the phosphorothionate method (Stein, "Physicochemical properties of phosphorothioate oligodeoxynucleotides." Nucleic Acids Res., 1988, 16: 3209-3221) based on the sequence information of a polynucleotide (for example, the sequence of SEQ ID NO: 1, 3, 5, or 7) encoding a polypeptide of the present invention.

Furthermore, suppression of the expression of endogenous genes can be also achieved utilizing nucleic acids (polynucleotides) encoding ribozymes. Ribozymes are RNA molecules having catalytic activity. There exist ribozymes with various activities, and research on ribozymes as an enzyme for cleaving RNA allowed for the design of ribozymes that cleave RNAs in a site-specific manner. There are ribozymes which are larger than 400 nucleotides, such as Group I intron type ribozymes and M1RNA, a member of RNaseP, as well as those which have an active domain of about 40 nucleotides, called hammer-head type or hairpin type ribozyme (Makoto Koizumi and Eiko Ohtsuka, Protein Nucleic Acid and Enzyme (PNE), 1990, 35: 2191).

For example, the hammer-head type ribozyme cleaves the 3'-side of C15 of G13U14C15 within its own sequence. A base pair formation of the U14 with the A at position 9 is important for the activity and the clevage is demonstrated to proceed even if the C at position 15 is A or U (M. Koizumi et al., FEBS Lett., 1988, 228: 225). Restriction enzymatic RNA-truncating ribozymes recognizing sequences of UC, UU or UA in a target RNA may be generated by designing the substrate binding site of the ribozyme complementary with the RNA sequence near the target site (M. Koizumi et al., FEBS Lett. 1988, 239: 285; Makoto Koizumi and Eiko Ohtsuka, Protein Nucleic Acid and Enzyme (PNE), 1990, 35: 2191); M. Koizumi et al., Nucleic Acids Res. 1989, 17: 7059).

Furthermore, the hairpin type ribozymes are also useful in the context of the present invention. The hairpin type ribozymes are found on, for example, the minus chain of a satellite RNA of tobacco ringspot virus (J.M. Buzayan, Nature 1986, 323: 349). It is also demonstrated that this ribozyme can be designed to cause a target specific RNA cleavage (Y. Kikuchi and N. Sasaki, Nucleic Acids Res. 1992, 19: 6751; Y. Kikuchi, Chemistry and Organism 1992, 30: 112).

Examples of preferable embodiments of the above siRNAs of the present invention are double stranded RNAs having an RNAi (RNA interference) effect (siRNAs) on a dermokine-α or -β gene. More specific examples comprise double stranded RNAs (siRNAs) comprising sense and antisense RNAs that correspond to a partial sequence of a nucleotide sequence of any one of SEQ ID NO: 1, 3, 5, or 7.

Although details of the mechanism of RNAi are still partially unclear, it is believed that an enzyme called DICER (a member of the family of RNase III nucleases) makes contact with double stranded RNAs and the double stranded RNAs are degraded into small fragments called small interfering RNAs, or siRNAs. Double stranded RNAs before such degradation by DICER are also comprised in the double stranded RNAs that have an RNAi effect of the present invention. In other words, because even a long RNA, which by itself has no RNAi effect, is expected to be degraded within cells to form siRNAs that have an RNAi effect, the length of the double stranded RNAs of the present invention is not particularly limited.

For example, a long double stranded RNA corresponding to a full length, or almost full length region of an mRNA of a dermokine gene of the present invention may be pre-degraded with DICER, and those degradation products can be appropriately used. Such degradation products are expected to comprise double stranded RNA molecules that have an RNAi effect (siRNAs). In such methods, it is not particularly necessary to select an mRNA region expected to have an RNAi effect. Thus, it is not particularly necessary to precisely define the region(s) on an mRNA of a dermokine gene of the present invention that has an RNAi effect.

With respect to the above RNA molecules, molecules that have a closed structure on one end, for example, siRNAs comprising a hairpin structure (shRNAs), are also comprised in the present invention. Thus, single stranded RNA molecules that can form a double stranded RNA structure within the molecule are also comprised in the present invention.

Those skilled in the art can appropriately construct the above "double stranded RNAs capable of inhibiting through RNAi effect" of the present invention, based on the nucleotide sequences of the dermokine genes of the present invention, which are targets for the double stranded RNAs. As an example, a double stranded RNA of the present invention may be constructed based on the nucleotide sequence of any of SEQ ID NO: 1, 3, 5, or 7. Thus, based on the nucleotide sequence of any one of SEQ ID NO: 1, 3, 5, or 7, it is within the range of standard practice for those skilled in the art to appropriately select an arbitrary continuous RNA region of an mRNA, which is a transcript of this nucleotide sequence, and construct a double stranded RNA corresponding to this region. Those skilled in the art can also use standard methods to appropriately select an siRNA sequence having a strong RNAi effect from the mRNA sequences which are the transcripts of this sequence. In addition, if one strand is determined (for example, the nucleotide sequence of any one of SEQ ID NO: 1, 3, 5, or 7), those skilled in the art can easily know the nucleotide sequence of the other strand (complementary strand). Those skilled in the art can appropriately generate siRNAs using a commercially available nucleic acid synthesizer. Moreover, common custom synthesis services may be used to synthesize a desired RNA.

Furthermore, DNAs (vectors) that can express the above RNAs of the present invention are also comprised in a preferred embodiment of the compounds capable of inhibiting the expression of a dermokine gene of the present invention. An example of DNAs (vectors) that can express the above double stranded RNAs of the present invention is a DNA in which a DNA encoding one strand of the double stranded RNA and a DNA encoding the other strand of the double stranded RNA are linked to a promoter such that both can be expressed. The above-described DNAs of the present invention can be appropriately constructed by those skilled in the art using standard genetic engineering techniques. More specifically, the expression vectors of the present invention can be constructed by appropriately inserting a DNA encoding an RNA of the present invention into a variety of known expression vectors.

When a polynucleotide suppressing the expression of a gene encoding a polypeptide of the present invention is used in gene therapy, for example, it may be administered to a patient by the *ex vivo method, in vivo* method and such, using, for example, viral vectors comprising, but not limited to, retroviral vector, adenoviral vector and adeno-associated viral vectors; and non-viral vectors such as liposome.

The present invention provides antibodies that bind to a polypeptide of the present invention. Herein, the term "antibodies" encompasses polyclonal antibodies, monoclonal antibodies, chimeric antibodies, single-stranded antibodies, humanized antibodies and Fab fragments comprising Fab or other products of the immunoglobulin expression library.

A polypeptide of the present invention or fragment or analog thereof, or a cell that expresses one of these can be used as an immunogen for producing antibodies binding to a polypeptide of the present invention. The antibodies are preferably immunospecific to a polypeptide of the present invention. The term "immunospecific" means that the antibody has substantially higher affinity to a polypeptide of the present invention than to other polypeptides.

The antibodies binding to a polypeptide of the present invention can be prepared by methods known to those in the art. For example, a polyclonal antibody can be obtained as follows: A polypeptide of the present invention or a fusion protein thereof with GST is immunized to small animals such as rabbit to obtain serum. The polyclonal antibody may be prepared by purifying the serum through ammonium sulfate precipitation, protein A or protein G column, DEAE ion exchange chromatography, or affinity column, wherein the polypeptide of the present invention is coupled and such. On the other hand, a monoclonal antibody can be prepared as follows: A polypeptide of the present invention is administered to a small animal such as a mouse and the spleen is subsequently excised from the mouse and ground to separate cells. Then, the cells are fused with mouse myeloma cells using reagents such as polyethylene glycol or such, and clones that produce antibodies binding to the polypeptide of the present invention are selected from these fused cells (hybridoma). The obtained hybridoma is then transplanted into the peritoneal cavity of a mouse and ascites is collected from the mouse. The monoclonal antibodies can be prepared by purifying the ascite using, for example, ammonium sulfate precipitation; protein A or protein G column; DEAE ion exchange chromatography; affinity column wherein the polypeptides of the present invention are coupled; and such.

The antibodies of the present invention can be used for the isolation, identification and purification of the polypeptides of the present invention and cells expressing them.

The present inventors succeeded in actually producing antibodies (polyclonal antibodies) against each of the human proteins of the present invention: dermokine-α, dermokine-β, Kdap, and suprabasin. Examples of antibodies of the present invention are antibodies against human dermokine-α, dermokine-β, Kdap, or suprabasin, as described in the Examples below.

Furthermore, dermokine knockout mice can be generated based on the discoveries of the present inventors. Such knockout animals (nonhuman animals) are extremely useful in examining the physiological roles of the SSC.

Thus, the present invention relates to animals (non-human animals) in which the expression of a gene of the present invention (dermokine-α/-β, for example) is artificially suppressed.

The aforementioned phrasing "the expression of a gene is artificially suppressed" usually refers to a condition in which gene expression is suppressed due to a genetic mutation, such as a nucleotide insertion, deletion, or substitution, in one or both of the gene pair. Expression of a mutant protein in which the function is diminished or lost compared to a normal protein is also comprised in the "suppressed gene expression". The above "suppression" comprises not only complete suppression of gene expression, but also suppression of the expression of only one of the gene pair.

In the present invention, subject animals for genetic modification are normally animals other than humans, and are preferably rodents such as mice, rats, hamsters, and rabbits. Of these, mice are particularly preferable. In the present invention, ES cells which are subjects of genetic modification are also preferably derived from rodents, and particularly preferably derived from mice. "Knockout mice", as referred to in general, are also comprised in the genetically modified animals of the present invention.

Examples of methods for artificially suppressing the expression of a gene in the genetically modified nonhuman animals of the present invention (also simply described as "genetically modified animals") comprise deletion of an entire gene, or a part thereof, and deletion of an entire expression regulatory region of a gene, or a part thereof. In a preferable method, however, the gene is inactivated by inserting an exogenous gene into one or both of the gene pair. Thus, in a preferable embodiment of the present invention, genetically modified animals typically comprise a foreign gene that is inserted into one or both of the gene pair.

Those skilled in the art can generate the genetically modified animals of the present invention using commonly known genetic engineering techniques. For example, a genetically modified mouse can be generated as follows: first, a DNA comprising exons of a gene of the present invention (the dermokine-α/-β gene, for example) is isolated from mice and an appropriate marker gene is inserted to this DNA fragment to construct a targeting vector. The targeting vector is introduced into a mouse ES cell line by electroporation or such, and cell lines in which homologous recombination has taken place are selected. The marker gene to be inserted is preferably an antibiotic resistance gene, such as the neomycin resistance gene. When an antibiotic resistance gene is inserted, cell lines in which homologous recombination has taken place can be selected by simply culturing cells in a medium comprising the antibiotic. Furthermore, for a more efficient selection, a thymidine kinase gene or such can be linked to the targeting vector. By doing this, cell lines in which non-homologous recombination had taken place can be eliminated. Furthermore, homologous recombinants may be examined by PCR and Southern blotting to efficiently obtain cells lines in which one of a gene pair of the gene of the present invention is inactivated.

When selecting cell lines in which homologous recombination had taken place, since there is a risk of unknown gene destruction in sites other than the site of homologous recombination due to gene insertion, it is preferable to use multiple clones to generate chimera. Chimeric mice can be obtained by injecting the obtained ES cell lines into mouse blastoderms. By crossing these chimeric mice, mice in which one of a gene pair of a gene of the present invention is inactivated can be obtained. Further, by crossing these mice, mice in which both of the gene pair of the gene of the present invention are inactivated can be obtained. Similar procedures may be employed to genetically modify animals other than mice for which ES cells are established.

ES cell lines in which a gene pair of a gene of the present invention is inactivated can also be obtained as follows: that is, by culturing ES cell lines in which one of the gene pair is inactivated in media with a high concentration of antibiotics, cell lines in which the other of the gene pair is inactivated, that is, ES cell lines in which both of a gene pair of a gene of the present invention are inactivated can be obtained. Moreover, they can be generated by selecting ES cell lines in which one of a gene pair is inactivated, introducing again a targeting vector into these cell lines, and selecting the cell lines in which homologous recombination has taken place. The marker gene inserted in the targeting vector is preferably different from the marker gene previously used.

The present invention also provides cell lines established from the genetically modified nonhuman animals of the present invention. Known methods can be used to establish cell lines derived from the genetically modified animals of the present invention. For example, primary culture methods for embryonic cells can be used in rodents ("Shin-Seikagaku Jikken Koza" Vol. 18, p125-129, Tokyo Kagaku Dojin Co., Ltd; and "Manual for manipulations of mouse embryos." p262-264, Kindai Shuppan).

The genetically modified animals of the present invention, and cell lines and ES cell lines established from these animals, can be used for detailed analyses of the functions of the genes of the present invention. Furthermore, the genetically modified animals of the present invention can be used in methods of screening for compounds that substitute the functions of the proteins of the present invention, and the like.

Further, the above polypeptides (proteins) of the present invention are expected to have the effect of inducing keratinocyte differentiation. Therefore, the present invention relates to agents for inducing keratinocyte differentiation (which may also be described as "differentiation inducers" in the present description), comprising the above polypeptides (proteins) of the present invention as active ingredients.

In a preferred embodiment, the present invention provides keratinocyte differentiation inducers, comprising a dermokine-α or dermokine-β protein as an active ingredient. Examples of the above dermokine-α protein comprise the polypeptide of SEQ ID NO: 2 or 6. Examples of the above dermokine-β protein comprise the polypeptide of SEQ ID NO: 4 or 8.

Moreover, DNA encoding the above polypeptides, which are components of the keratinocyte differentiation inducers of the present invention, are also comprised in the present invention.

In addition, the present invention relates to keratinocyte differentiation inducers comprising substances that activate expression of the SSC, or of a dermokine-α or -β protein.

In the present invention, "substances that activate the expression of a protein" are substances (compounds) that significantly activate (increase) the expression of a protein. In the present invention, the above "activation of expression" comprises transcriptional activation of a gene encoding the protein, and/or activation of translation of transcripts of the gene.

Examples of substances that activate the expression of the SSC or a dermokine-α/-β protein of the present invention comprise substances that bind to transcription regulatory regions (for example, a promoter region) of the SSC or a dermokine-α/-β gene and promote the transcription of these genes (such as transcriptional activators).

In the present invention, those skilled in the art can easily measure the expression activation of the SSC or dermokine-α/-β protein using known methods such as RT-PCR, Northern blotting, and Western blotting.

Further, substances (compounds) that inhibit the expression or function of a dermokine-α protein or a dermokine-β protein (these two proteins (genes) may also be collectively referred to as a "dermokine-α/-β protein (gene)") are expected to have the effect of suppressing keratinocyte differentiation. Therefore, the present invention relates to agents for suppressing keratinocyte differentiation (also described as "differentiation inhibitors" in the present description), comprising as an active ingredient a substance (compound) that can inhibit the expression or function of a dermokine-α or -β protein.

In the present invention, "expression inhibitors" are substances (compounds) that significantly reduce or completely suppress the expression of a protein. In the present invention, the above "inhibition of expression" comprises inhibition of transcription of genes encoding proteins, and/or inhibition of translation from transcripts of the genes.

In a preferred embodiment, the above differentiation inhibitors are drugs comprising as active ingredient a compound selected from the group of (a) to (d) below:
(a) antisense nucleic acids for a transcript, or a part thereof, of a dermokine-α/-β gene;
(b) nucleic acids having a ribozyme activity that specifically cleaves a transcript of a dermokine-α/-β gene;
(c) nucleic acids having an inhibitory effect on the expression of a dermokine-α/-β gene through an RNAi effect; and
(d) antibodies that bind to a dermokine-α/-β protein (anti-dermokine-α/-β protein antibodies).

Preferable examples of the nucleic acids in the above-described drugs of the present invention comprise the aforementioned antisense nucleic acids and nucleic acids having a ribozyme activity. Furthermore, expression of endogenous genes can also be inhibited by RNA interference (RNAi) using a double stranded RNA comprising a sequence identical or similar to the sequence of a target gene. RNAi refers to a phenomenon in which, when a double stranded RNA comprising a nucleotide sequence identical or similar to the sequence of a target gene is introduced into a cell, the expression of both exogenously introduced genes and endogenous genes are inhibited. Although details of the mechanisms of RNAi are unclear, it is thought that the introduced double stranded RNAs are first degraded into small fragments which somehow become an indicator for the target genes, and the target genes are degraded. Although it is not necessary that the RNAs used for RNAi are completely identical to a dermokine-α/-β gene or partial regions of the gene, they preferably have a complete homology.

In addition, examples of inhibitors of the function of dermokine-α/-β proteins comprise the above antibodies of the present invention, for example, antibodies that bind to dermokine-α/-β proteins (antibodies that recognize dermokine-α/-β proteins; anti-dermokine-α/-β protein antibodies). These antibodies inhibit the functions of dermokine-α/-β proteins by binding to the dermokine-α/-β proteins. As a result, keratinocyte differentiation is expected to be induced. There is no particular limitation on the above antibodies of the present invention, however, they are preferably antibodies that specifically bind to dermokine-α/-β proteins (recognize dermokine-α/-β proteins) and do not bind to other proteins.

Possible uses of the antibodies of the present invention that bind to the dermokine-α/-β proteins comprise the use aiming for the suppression of keratinocyte differentiation. When the antibodies are used with the aim of administering to humans (antibody therapy), human antibodies or humanized antibodies are preferably used to reduce immunogenicity.

The agents of the present invention for inducing keratinocytes differentiation are expected to become drugs for the treatment of disorders caused by abnormal keratinocyte differentiation (reduced induction of differentiation). Examples of such diseases are psoriasis vulgaris accompanied by parakeratosis, solar keratosis, and so on.

In addition, the agents of the present invention for suppressing keratinocyte differentiation are expected to become drugs for the treatment of disorders caused by increased keratinocyte differentiation. Examples of such diseases comprise skin diseases such as ichthyosis vulgaris accompanied by excessive keratinization, X-linked ichthyosis, common wart, lichen amyloidosis, lichen planus accompanied by hypergranulosis, and bullous typed ichthyosiform erythroderma, and so on.

The keratinocyte differentiation inducers and inhibitors of the present invention (also collectively referred to as "drugs" in the present description) can be orally or parenterally administered as medicinal compositions after mixing with a physiologically acceptable carrier, vehicle, diluting agent or such. Drugs for oral administration may be prepared as granules, powders, tablets, capsules, solvents, emulsions, suspensions, etc. Drugs for parenteral administration may be prepared in a formula selected from injections, drips, drugs for external use, suppositories, etc. Injections may be performed subcutaneously, intramuscularly, intraperitoneally, or such. Drugs for external use may be intranasal drugs, ointments, and so on. Technologies for manufacturing drugs that comprise the differentiation inducers of the present invention as principal components in the above forms are known.

For example, tablets for oral administration can be prepared by mixing a drug of the present invention with a vehicle, disintegrating agent, binding agent, lubricating agent, and the like, and compressing. Lactose, starch, mannitol, or such, are generally used as vehicles. Calcium carbonate, carboxymethylcellulose calcium, and such, are generally used as disintegrating agents. Gum arabic, carboxymethylcellulose, or polyvinyl pyrrolidone are used as binding agents. Talc, magnesium stearate, and such are known as lubricating agents.

Tablets comprising a drug of the present invention may be masked or coated with a known coating to prepare an enteric-coated drug. Ethyl cellulose, polyoxyethylene glycol, and the like may be used for the coating agent.

Injections may be obtained by dissolving a drug of the present invention, which is the main ingredient, with an appropriate dispersing agent, or by dissolving or dispersing it in a dispersing solvent. Depending on the choice of dispersing agent, the drugs can have the formula of either a water-based or oil-based solvent. To make them water-based solvents, distilled water, physiological saline, Ringer's solution, or the like are used as dispersing solvents. For oil-based solvents, a variety of vegetable oils, propylene glycols, or such are used as dispersing solvents. If necessary, preservatives such as paraben can be added at this time. Also, known isotonic agents such as sodium chloride, glucose, and such can be added to injections. Furthermore, analgesic agents such as bezalkonium chloride or procaine hydrochloride can be added.

The drugs of the present invention can be made into external use agents by preparing them as solid, liquid, or semisolid compositions. The solid or liquid compositions can be made into external use agents by preparing them as compositions similar to those as mentioned above. Semisolid compositions can be prepared by adding a thickening agent into an appropriate solvent, as necessary. Water, ethyl alcohol, polyethylene glycol, and the like may be used as solvents. Bentonite, polyvinyl alcohol, acrylic acid, methacrylic acid, polyvinyl pyrrolidone, and such are generally used as thickening agents. Preservatives such as bezalkonium chloride can be added to these compositions. In addition, an oil-based substrate such as cacao oil, or a water-based gel substrate such as a cellulose derivative may be combined as a carrier to prepare suppository drugs.

The drugs of the present invention are administered to mammalian animals comprising humans at a necessary dose within the range of doses that are determined as safe. Doses of the drugs of the present invention may be appropriately determined after considering the type of formula, the method of administration, the age and body weight of the patient, the condition of the patient, and such, and ultimately based on the judgment of doctors or veterinarians.

In addition to the above diseases, the drugs of the present invention (keratinocyte differentiation inducers and inhibitors) are expected to have a therapeutic or preventive effect on abnormal biological conditions or damage of the skin such as dry skin, dandruff, acne, keratosis, eczema, skin fall, pruritus, senile plaques, moles, melanodermas, wrinkles, warts, skin plaques, hyperpigmentation, hyperkeratinization, inflammatory skin, or age-associated changes of the skin. The drugs of the present invention are normally applied to humans, although they can also be applied to skin diseases in dogs, cats, and so on, for example.

Further, the present invention provides methods of screening for agents (compounds) for keratinocyte differentiation inducers (compounds having a differentiation-inducing effect).

In a preferred embodiment of the screening methods of the present invention, the methods use the expression level or activities of dermokine-α/-β proteins as an index. In other words, compounds that increase the expression or activity of a dermokine-α/-β protein are expected to have a keratinocyte differentiation-inducing effect.

In the above methods of the present invention, test compounds are first contacted with cells expressing a dermokine-α and/or-β protein (gene).

The "cells" used in the present methods are not limited to any particular cells, but are preferably human-derived cells. The "cells expressing a dermokine-α/-β protein" may be cells expressing the endogenous dermokine-α/-β protein, or cells into which an exogenous dermokine-α/-β gene has been introduced and the gene is expressed. The cells expressing an exogenous dermokine-α/-β gene can normally be produced by introducing an expression vector into which a dermokine-α/-β gene has been inserted into host cells. Such expression vectors can be constructed using standard gene engineering techniques.

Test compounds in the screening methods of the present invention are not particularly limited. Examples comprise, without limitation, single compounds such as natural compounds, organic compounds, inorganic compounds, proteins, and peptides, as well as compound libraries, expression products of gene libraries, cell extracts, cell culture supernatants, products of fermenting microorganisms, extracts of marine organisms, plants extracts.

Moreover, these test compounds can be appropriately labeled and used, as necessary. Examples of labels comprise radioisotopes and fluorescent labels.

Normally, "contact" of the test compounds with cells expressing a dermokine-α/-β gene is carried out by adding the test compounds to the culture media of these cells, but the methods are not limited thereto. When the test compounds are proteins and such, "contact" can be carried out by introducing DNA vectors for expressing the proteins into these cells.

In the present methods, an expression level or activity of a dermokine-α/-β protein is then measured in the cells expressing that protein. Those skilled in the art can easily and appropriately measure the expression level, using general methods such as Northern blotting and Western blotting. For example, measurement can be carried out using the label on the test compound contacted with the cells expressing the dermokine-α/-β proteins as an index.

In the present invention, "expression" means both transcription from a gene encoding a protein (mRNA production), and translation from transcripts of this gene.

Moreover, when the above screening methods are performed using an activity of a dermokine-α/-β protein as an index, the aforementioned functions (activities) of dermokine-α/-β protein, for example, the activity of inducing keratinocyte differentiation, can be used as an index. Those skilled in the art can appropriately measure the activities of a dermokine-α/-β protein using known methods.

In the methods of present invention, the aforementioned "activities" are preferably the "activity of inducing the differentiation of keratinocytes into stratified epithelial cells when dermokine-α/-β proteins are expressed in the keratinocytes" as described above.

In the next step of the above methods, compounds that increase an expression level or activity of a dermokine-α/-β protein, as compared to in the absence of contact with the test compound, are selected.

Compounds obtained by the above screening methods are expected to become drugs for treating diseases caused by abnormal keratinocytes differentiation, for example.

Further, the present invention provides methods of screening for keratinocyte differentiation inhibitors (compounds having a differentiation-inducing effect).

In a preferred embodiment of the screening methods of the present invention, the expression level or activity of a dermokine-α/-β protein is used as an index. That is, compounds that decrease (inhibit) the expression or activity of a dermokine-α/-β protein are expected to have a keratinocyte differentiation-inhibiting effect.

The above methods of the present invention can be appropriately performed according to the aforementioned methods of screening for differentiation inducers. In other words, in the aforementioned methods of the present invention, first, test compounds are contacted with cells expressing a dermokine-α and/or -β proteins (genes). Then, compounds are selected that decrease an expression level or activity of a dermokine-α/-β protein, compared to in the absence of contact with the test compound.

Compounds obtained by the above screening methods are expected to become drugs for treating diseases caused by increased keratinocyte differentiation, for example.

In a preferred embodiment of the above screening methods of the present invention, an "activity of making keratinocytes differentiate into stratified epithelial cells when dermokine-α/-β protein is expressed in the keratinocytes" is used as an index.

Thus, the present invention relates to methods of screening for keratinocyte differentiation inducers, where the methods comprise the following steps:
(a) contacting a test compound with keratinocytes that express a dermokine-α protein or a dermokine-β protein;
(b) measuring the activity of the aforementioned dermokine-α protein or dermokine-β protein in making keratinocytes differentiate into stratified epithelial cells; and
(c) selecting compounds that increase the aforementioned activity compared to in the absence of contact with the test compound.

Also, the present invention provides methods of screening for keratinocyte differentiation inhibitors, where the methods comprise the following steps:
(a) contacting a test compound with keratinocytes that express a dermokine-α protein or a dermokine-β protein;
(b) measuring the activity of the aforementioned dermokine-α protein or dermokine-β protein in making keratinocytes differentiate into stratified epithelial cells; and
(c) selecting compounds that decrease the aforementioned activity compared to in the absence of contact with the test compound.

Another embodiment of the present invention is methods of screening for keratinocyte differentiation inducers, where the methods comprise the following steps:
(a) coexisting a test compound and keratinocytes with a dermokine-α or dermokine-β protein, or cells expressing the protein;
(b) measuring the differentiation of keratinocytes into stratified epithelial cells; and
(c) selecting compounds that increase differentiation into stratified epithelial cells, compared to in the absence of the test compound.

Furthermore, another embodiment of the present invention relates to methods of screening for keratinocyte differentiation inhibitors, where the methods comprise the following steps:
(a) coexisting a test compound and keratinocytes with a dermokine-α or dermokine-β protein, or cells expressing the protein;
(b) measuring the differentiation of keratinocytes into stratified epithelial cells; and
(c) selecting compounds that decrease differentiation into stratified epithelial cells, compared to in the absence of the test compound.

The cells expressing dermokine-α/-β proteins in the above step (a) can be any cells as long as they can be co-cultured with keratinocytes; and keratinocytes themselves are acceptable. The cells expressing dermokine-α/-β proteins can usually be prepared by introducing keratinocytes with a vector in which a DNA encoding a dermokine-α/-β protein is carried in an expressible form (dermokine-α/-β expression vectors).

The "activity of making keratinocytes differentiate into stratified epithelial cells when dermokine-α/-β proteins are expressed in the keratinocytes" in the above step (b) can be appropriately measured by, for example, using the expression of a differentiation marker for stratified epithelia, or preferably that of involucrin, as an index. For example, compounds that increase the expression of the aforementioned involucrin are judged to have a keratinocyte differentiation-inducing effect, and compounds that decrease involucrin expression are judged to have a keratinocyte differentiation-inhibiting effect.

Moreover, since the dermokine-α/-β proteins of the present invention are expressed in the stratified epithelium, they are expected to become markers for cancer cells derived from the stratified epithelia. By using the presence or absence of the expression or activity of the dermokine-α/-β proteins of the present invention as an index, it becomes possible to determine whether cancer cells of interest are derived from the stratified epithelia or not. The present invention provides methods for examining whether or not tested cells are cancer cells derived from stratified epithelia, wherein the presence or absence of an expression or activity of a dermokine-α/-β protein is used as an index.

In a preferred embodiment, the above examination methods of the present invention comprise the following steps:
(a) measuring an expression level or activity of a dermokine-α protein or a dermokine-β protein in tested cells; and
(b) determining that the tested cells are cancer cells that are derived from the stratified epithelium when the above expression level or activity differs from the control.

"Measuring" in the above step (a) is appropriately performed according to the aforementioned methods. Moreover, the "control" in the above step (b) usually refers to normal cells. Specifically, they are cells already determined as not being cancer cells, and are generally cells derived from a healthy person. Furthermore, cancer cells already determined as not being cancer cells that are derived from the stratified epithelium can also be used as the above "control".

In the above step (b), cells in which the expression or activity of a dermokine-α/-β protein differs from the control can be judged as being cancer cells that are derived from the stratified epithelium, for example, squamous epithelial cancer cells.

In the present invention, the above "differ" normally means "increase" or "decrease", but preferably indicates "decrease". Thus, in a preferred embodiment of the present invention, the cells in which the expression or activity of a dermokine-α/-β protein is decreased compared to a control can be judged to be cancer cells that are derived from the stratified epithelium, for example, squamous epithelial cancer cells.

The proteins of the present invention are thought to be involved in diseases with abnormal proliferation or differentiation derived from the stratified epithelium. For example, squamous epithelial cancers and basal cell cancers that are derived from the stratified squamous epithelium (the pseudo-stratified epithelium in the case of lung cancers) are frequently observed in skin cancers, esophageal cancers, cervical cancers, lung cancers, thymic cancers, and such. The proteins (genes) of the present invention are predicted to be involved in the degree of progression or differentiation of cancer in these cancers, and are thus useful as a marker for these cancers. In the present invention, the aforementioned "cancer cells that are derived from the stratified epithelium" preferably refers to cells from the above cancers. The above methods of the present invention are particularly effective for cancers of the squamous epithelia of the bronchi of the lung (pseudo-stratified epithelium) which are not originally stratified squamous epithelia.

Furthermore, using the above examination methods, a diagnosis can be carried out to see whether or not a subject is affected with squamous epithelial cancer or basal cell cancer. Thus, the present invention relates to methods for diagnosing squamous epithelial cancers or basal cell cancers in subjects, comprising the following steps:
(a) determining whether or not cell samples prepared from a subject are cancer cells that are derived from the stratified epithelium by using an aforementioned examination method of the present invention; and
(b) determining that a subject is affected with squamous epithelial cancer or basal cell cancer when cell samples were judged by the above step to be derived from the stratified epithelium.

Moreover, the present invention enables to carry out the aforementioned examination (diagnosis) methods using body fluids such as blood as test samples. Since the proteins of the present invention are secreted proteins, diagnosis of lung cancers and such or of cancers that are derived from the stratified epithelium, such as esophageal cancers, can be appropriately performed, especially when the proteins of the present invention are secreted into blood.

Furthermore, the proteins of the present invention are considered to be involved in a variety of skin diseases. For example, in pathological conditions of psoriasis and such, in which the turnover of the epidermis is enhanced, the basal layer is significantly enlarged, proliferation is increased, and parakeratosis occurs. In lichen planus and such, a thickening of the cornified and granular layers without parakeratosis is observed. The proteins of the present invention (for example, dermokine-α/-β proteins) may also be involved in diseases caused by abnormal differentiation and proliferation such as those mentioned above.

The present invention provides methods for diagnosing skin diseases in subjects, comprising the following steps:
(a) measuring an expression level or activity of a dermokine-α protein or a dermokine-β protein in samples prepared from a subject; and
(b) determining that a subject is affected with a skin disease when the above expression level or activity differs from a control.

In the above methods, when the expression level or activity is increased in step (b), the subject is judged to be affected with a skin disease that accompanies an increase in dermokine-α/-β protein expression or activity. On the other hand, when the expression level or activity is decreased in step (b), the subject is judged to be affected with a skin disease that accompanies a reduction in dermokine-α/-β protein expression or activity.

The above "skin disease" usually refers to a disease that accompanies (is caused by) a "change" in the expression or activity of a dermokine-α/-β protein. More specifically, examples of skin diseases comprise psoriasis, lichen planus, keratosis, eczema, pruritus, melanoderma, inflammatory dermatitis.

Examples of biological samples that can be subjected to the above diagnosis methods comprise a subject's skin tissues (cells) and such, however, as long as the samples comprise cells or cell extracts derived from a subject, they are not limited thereto.

Herein, subjects in the present invention usually refer to humans, however, as long as subjects are organisms comprising a protein of the present invention (for example, a dermokine-α/-β), they are not limited thereto. For example, the examination and diagnosis methods of the present invention (for example, the methods for diagnosing skin diseases) can also be performed with animals such as dogs and cats.

In the present invention, measurement of dermokine-α/-β protein expression or activities can also be carried out by measuring the expression or activities of the SSC, a complex comprising dermokine-α/-β protein as a component.

Furthermore, the present invention relates to methods for treating or preventing a disease caused by a reduction in the ability to induce keratinocyte differentiation, where the methods comprise administering a dermokine-α protein, a dermokine-β protein, or a keratinocyte differentiation inducer obtained by an aforementioned screening method to an individual (for example, a patient).

Moreover, the present invention relates to methods for treating or preventing a disease caused by an increase in keratinocyte differentiation, where the methods comprise administering to an individual (a therapeutically effective dose of) keratinocyte differentiation inhibitors obtained by an aforementioned screening method or a compound selected from the group of (a) to (d) below:
(a) antisense nucleic acids corresponding to a transcript of a dermokine-α gene or a dermokine-β gene;
(b) nucleic acids having a ribozyme activity which specifically cleaves a transcript of a dermokine-α gene or a dermokine-β gene;
(c) nucleic acids having an inhibitory effect through an RNAi effect on the expression of a dermokine-α gene or a dermokine-β gene; and
(d) antibodies that bind to a dermokine-α protein or a dermokine-β protein.

Furthermore, the present invention relates to uses of a dermokine-α protein, a dermokine-β protein, or a keratinocyte differentiation inducer obtained by an aforementioned screening method in the production of drugs for treating diseases caused by a reduction in the induction of keratinocyte differentiation.

Moreover, the present invention relates to uses, in the production of drugs for treating diseases caused by an increase in keratinocyte differentiation, of an inhibitor of keratinocyte differentiation obtained by an aforementioned screening method or a compound selected from the group of (a) to (d) below:
(a) antisense nucleic acids corresponding to a transcript of a dermokine-α gene or a dermokine-β gene;
(b) nucleic acids having a ribozyme activity which specifically cleaves a transcript of a dermokine-α gene or a dermokine-β gene;
(c) nucleic acids having an inhibitory effect through an RNAi effect on the expression of a dermokine-α gene or a dermokine-β gene; and
(d) antibodies that bind to a dermokine-α protein or a dermokine-β protein.

In the present invention, examples of diseases caused by a reduction in the induction of keratinocyte differentiation comprise psoriasis vulgaris and solar keratosis. In the present invention, examples of diseases caused by an increase in keratinocyte differentiation comprise, for example, ichthyosis vulgaris accompanied by excessive keratinization, X-linked ichthyosis, common wart, lichenoid amyloidosis, lichen planus accompanied by hypergranulosis, and bullous typed ichthyosiform erythroderma.

Individuals in the methods for treatment or prevention of the present invention normally refer to patients with an above disease, and although they are not particularly limited thereto, they are preferably humans.

Administration to patients can generally be carried out using methods known to those skilled in the art such as intraarterial injection, intravenous injection, and subcutaneous injection. The administered doses vary depending on the weight and age of patients, the administration methods, or such, but those skilled in the art can appropriately select the proper doses. Furthermore, if the compounds can be encoded by a DNA, the DNA may be incorporated into a vector for gene therapy to perform gene therapy.

Examples of vectors for gene therapy comprise viral vectors such as retrovirus vectors, adenovirus vectors, and adeno-associated virus vectors, as well as non-viral vectors such as liposomes. Using these vectors, DNAs of interest can be administered into patients by *ex vivo methods, in vivo* methods, and such.

All prior art references cited herein are included in this description by reference.

### Examples

The present invention is be explained more specifically below with reference to Examples, but it is not to be construed as being limited thereto.

### [Cell cultures and antibodies]

Normal human embryonic keratinocytes (NHEK) were purchased from as frozen cells after primary culture by the supplier (KURABO, Osaka, Japan). NHEK cell were cultured in serum-free medium, Humedia KG2 (KURABO). 293/EBNA-1 cells were purchased from Invitrogen (San Diego, CA), and maintained in Dulbecco's Modified Eagle's medium (Sigma) supplemented with 10% fetal calf serum. Anti-His antibody (Penta-His antibody, BSA free) was purchased from Qiagen (Valencia, CA).

### [Construction and equalization of a mouse skin cDNA library and preparation of DIG-labeled RNA probes]

cDNA libraries were constructed and equalized according to methods previously described (Komiya T., Tanigawa Y., and Hirohashi S. Anal. Biochem. 254: 23-30, 1997). Briefly, mouse skin cDNAs were constructed from total RNAs from mouse back skin (8-week-old female Balb/c mice) using oligo(dT) primers and the Not I restriction site. cDNAs were amplified by PCR, and then two rounds of equalization were carried out following previously-described methods (Ko, Nucleic Acids Res. 18: 5705-5711, 1990; Takahashi N., and Ko M.S.H., genomics 23: 202-210, 1994). In each equalization step (E1 and E2), the Cot value was 10 and 100, respectively.

Equalization was assessed by quantitative real-time PCR analysis in duplicate, as described below. Each gene-specific primer pairs shown below were constructed from each of the cDNA sequences NM016958, NM027011, NM008401, and NM008508:
mouse keratin 14 (5'-GGACGCCCACCTTTCATCTTC-3' (SEQ ID NO: 9), forward; 5'-ATCTGGCGGTTGGTGGAGG-3' (SEQ ID NO: 10), reverse);
mouse keratin 5 (5'-CAGTTCTACATTTGTGTTGCACGTC-3' (SEQ ID NO: 11), forward; 5'-TTGGACAGACTCTGGAGGAAGTCAG-3' (SEQ ID NO: 12), reverse);
mouse integrin α-M (5'-TTGAAAGGACCCCAGTGCTGAACTGC-3' (SEQ ID NO: 13), forward; 5'-ATGGAGCTGCCCACAATGAGTGGTACAG-3' (SEQ ID NO: 14), reverse); and
mouse loricrin (5'-CCTACCTGGCCGTGCAAG-3' (SEQ ID NO: 15), forward; 5'-CATGAGAAAGTTAAGCCCATCG-3' (SEQ ID NO: 16), reverse).
Same amounts of equalized libraries (E1 and E2) and starting library (E0) were used for the PCR analyses. All data were normalized to an internal standard (loricrin; ΔCt method, User Bulletin 2, Applied Biosystems.

The E2 library was digested with Not I and Sal I, and the obtained fragments were cloned into pBlueScript KS(-) (Stratagene, La Jolla, CA). Colonies were randomly selected, and the cDNA inserts were amplified by PCR using vector primer pairs and Native Pfu DNA polymerase (Stratagene). T3 and T7 RNA polymerase promoter sequences derived from the vector were thus added to the amplified fragments. To generate antisense probes (Stratagene), a cRNA transcription was carried out in Nunc MicroWell™ plates (Nunc, Roskilde, Denmark) using T7 RNA polymerase. Sense probes were generated using T3 RNA polymerase (Stratagene).

### [High-throughput in situ hybridization (HT-ISH)]

HT-ISH in a 96-well plate was performed as described previously (Komiya T., Tanigawa Y., and Hirohashi S., Anal. Biochem. 254: 23-30, 1997). Briefly, adult mouse foot pad skin was freshly isolated from 8-week-old female Balb/c mice. Isolated skin was fixed with 1% diethylpyrocarbonate (DEPC) in phosphate-buffered saline (PBS) for one hour at room temperature. Then, samples were dehydrated and embedded, sections were produced, mounted, and hybridized in 96-well plates.

### [Northern blotting]

Total RNAs were prepared from various adult mouse tissues according to the method described by Chomczynski and Sacchi (Chomczynski P., and Sacchi N. Anal. Biochem. 162: 23-30 (1997)). Total RNAs (20 µg) were electrophoresed and transferred onto Hybond-N+ membranes (Amersham Pharmacia Biotech, Little Chalfont, UK).

cDNA fragments of dermokine-α/-β (SK063F08 in Fig. 2B), dermokine-β (β-probe in Fig. 2B), Kdap (XM149907), and suprabasin (318-765 bp ofBC051531) were labeled with DIG using a DIG RNA labeling kit (Roche Applied Science). Hybridization with DIG-labeled RNA probes was performed according to the protocol described by the manufacturer (Roche Applied Science). The expression of dermokine-α/-β in various stages of embryonic development was examined using a Mouse Embryo Full Stage Blot (Seegene, Seoul, Korea).

### [cDNA cloning and construction of SEAP fusion proteins]

First-strand cDNAs were prepared from human skin total RNA (Stratagene) using Superscript II reverse transcriptase (Invitrogen). DNA fragments encoding the ORFs of human dermokine-α and -β were amplified by PCR using 5' SalI-dermokine-α primer
(GTCGACGCCACCATGAACATGAAGCCGGCCACTGC (SEQ ID NO: 17))/3'NotI-dermokine-α primer (GCGGCCGCCCAAAACTTCACCCACTGCAGCAGG (SEQ ID NO: 18)), and 5' SalI-dermokine-β primer
(GTCGACGCCACCATGAAGTTCCAGGGGCCCCTGG (SEQ ID NO: 19))/3'NotI-dermokine-β primer (GCGGCCGCCCAAAACTTCACCCACTGCAGCAGG (SEQ ID NO: 20)), respectively. The cDNAs of human Kdap and suprabasin (0.7-kb fragment; Park G.T., Lim S.E., Jang S., and Morasso M.I.; Suprabasin, a novel epidermal differentiation marker and potential cornified envelope precursor; J.Biol. Chem. 277: 45195-45202; 2002) were amplified using 5'SalI-Kdap primer (GTCGACGCCACCATGAAGATCCCGGTCCTTCCTGCC (SEQ ID NO: 21))/ 3'NotI-Kdap primer (GCGGCCGCCTGGGCATCAGGAGTTGCGCTC (SEQ ID NO: 22)), and 5'SalI-suprabasin primer
(AATTGTCGACGCCACCATGCATCTTGCACGTCTGGTCGG (SEQ ID NO: 23))/3'NotI-suprabasin primer (ATATGCGGCCGCGCAGCTGGTTGGCCTCCTTGCTGG (SEQ ID NO: 24)), respectively. These primers were designed based on sequences with the following GenBank accession numbers: AK003695 (dermokine-α), BC035311 (dermokine-β), BX112106 (Kdap), and BC063640 (suprabasin).

These cDNAs were fused to the 5'-end of a cDNA encoding secreted alkaline phosphatase (SEAP) with a carboxyl-terminal (His)₆ tag and lacking its own signal peptide. A SEAP(His)₆ expression vector, named pcDNA3.1-SEAP(His)₆, was constructed from pDREF-SEAP(His)₆ (Ikeda W., Kakunaga S., Itoh S., Shingai T., Takeuchi K., Satoh K., Inoue Y., Hamaguchi A., Morimoto K., Takeuchi M., Imai T., and Takai Y; J. Biol. Chem. 278: 28167-28172, 2003). After digestion with Sal I and Not I, the cDNAs of dermokine-α, dermokine-β, Kdap, and suprabasin were cloned into the Sal I-Not I sites of pcDNA3.1-SEAP(His)₆ to yield pcDNA3.1-dermokine-α-SEAP(His)₆, pcDNA3.1-dermokine-β-SEAP(His)₆, pcDNA3.1-Kdap-SEAP(His)₆, and pcDNA3.1-suprabasin-SEAP(His)₆, respectively. These expression vectors were introduced into 293/EBNA-1 cells using TransIT LT1 (Mirus, Madison, WI). The SEAP fusion proteins that were transiently expressed and secreted into the medium were purified as described previously (Imai T., Baba M., Nishimura M., Kakizaki M., Takagi S., and Yoshie O. J. Biol. Chem. 272: 15036-15042, 1997), except for those used with TALON Superflow Metal Affinity Resin (BD Biosciences Clontech, Palo Alto, CA). Purified SEAP fusion proteins were subjected to N-terminal amino acid sequencing.

### [In vitro keratinocyte differentiation]

Subconfluent normal human epidermal keratinocytes were seeded in dishes at a density of 2.5 x 10³/cm² in Humedia KG2 medium (0.15 mM Ca²⁺ concentration; containing bovine pituitary extract (BPE)) and cultured for two days. Cells were then washed a few times with either a normal-Ca²⁺ (0.15 mM Ca²⁺) or high-Ca²⁺ (1.5 mM Ca²⁺) medium without BPE. Keratinocytes were then cultured in the normal-Ca²⁺ medium for two, four and six days or in the high-Ca²⁺ medium for two days. Total RNAs to be used in qRT-PCR were prepared from these cells using an RNeasy Mini Kit (Qiagen). When performing a transfection, keratinocytes cultured in 0.15 mM Ca²⁺ for two days were transfected using TransIT LT1 keratinocyte (Mirus).

### [Quantitative real-time RT-PCR analyses]

For quantitative real-time PCR analyses of mouse tissues, total RNAs were prepared from various mouse tissues (8-week-old female Balb/c mice) using an RNeasy Fibrous Tissue Mini Kit (Qiagen). First-strand cDNA templates were prepared from total RNAs using an RNA PCR Kit (AMV) Ver. 2.1 (Takara, Shiga, Japan) and random 9-mer primers. Quantitative real-time PCR was performed in duplicate with a QuantiTect SYBR Green PCR Kit (Qiagen) and ABI Prism 7700 Sequence Detection System (Applied Biosystems, Foster city, CA), by monitoring the increase in fluorescence of the SYBR Green I dye. Primers were designed to be compatible with a single real-time PCR thermal profile (95°C for 15 minutes, followed by 40 cycles of 95°C for 15 seconds and 60°C for one minute) such that multiple transcripts could be analyzed simultaneously. All data were normalized to an internal standard (glyceraldehyde-3-phosphate dehydrogenase mRNA; ΔCt method, User Bulletin 2, Applied Biosystems). Primer sets were as follows:
mouse dermokine-α (AK003695), forward primer (5'-GACTGTACGAGAGCACAACCATG-3' (SEQ ID NO: 25) and reverse primer (5'-CTGAACCCCAGCTGTGGC-3' (SEQ ID NO: 26)); mouse dermokine-β (AK081753), forward primer (5'-CATGCCCCATCTCCCAGC-3' (SEQ ID NO: 27) and reverse primer (5'-CCCTCAATCTGTTTCCAGTTGAAG-3' (SEQ ID NO: 28)); mouse Kdap (XM149907), forward primer (5'-ACTGGCACGTCATCACTGATATGTTC-3' (SEQ ID NO: 29) and reverse primer (5'-GGAATCAGGAGCGGCACTTC-3' (SEQ ID NO: 30));
mouse suprabasin (AY115494), forward primer (5'-GTCAACAAGCCATTTATCAACTTCC-3' (SEQ ID NO: 31) and reverse primer (5'-GTGTGACAACCGGAGCATTC-3' (SEQ ID NO: 32));
mouse GAPDH (NM008084), forward primer (5'-AAGGTGGTGAAGCAGGCATCTGAG-3' (SEQ ID NO: 33) and reverse primer (5'-GGAAGAGTGGGAGTTGCTGTTGAAGTC-3' (SEQ ID NO: 34));
human dermokine-α (AL832080), forward primer (5'-ATGAACATGAAGCCGGCCAC-3' (SEQ ID NO: 35) and reverse primer (5'-CGTTTCTGCAGTGATGACGCG-3' (SEQ ID NO: 36)); human dermokine-β (BC035311), forward primer (5'-AAAGGCCATTGGCAAAGAGGCC-3' (SEQ ID NO: 37) and reverse primer (5'-ACCCTGTTGCCCAAAGCATCTG-3' (SEQ ID NO: 38));
human Kdap (BX112106), forward primer (5'-AACTGGCACGCCCTCTTTGAGTCTATC-3' (SEQ ID NO 39:) and reverse primer (5'-ATGGTCACTGGGCATCAGGAGTTG-3' (SEQ ID NO: 40));
human suprabasin (BC063640), forward primer (5'-AACCAGCTGCTGAATGGCAACCA-3' (SEQ ID NO: 41) and reverse primer (5'-ATGAAAGGCGTGTTGACCGAGG-3' (SEQ ID NO: 42));
human keratin 10 (J04029), forward primer (5'-CTTGGCAGAAACAGAAGGTCGCTAC-3' (SEQ ID NO: 43) and reverse primer (5'-CGGTTTCAGCTCGAATCTCTTGC-3' (SEQ ID NO: 44));
human involucrin (M13903), forward primer (5'-CCACCCAAACATAAATAACCACCCG-3' (SEQ ID NO: 45) and reverse primer (5'-TAGCGGACCCGAAATAAGTGGAGC-3' (SEQ ID NO: 46));
human TGase 1 (NM000359), forward primer (5'-ATGTCTCAGGCCACGTCAAG-3' (SEQ ID NO: 47) and reverse primer (5'-CTGCTCCCAGTAACGTGAGG-3' (SEQ ID NO: 48));
human β-actin (NM001101), forward primer (5'-ATCAAGATCATTGCTCCTCCTGAG-3' (SEQ ID NO: 49) and reverse primer (5'-TGCTTGCTGATCCACATCTGC-3' (SEQ ID NO: 50)); human GAPDH (NM002046), forward primer (5'-ACTTCAACAGCGACACCCACTC-3' (SEQ ID NO: 51) and reverse primer (5'-CCTGTTGCTGTAGCCAAATTCG-3' (SEQ ID NO: 52));

### [High-throughput in situ hybridization screening of genes expressed in the mouse epidermis]

To generate cRNA probes for *in situ* hybridization of sections of mouse skin, the present inventors prepared a cDNA library from the back skin of 8-week-old female Balb/c mice, and equalized the cDNA library as described in the above experimental procedures. Equalization is required for this type of screening that efficiently picks up genes transcribed at a low level. To confirm that the cDNA libraries were successful equalized, the present inventors performed a quantitative real-time PCR analysis with gene-specific primers for mouse keratin 14 (NM016958), keratin 5 (NM027011), integrin α-M (NM008401), and loricrin (NM008508), with the expectation that keratin 14 and keratin 5 would be expressed in large amounts, while the expression level of integrin α-M would be relatively low. To normalize the amount between cDNA templates, the amount of loricrin cDNA was determined using primers specific to mouse loricrin. As shown in Fig. 1A, after equalization of the library, the amount of both keratin 14 and keratin 5 were shown to have decreased to about one fifth, whereas the amount of integrin α-M had increased by about 42-fold. The efficiency of equalization was also evaluated by DNA sequencing of about 500 clones randomly selected from the library before and after equalization. Cluster analyses showed that preliminary redundancy rates for the non-equalized and equalized libraries were 35.3% and 1.3%, respectively. These findings indicate that the equalization method successfully reduced the amount of highly abundant cDNA species, that is, it successfully reduced clone redundancy.

The mouse foot pad epidermis is thicker than its interfollicular epidermis, such as back skin, and is similar to the human epidermis in morphology. Thus, the present inventors used sections of mouse foot pad epidermis (8-week-old Balb/c mice) to identify the layer-specific expression of genes in high-throughput *in situ* hybridization (HT-ISH). After placing sections on each well of 96-well plastic plates (100 plates; 9600 sections in total), DIG-labeled cRNA probes prepared from the equalized cDNA library were hybridized. Hybridization signals were observed in about 1000 of the 9600 sections.

Subsequent sequencing eliminated about 600 redundant cDNA clones, leaving the independent genes. The present inventors assessed the reproducibility of the *in situ* hybridization for these genes, eliminated clones that stained the nucleus, and finally selected 116 unique clones that were expressed in specific layers in the mouse foot pad epidermis. These clones, of course, comprised genes that were already well characterized. The HT-ISH signals for cyclophilin A, Kdap, Prothymosin α, diazepam-binding inhibitor, Tubulin β-2 chain homologue, lamin A, nuclear distribution C homologue, desmoplakin, keratin-associated protein 16-5, nucleoside diphosphate kinase C, small proline rich-like 2, and loricrin are shown in Fig. 1B as examples. Consistent with previous reports, the expression of cyclophilin A, Kdap, and loricrin was restricted to the basal/suprabasal, suprabasal, and granular layers, respectively (AL-Daraji WI, Grant KR, Ryan K, Sacton A, Reynolds NJ: Localization of calcinurin/ NFAT in human skin and psoriasis and inhibition of calcinurin/ NFAT activation in human keratinocytes by cyclosporin A. J. Invest. Dermatol. 118: 779-788, 2002.; Oomizu S, Sahuc F, Asahina K, Inamatsu M, Matsuzaki T, Sasaki M, Obara M, Yoshizato K: Kdap, a novel gene associated with the stratification of the epithelium. Gene. 256:19-27, 2000.; Mehrel T, Hohl D, Rothnagel JA, Longley MA, Bundman D, Cheng C, Lichti U, Bisher ME, Steven AC, Steinert PM et al.: Identification of a major keratinocyte cell envelope protein, loricrin. Cell 61:1103-1112, 1990). These findings indicated that the HT-ISH screening had worked well.

### [Identification of novel genes expressed in the spinous layer]

From these 116 cDNA clones, the inventors identified a novel cDNA fragment (SK063F08), the *in situ* hybridization signal of which was specifically detected in the spinous layer (Fig. 2A). To obtain a full-length clone of this novel cDNA, publicly available mouse EST databases were searched, and an EST clone (AK003695) comprising the entire sequence of SK063F08 was identified (Fig. 2B). The expression of AK003695 in mouse skin was verified by RT-PCR. The EST clone AK003695 was composed of a short 5'-untranslated region (UTR) of 22 bp, an open reading frame (ORF) of 315 bp encoding 104 amino acids (SEQ ID NO: 53), and a 3'-UTR of 324 bp comprising a polyadenylation signal.

Furthermore, another, longer EST (AK081753) was found in the database. This EST had the same sequence as AK003695 at its 3'-end (98-657 bp), indicating that these ESTs were splicing variants (Fig. 2B). The expression of AK081753 in mouse skin was also confirmed by RT-PCR. Close examination of the nucleotide sequence of several cloned cDNAs corresponding to AK081753 identified a single nucleotide deletion at the position of 743 bp, a 27 bp insertion between 1132 bp and 1133 bp, and a 48 bp deletion between 1197 bp and 1244 bp of AK081753. The corrected AK081753 EST sequence comprised a short 5'-UTR of 170 bp, an ORF of 1554 bp encoding a protein of 517 amino acids, and a 3'-UTR of 320 bp comprising a polyadenylation signal (Fig. 2B).

Two EST clones (AL832080 and BC035311) homologous to each of the mouse ESTs (AK003695 and AK081753) were identified in human EST databases (Fig. 2B). These were approximately 63% and 55% identical at the nucleotide sequence level and both were 54% identical at the amino acid sequence level (Figs. 2B and C). The sequences proximal to the initiation codon of the mouse and human ESTs, AK003695, AK081753, AL832080, and BC035311, matched the translation initiation start site proposed by Kozak (Kozak M., Nucleic Acids Res., 15: 8125-8148, 1987).

The deduced amino acid sequences of AK003695, AK081753, AL832080, and BC035311 showed no similarity to any other sequences in GenBank. There were two conserved cysteine residues (arrows in Fig. 2C) and a conserved potential N-glycosylation site (arrowhead in Fig. 2C) in dermokine-β, and several potential N-myristoylation sites in dermokine-α/-β. Interestingly, from the amino acid sequences deduced from all these ESTs, the SignalP server predicted a typical signal sequence in their N-terminus (http://www.cbs.dtu.dk/services/SignaIP-2.0/#submission). Furthermore, the C-terminal portion shared by the two splicing variants showed a very high pI value (pI = 10.3; see Figs. 2B and C and Fig. 4A), which is common in various cytokines, such as bone morphogenetic proteins (BMPs), eotaxin, fibroblast growth factors (FGFs), interferon-β (IFN-β), interleukins (ILs), platelet-derived growth factor (PDGF), and Wnts. Thus, considering that the mRNAs of these peptides are expressed abundantly in the skin as explained below, the present inventors tentatively named the short and long splicing variants "dermokine-α and -β", respectively (Fig. 2C).

### [Structure of the dermokine-α/-β gene]

Using the Ensembl Human Genome Browser, the gene for human dermokine-α and -β was localized on chromosome 19q13.1 (ENSG00000161249), spanning approximately 14 kb. As shown in Fig. 3A, human dermokine-α and -β comprise six and 16 exons, respectively. Interestingly, exon 1 of the dermokine-α gene comprises both a putative translation initiation site and a signal sequence, and is located between exons 11 and 12 of the dermokine-β gene, suggesting that they are transcribed by distinct promoters. The putative termination codon is comprised in exon 5 of dermokine-α, which is exon 15 of dermokine-β. The mouse dermokine-α/-β gene was mapped as ENSMUSG00000036665 by the Ensembl Mouse Genome Browser on chromosome 7, which is a region of shared synteny with human chromosome 19q13.1. The precise structure of the mouse dermokine-β gene is not determined due to incomplete information from the available genomic databases, however, the same genomic structure was confirmed in the vicinity of the mouse dermokine-α gene locus.

Two genes, the keratinocyte-associated peptide suprabasin and the keratinocyte differentiation-associated protein (Kdap), have been mapped on both sides of the dermokine gene on human chromosome 19q13.1 and mouse chromosome 7 (Fig. 3B). Both proteins were recently reported to be highly expressed in stratified epithelia (Oomizu S., Sahuc F., Asahina K., Inamatsu M., Matsuzaki T., Sasaki M., Obara M., and Yoshizato K., a novel gene associated with the stratification of the epithelium, Gene 256: 19-27, 2000; Park G.T., Lim S.E., Jang S., and Morasso M.I., Suprabasin, a novel epidermal differentiation marker and potential cornified envelope precursor, J. Biol. Chem. 277: 45195-45202, 2002). Interestingly, the suprabasin, dermokine-α/-β, and Kdap genes are aligned in this order with the same direction of transcription. The genes for testis-specific glyceraldehyde-3-phosphate dehydrogenase and the ubiquitously expressed GPR43, which do not encode keratinocyte-specific proteins, are located upstream of suprabasin and downstream of Kdap, respectively (Welch J.E., Schatte E.C., O'Brien D.A., and Eddy E.M., Expression of a glyceraldehyde 3-phosphate dehydrogenase gene specific to mouse spermatogenic cells, Biol. Reprod. 46: 869-878, 1992; Sawzdargo *et al.*, 1997); however, these genes are transcribed in the opposite direction to suprabasin, dermokine-α/-β, and Kdap. This finding, that is, the existence of a novel cluster of keratinocyte-associated genes, suggests that the transcription of suprabasin, dermokine-α/-β, and Kdap mRNAs is regulated in a coordinated manner.

### [Secretion of dermokine-α/-β, Kdap, and suprabasin]

As shown in Fig. 4A, the SignalP server predicted that not only dermokine-α/-β but also Kdap and suprabasin comprise a putative signal sequence at their N terminus (Fig. 4A). This was previously pointed out in Kdap (Oomizu S., Sahuc F., Asahina K., Inamatsu M., Matsuzaki T., Sasaki M., Obara M., and Yoshizato K., Kdap, a novel gene associated with the stratification of the epithelium, Gene 256: 19-27, 2000); on the other hand , the N-terminal hydrophobic sequence of suprabasin is thought to be a potential transmembrane domain (Park G. T., Lim S.E., Jang S., and Morasso M.I., Suprabasin, a novel epidermal differentiation marker and potential cornified envelope precursor, J. Biol. Chem. 277: 45195-45202, 2002). However, since the dermokine-α/-β, Kdap, and suprabasin genes form a gene complex, it was tempting to speculate that their products are all secreted from keratinocytes. To test this idea, human dermokine-α/-β, Kdap, and suprabasin (0.7-kb transcript) cDNAs were inserted into the mammalian expression vector pcDNA3.1-SEAP(His)₆ to express fusion proteins tagged with SEAP(His)₆ at their C-termini. These fusion proteins were transiently expressed in cultured 293/EBNA-1 cells, and detected in the culture medium by immunoblotting with anti-His antibodies (Fig. 4B). Interestingly, compared to the estimated molecular masses of the fusion proteins, cells detected in the medium experienced a significant upward shift in SDS-PAGE gels, suggesting that the secreted proteins may be posttranslationally modified by glycosylation and/or intermolecular disulfide bonding.

Next, the present inventors examined whether the N-terminal putative signal sequences were removed from these secreted fusion proteins. Each secreted fusion protein was purified as a single band from the culture medium using TALON Superflow Metal Affinity Resin (Fig. 4C). Under the conditions for transfection and culture used in the present study, dermokine-α-SEAP(His)₆, dermokine-β-SEAP(His)₆, Kdap-SEAP(His)₆, and suprabasin-SEAP(His)₆ were purified with yields of 0.15 mg/L, 0.3 mg/L, 13.5 mg/L, and 15 mg/L, respectively. Direct amino acid sequencing of the N-terminus of these purified proteins revealed that the secreted proteins lacked putative N-terminal signal sequences such as those predicted in Fig. 4A, to be precise (Fig. 4C). These findings indicate that dermokine-α/-β, Kdap, and suprabasin are cleaved at their N-termini, modified further posttranslationally, and then secreted as a result.

### [Expression patterns of dermokine-α/-β, Kdap, and suprabasin in tissues]

The expression of dermokine-α/-β, Kdap, and suprabasin transcripts in various mouse tissues was examined by Northern blotting (Fig. 5A). The SK063F08 probe, which hybridizes with both dermokine-α and dermokine-β (see Fig. 2B), detected two bands of 0.6 kb and 2.0 kb whose intensities were strong in the skin and weak in the stomach (Dermokine-α/β; Fig. 5A); after a long exposure, these bands were also detected in the lung (data not shown). Judging from their sizes, these bands were supposed to correspond to dermokine-α and -β, respectively. Indeed, when the dermokine-β-specific probe (β-probe; Fig. 2B) was used, only the 2.0 kb band was detected (Dermokine-β; Fig. 5A). Kdap was expressed in the stomach and skin in large amounts and in the lung in small amounts (Kdap; Fig. 5A). The expression patterns of the 0.7 kb and 2.2 kb splicing variants of the suprabasin gene were very similar to those of dermokine-α/-β (Suprabasin; Fig. 5A). Furthermore, the present inventors examined the expression of these proteins using quantitative RT-PCR (qRT-PCR) (Table 1).

Table 1 shows the results of quantitative real-time RT-PCR analyses of the expression of dermokine-α, dermokine-β, Kdap, and suprabasin in various epithelial tissues. Total RNAs were prepared from various epithelial tissues of 8-week-old female Balb/c mice, and SYBR Green-based quantitative real-time RT-PCR was performed using primers specific to mouse dermokine-α and -β, Kdap, and suprabasin. All data were normalized to an internal GAPDH mRNA control. Ratios relative to GAPDH (x10⁻³): - = 0-0.1; + = 0.1-1; ++ = 1-10; +++ = 10-100; ++++ = 100-1000; +++++ = 1000-2000; ++++++ = 2000-3000.

**[Table 1]**

| TISSUE | DERMOKINE-α | DERMOKINE-β | Kdap | SUPRABASIN |
|---|---|---|---|---|
| BACK SKIN | +++ | ++++ | +++ | +++ |
| FOOT PAD SKIN | ++++ | +++++ | ++++ | ++++ |
| TONGUE | +++ | ++++ | +++ | +++ |
| ESOPHAGUS | +++ | ++++ | ++++ | +++ |
| FORESTOMACH | ++++ | ++++++ | +++++ | ++++ |
| GLANDULAR STOMACH | ++ | ++ | ++ | + |
| VAGINA | +++ | +++ | +++ | ++ |
| TRACHEA | ++++ | +++ | ++ | ++ |
| LUNG | +++ | +++ | - | ++ |
| BLADDER | + | + | + | + |
| THYMUS | ++ | ++ | + | + |
| SMALL INTESTINE | - | - | - | - |
| LIVER | - | - | - | - |

In good agreement with the Northern blotting results, dermokine-α/-β, Kdap, and suprabasin were expressed abundantly in stratified epithelia of the skin, tongue, esophagus, forestomach, vagina, and such. They were expressed in the trachea and bladder as well as in the thymus, which was consistent with reports that Kdap is expressed in the trachea and bladder and that suprabasin is expressed in the thymus (Oomizu S., Sahuc F., Asahina K., Inamatsu M., Matsuzaki T., Sasaki M., Obara M., and Yoshizato K., Gene 256: 19-27, 2000; Park GT., Lim S.E., Jang S., and Morasso M.I., J. Biol. Chem. 277: 45195-45202, 2002). They were undetectable in typical simple epithelia such as liver and small intestine even by RT-PCR. Therefore, the present inventors concluded that dermokine-α/-β, Kdap, and suprabasin were secreted proteins specific to the stratified epithelium, although with some exceptions. This conclusion is consistent with the notion that the expression of these genes, which form a complex on a chromosome, is regulated in harmony in a stratified epithelium-specific manner. Therefore, the present inventors herein propose that this gene complex be designated SSC (stratified epithelium secreted peptides complex).

Next, the present inventors compared in detail the expression of SSC products in the stratified epithelium by carrying out *in situ* hybridization analyses on serial sections of the mouse foot pad epidermis. Eight serial sections of the epidermis were hybridized with antisense or sense cRNA probes for dermokine-α/-β, Kdap, and suprabasin. As shown in Fig. 5B, the antisense probe for dermokine-α/-β (SK063F08, Fig. 2B), dermokine-β (β-probe, Fig. 2B), and suprabasin produced hybridization signals evenly throughout the spinous layer of the epidermis. In contrast, the Kdap-specific probe hybridized largely to the suprabasal portion of the spinous layer, as reported previously by Oomizu *et al.* (Oomizu S., Sahuc F., Asahina K., Inamatsu M., Matsuzaki T., Sasaki M., Obara M., and Yoshizato K., Kdap, a novel gene associated with the stratification of the epithelium, Gene 256: 19-27, 2000). These results suggest that the expression of the SSC products was activated at the onset of keratinocyte stratification and differentiation within the epidermis.

### [SSC products and keratinocyte differentiation]

Dermokine-α/-β expression during embryonic development was examined by Northern blotting using the SK063F08 probe. As shown in Fig. 6A, dermokine-β began to be expressed at E15.5, and this timing coincides well with the epidermal stratification. Dermokine-α is detectable at E16.5, increases until E17.5, and decreases by E18.5. These findings suggest that dermokine-α and -β are associated with *in vivo* stratification of the epidermis during early development in mice.

Finally, to examine further the relationship between SSC products and keratinocyte differentiation, changes in the expression levels of SSC products during the period of *in vitro* differentiation of primary cultured human keratinocytes were monitored by qRT-PCR. Differentiation was induced either by an increase in the Ca²⁺ concentration (from 0.15 mM to 1.50 mM) or by an increase in cell density (from a two-day culture to a six-day culture) under 0.15 mM Ca²⁺. In human keratinocytes, the latter method has been reported to be more effective for inducing terminal differentiation (Poumay Y, and Pittelkow M.R., Cell density and culture factors regulate keratinocyte commitment to differentiation and expression of suprabasal K1/K10 keratins, J. Invest. Dermatol. 104: 271-276, 1995), and this was confirmed by measuring the mRNA levels of keratin 10, TGase 1, and involucrin using qRT-PCR (Fig. 6B). Interestingly, expression of all SSC products was clearly induced as long as terminal differentiation proceeded *in vitro.*

### [Effect of dermokine-α on keratinocyte differentiation]

The effect of dermokine-α on keratinocyte differentiation was then examined. A cDNA for human dermokine-α with an HA-tag at the C-terminus was transfected and transiently expressed in keratinocytes cultured at 2500 cells/cm² in 12-well dishes in the presence of 0.15 mM Ca²⁺. As a result, the yield of total RNAs recovered six days later was significantly lower for dermokine-α-expressing keratinocytes compared to mock cells (Fig. 7A). This suggests that proliferation was suppressed.

Furthermore, the expression level of involucrin, a differentiation marker for stratified epithelia, was measured from such RNAs by qRT-PCR using primers for involucrin and using GAPDH as a standard, and was found to be significantly increased as compared to mock (Fig. 7B). This suggested that keratinocytes differentiation was promoted by dermokine-α expression. These above results suggest that dermokine-α plays an important role in the proceeding of the proliferation and differentiation of keratinocytes.

### [Generation of antibodies against the SSC protein group]

To generate rabbit polyclonal antibodies against human dermokine-α, and -β, Kdap, and suprabasin, pcDNA3.1-SEAP(His)₆ vectors into which human dermokine-α, Kdap, and suprabasin have been introduced, which were used in Fig. 4, and one in which the region shared with dermokine-α has been deleted from dermokine-β (dermokine-β-ΔC) were produced (Fig. 8A) and transiently expressed in 293/EBNA-1. Fusion proteins were purified from the resulting culture supernatants as a single band using TALON Superflow Metal Affinity Resin (Fig. 8B). Rabbits were immunized with these fusion proteins and antisera were obtained. Using the obtained antisera, immunoblotting was performed on culture supernatants of the same 293/EBNA-1 transiently expressing human dermokine-α, -β, -β-ΔC, Kdap, and suprabasin. The results indicated that the antisera reacted with these four types of protein (Fig. 8C). pcDNA3.1-SEAP(His)₆ vectors into which a cDNA for a C-terminal deletion form of human or mouse dermokine-β (dermokine-β-ΔC) has been introduced were introduced in 293/EBNA-1 and transiently expressed.

### [Generation of monoclonal antibodies]

To produce antigens for monoclonal antibodies, pcDNA3.1-SEAP(His)₆ vectors into which a cDNA for a C-terminal deletion form of human or mouse dermokine-β (dermokine-β-ΔC) has been introduced were introduced in 293/EBNA-1 and transiently expressed. Human and mouse dermokine-β-ΔC-SEAP(His)₆ proteins were purified from the obtained culture supernatants using TALON Superflow Metal Affinity Resin. The human and mouse dermokine-β-ΔC-SEAP(His)₆ fusion proteins were used to immunize mice (Balb/c female) and rats (Wister rat female), respectively, and monoclonal antibodies were generated (designated as Anti-hDK mAB and Anti-mDK mAb, respectively).

### [Immunoblotting of the 293 cell culture supernatants]

Human and mouse dermokine-β and -β-ΔC expressed in 293 cells were analyzed by immunoblotting.

pcDNA3.1-human dermokine-β, pcDNA3.1-human dermokine-β-ΔC, pcDNA3.1-mouse dermokine-β, and pcDNA3.1-mouse dermokine-β-ΔC were transfected into 293/EBNA-1 cells, the resulting culture supernatants were subjected to SDS-PAGE, and transferred onto nitrocellulose membranes. Immunoblotting was carried out by reacting these membranes with anti-hDK mAb and anti-mDK mAb.

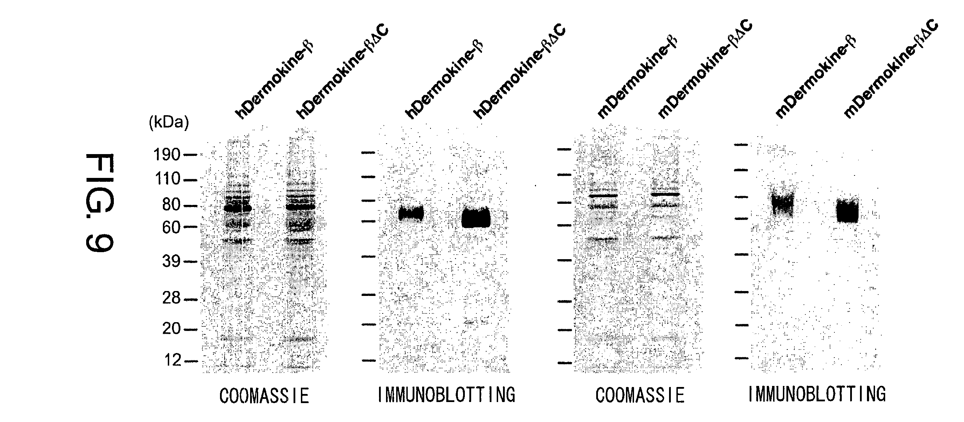

Results are shown in Fig. 9 right(mice) and left (humans). While many proteins in the culture supernatant were stained with CBB, a band was specifically detected at around 80 kDa by immunoblotting, indicating that both anti-hDK mAb and anti-mDK mAb specifically recognized dermokine-β and -β-ΔC.

### [ELISA for dermokine]

50 µl of anti-rat IgG polyclonal antibody, diluted to 2 µg/ml with PBS, was added to each well of 96-well ELISA microplates (Nunc), incubated at 37°C for one hour, and absorbed onto the microplates. After the solution of polyclonal antibody was removed, 100 µl of a blocking reagent (Block-Ace, Dainippon Pharmaceutical Co. Ltd.) was added to each well, and incubated at room temperature for one hour to block the sites where the antibody did not bind. Each well was washed once with phosphate-buffered saline containing 0.02% Tween-20 (0.02% T-PBS), and 50 µl of the produced anti-hDK mAb and anti-mDK mAb (serum-free culture media of hybridomas) were added, and incubated at room temperature for one hour. After the solution was discarded, each well was washed three times with 0.02% T-PBS, and the antibodies were immobilized.

Next, each well was treated at 65°C for 10 minutes, and then 50 µl of human or mouse dermokine-β-SEAP(His)₆, or human or mouse dermokine-β-ΔC-SEAP(His)₆, diluted with PBS to 6.25-50 nM, was added, and this was incubated at room temperature for one hour. The microplate was washed five times with 0.02% T-PBS, and the alkaline phosphatase activity of human or mouse dermokine-β-SEAP(His)₆, or dermokine-β-AC-SEAP(His)₆ that had bound to the immobilized antibodies was detected using the AURORA AP Chemiluminescent Reporter Gene Assay Kit (ICN). The reaction solution was measured with a 1420 ARVO multi-label counter, Wallac 1420 Manager, at 460 nm.

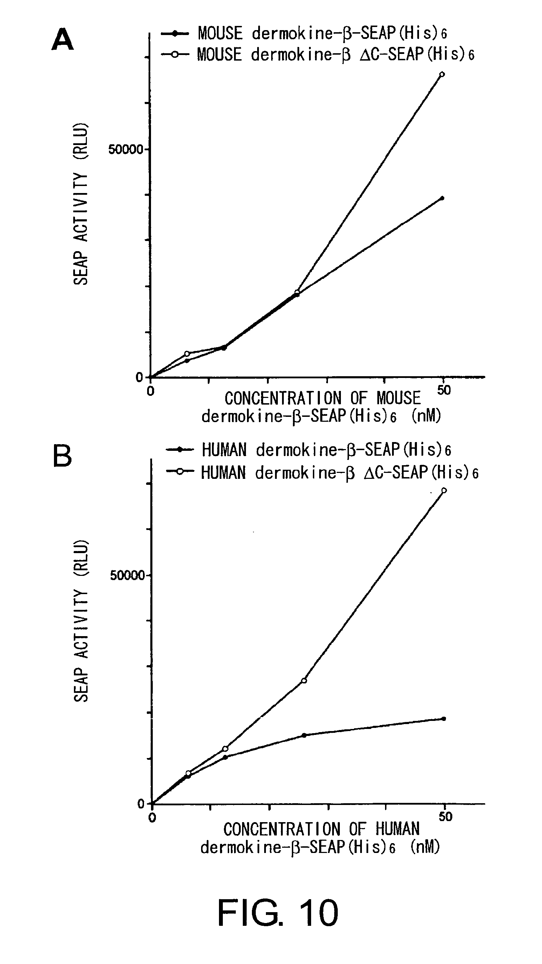

Fig. 10A (mice) and 10B (humans) show the binding results of dermokine-β-SEAP(His)₆ (-•-) and dermokine-β-ΔC-SEAP(His)₆ (-o-). The immobilized anti-hDK mAb and anti-mDK mAb both bound to dermokine-β in a concentration-dependent manner. There was almost no binding of the recombinant protein for SEAP alone to the immobilized antibodies.

### [Purification of recombinant human dermokine-β and dermokine-β ΔC]

pcDNA3.1-human dermokine-β and pcDNA3.1-human dermokine-β-ΔC were transfected into 293/EBNA-1 cells. The obtained culture supernatants (medium: Opti-MEM I, GIBCO) were supplemented with NaCl to a final concentration of 0.5 M, and then passed through Lentyl Lectin Sepharose 4B beads (Amersham Bioscience) that had been equilibrated with 20 mM HEPES (pH 7.5) buffer comprising 0.5 M NaCl to absorb the proteins.
Non-specifically binding proteins were then washed away with 20 mM Tris-HCl (pH 7.5) containing 1 M NaCl, and following an additional wash with 20 mM Tris-HCl (pH 7.5), bound proteins were eluted with 20 mM Tris-HCl (pH 7.5) comprising 1 M methl-α-D-mannopyranoside (α-MM) and 0.6% CHAPS. Next, these eluates were loaded onto an anionic exchange chromatography column, the HiTrap Q HP column (Amersham Bioscience), equilibrated with 20 mM Tris-HCl (pH 7.5) comprising 0.6% CHAPS, washed with the same buffer, and eluted with a 0 to 0.2 M NaCl concentration gradient. Column manipulations were performed at 4°C using the AKTA explorer 10S system (P-903 pump, UV-900 monitor, pH/C-900 monitor, Frac-901 fraction collector (Amersham Bioscience)). Proteins in each fraction were separated by SDS-polyacrylamide gel electrophoresis (SDS-PAGE) in 15% acrylamide gels. After electrophoresis, the gels were stained with silver nitrate (2D-Silver Stain II "Daiichi" kit, Daiichi Pure Chemicals Co.) to visualize protein bands. Moreover, proteins bands transferred onto nitrocellulose membranes were reacted with anti-hDK-mAb (immunoblotting), and the elution of human dermokine-β and human dermokine-β-ΔC in fractions 11-23 was confirmed.

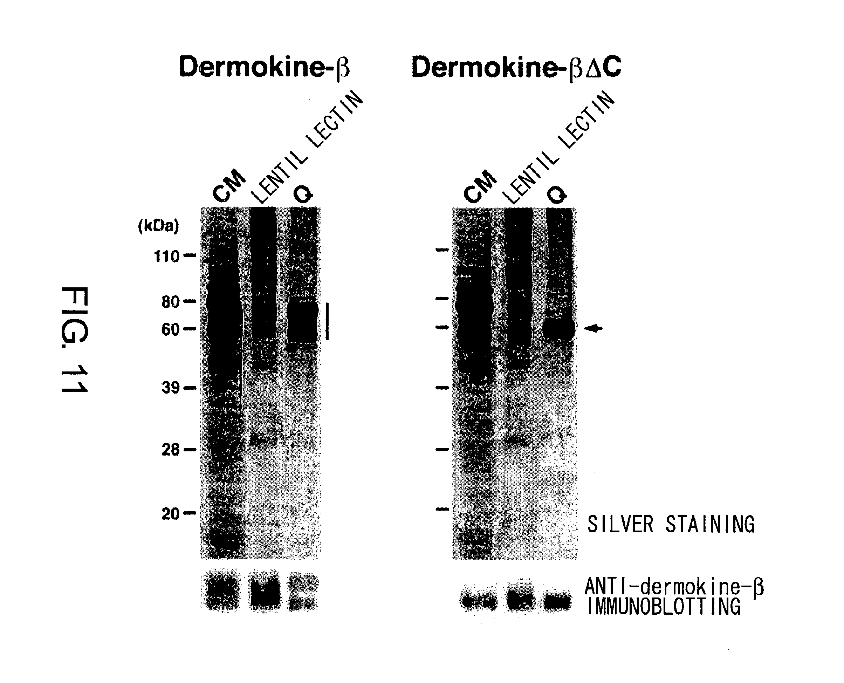

The silverstain images and images of immunoblots by anti-hDK mAb at each step (CM: culture media; Lentyl Lectin: Lentyl Lectin Sepharose 4B; Q: HiTrap Q HP column) are shown (Fig. 11). Dermokine-β was detected as doublet bands (Fig. 11, left), whereas dermokine-β-ΔC was detected as a single band (Fig. 11, right).

### Industrial Applicability

The present invention demonstrated that SSC is expressed in the stratified epithelia, and induced in a differentiation-specific manner. Thus, SSC may be involved in the differentiation and/or proliferation of epidermal keratinocytes. The exact physiological roles of SSC can be examined by generating dermokine-α/-β knockout mice. In addition, this gene may be used as a novel marker for the characterization of diseases of the skin and other stratified epithelia.

## Claims

1. A polynucleotide of any one of (a) to (d):
(a) a polynucleotide encoding a polypeptide comprising an amino acid sequence of any one of SEQ ID NO: 2, 4, 6, or 8;
(b) a polynucleotide comprising the coding region of a nucleotide sequence of any one of SEQ ID NO: 1, 3, 5, or 7;
(c) a polynucleotide encoding a polypeptide having an activity of causing a keratinocyte to differentiate into a stratified epithelial cell when expressed in the keratinocyte, wherein the polypeptide comprises an amino acid sequence with a substitution, deletion, insertion, and/or addition of one or more amino acids in the amino acid sequence of any one of SEQ ID NO: 2, 4, 6, or 8; and
(d) a polynucleotide encoding a polypeptide having an activity of causing a keratinocyte to differentiate into a stratified epithelial cell when expressed in the keratinocyte, wherein the polynucleotide hybridizes under stringent conditions with a DNA comprising a nucleotide sequence of any one of SEQ ID NO: 1, 3, 5, or 7.

2. The polynucleotide of claim 1 that is a gene involved in keratinocyte differentiation or proliferation, wherein the polynucleotide encodes a secreted protein.

3. A polypeptide encoded by the polynucleotide of claim 1 or 2.

4. A vector into which the polynucleotide of claim 1 or 2 is inserted.

5. A host cell carrying the polynucleotide of claim 1 or 2 or the vector of claim 4.

6. A method for producing the polypeptide of claim 3, comprising the steps of culturing the host cell of claim 5, and recovering a produced polypeptide from the host cell or its culture supernatant.

7. A gene complex related to keratinocyte differentiation or proliferation, comprising each of (1) a Kdap gene; (2) a dermokine-α gene; (3) a dermokine-β gene; and (4) a suprabasin gene, wherein regulation of gene expression is commonly controlled.

8. A polynucleotide that hybridizes specifically with the polynucleotide of claim 1 or 2, wherein the polynucleotide has a chain length of at least 15 nucleotides.

9. An antisense polynucleotide against the polynucleotide of claim 1 or 2, or a portion thereof.

10. An antibody that binds to the polypeptide of claim 3.

11. An agent for inducing keratinocyte differentiation, comprising a dermokine-α protein or a dermokine-β protein as an active ingredient.

12. An inhibitor of keratinocyte differentiation, comprising a compound selected from the group of (a) to (d):
(a) an antisense nucleic acid against a transcript of a dermokine-α gene or a dermokine-β gene;
(b) a nucleic acid having a ribozyme activity that specifically cleaves a transcript of a dermokine-α gene or a dermokine-β gene;
(c) a nucleic acid having an effect of inhibiting the expression of a dermokine-α gene or a dermokine-β gene through an RNAi effect; and
(d) an antibody that binds to a dermokine-α protein or a dermokine-β protein.

13. A method of screening for an inducer of keratinocyte differentiation, comprising steps (a) to (c):
(a) contacting a cell that expresses a dermokine-α protein or a dermokine-β protein with a test compound;
(b) measuring an expression level or an activity of a dermokine-α protein or a dermokine-β protein in the cell; and
(c) selecting a compound that increases the above expression level or activity, compared to in the absence of contact with the test compound.

14. A method of screening for an inhibitor of keratinocyte differentiation, comprising steps (a) to (c):
(a) contacting a cell that expresses a dermokine-α protein or a dermokine-β protein with a test compound;
(b) measuring an expression level or an activity of a dermokine-α protein or a dermokine-β protein in the cell; and
(c) selecting a compound that decreases the above expression level or activity, compared to in the absence of contact with the test compound.

15. A method of screening for an inducer of keratinocyte differentiation, comprising steps (a) to (c):
(a) coexisting a test compound and a keratinocyte with a dermokine-α protein, a dermokine-β protein, or a cell expressing these proteins;
(b) measuring the differentiation of a keratinocyte into a stratified epithelial cell; and
(c) selecting a compound that increases differentiation into a stratified epithelial cell, compared to in the absence of the test compound.

16. A method of screening for an inhibitor of keratinocyte differentiation, comprising steps (a) to (c):
(a) coexisting a test compound and a keratinocyte with a dermokine-α protein, a dermokine-β protein, or a cell expressing these proteins;
(b) measuring the differentiation of a keratinocyte into a stratified epithelial cell; and
(c) selecting a compound that decreases differentiation into a stratified epithelial cell, compared to in the absence of the test compound.

17. A method for examining whether or not a subj ect cell is a cancer cell derived from a stratified epithelium, comprising steps (a) and (b):
(a) measuring an expression level or an activity of a dermokine-α protein or a dermokine-β protein in a subject cell; and
(b) determining that the subject cell is a cancer cell derived from a stratified epithelium when the above expression level or activity is different from a control.

18. A method for diagnosing a squamous epithelial cancer or a basal cell cancer in a subject, comprising steps (a) and (b):
(a) determining whether or not a cell sample prepared from a subject is a cancer cell derived from a stratified epithelium, using the method of claim 17; and
(b) determining that the subject is affected with a squamous epithelial cancer or a basal cell cancer when the cell sample is determined to be a cancer cell derived from a stratified epithelium in the above step.

19. A method for diagnosing a skin disease in a subject, comprising steps (a) and (b):
(a) measuring an expression level or activity of a dermokine-α protein or a dermokine-β protein in a test sample prepared from a subject; and
(b) determining that the subject is affected with a skin disease when the above expression level or activity is different from a control.

20. The method for diagnosing of claim 19, wherein the skin disease is a xeroderma, psoriasis, or ichthyosis.
